# EUROPEAN PATENT APPLICATION

(11) **EP 4 545 644 A1**
(43) Date of publication of application: **30.04.2025**
(21) Application number: 23205639.0
(22) Date of filing: 24.10.2023
(51) Int. Cl.: C12N 15/86

(54) **POLYNUCLEOTIDES**

(71) Applicant: Ospedale San Raffaele S.r.l., 20132 Milano (IT); Fondazione Telethon ETS, 00185 Roma (IT)
(72) Inventor: MONTINI, Eugenio, 20154 Milano (IT); CESANA, Daniela, 23847 Molteno (IT); VOLPIN, Monica, 20146 Milano (IT)
(74) Representative: D Young & Co LLP

(57) **Abstract**

The present invention relates to polynucleotides comprising a nucleotide sequence comprising an expression cassette and a transcription termination site, wherein the nucleotide sequence further comprises: (a) one or more miRNA target sequences having the same orientation as the expression cassette, upstream from the expression cassette and/or downstream from the transcription termination site; (b) one or more miRNA target sequences having the opposite orientation as the expression cassette; and (c) one or more insulator elements.

## Description

### FIELD OF THE INVENTION

The present invention relates to genetic elements and polynucleotides for improving safety of gene therapy, gene editing and cancer immunotherapy. The present invention also relates to vectors, cells, and pharmaceutical compositions comprising the same.

### BACKGROUND TO THE INVENTION

Lentiviral Vectors (LVs) with self-inactivating (SIN) Long Terminal Repeats (LTRs) are widely used in gene therapy applications to efficiently transduce and insert therapeutic transgenes into the genome of cells from patients affected by genetic diseases. LVs with SIN LTRs have been shown to pose a lower risk of insertional mutagenesis. However, there is evidence that SIN LVs may upregulate the expression of genes flanking the integration site, especially when carrying strong enhancer/promoter sequences in internal positions or induce aberrant splicing and/or premature termination of endogenous transcripts of the targeted gene and cause loss-of-function or gain-of-function mutations (see e.g. Cesana, D., et al., 2014. Molecular therapy, 22(4), pp.774-785).

Indications of genotoxicity mediated by LV integrations have been reported in clinical trials, as dominant clones with SIN LV integrations targeting HMGA2 and other cancer-related genes were observed in the Hematopoietic Stem and Progenitor Cell (HSPC) gene therapy clinical trials for beta-thalassemia (Cavazzana-Calvo, M., et al. 2010. Nature 467, 318-322), X-linked severe immunodeficiency (De Ravin, S.S., et al. 2016. Sci Transl Med 8, 335ra357) and adrenoleukodystrophy (ALD) (Eichler, F., et al. 2017. N Engl J Med 377, 1630-1638), and in CAR-T cancer immunotherapy trials (Shah, N.N., et al. 2019. Blood Adv 3, 2317-2322). SIN LV integrations may also have induced cancer in three patients in a clinical trial for ALD (Tucci, F., et al. 2022. Nat Commun 13, 1315).

Several modifications of the LV backbone have been proposed to increase safety. These include the recoding of vector splice sites and the polyadenylation site of the LTR to avoid vector-induced aberrant splicing and read-through transcription from the integrated provirus into neighbouring genes or the inclusion of chromatin insulators to block the enhancer-mediated activation of cancer genes. However, escape mechanisms of genotoxicity, such as inactivation of tumour suppressors, may be present even when insulator sequences are used (see e.g. Cesana, D., et al., 2014. Molecular therapy, 22(4), pp.774-785).

Thus, there is a need for further polynucleotides and genetic elements for improving safety of gene therapy, gene editing and cancer immunotherapy.

### SUMMARY OF THE INVENTION

The present inventors have developed polynucleotides and genetic elements which may improve the safety of gene therapy, gene editing and cancer immunotherapy, for example by reducing the genotoxicity of lentiviral vectors comprising the same.

The present inventors have identified that the mechanisms underlying genotoxicity events can be roughly divided into three classes: (i) enhancer-mediated genotoxicity, where vector enhancer sequences activate genes surrounding the insertion sites; (ii) promoter-insertion and read-through events, driven or captured by the vector sequences leading to chimeric fusion transcripts with host sequences; and (iii) aberrant splicing promoting gene truncation upon vector insertion.

The present inventors have developed novel strategies to overcome all these insertional mutagenesis mechanisms using microRNA (miRNA) target sequences and chromatin insulators. The present inventors have surprisingly shown that vectors harbouring novel combinations of miRNA target sequences and chromatin insulators are produced efficiently and have an improved safety profile.

miRNA target sequences are DNA sequence complementary to the sequence of micro-RNA sequences triggering degradation of the transcript carrying such target sites. miRNA target sequences have been used to target transgene expression to specific target cells in gene therapy, thereby reducing immune response related to the transgene or toxic effects in off-target cells. For this purpose, miRNA target sequences are typically placed in the expression cassette, downstream from the open reading frame, upstream from the transcription termination site, and in the same orientation as the open reading frame (see e.g. Brown, B.D. and Naldini, L., 2009. Nature Reviews Genetics, 10(8), pp.578-585).

On the other hand, in the context of vector safety, the present inventors have exploited miRNA target sequences to promote the degradation of chimeric and potentially oncogenic transcript generated by fusion of vectors and host genomic RNA sequences. For this purpose, miRNA target sequences are introduced in specific orientations and configurations, e.g. (a) one or more miRNA target sequences having the same orientation as the expression cassette upstream from the expression cassette and/or downstream from the transcription termination site; and/or (b) one or more miRNA target sequences having the opposite orientation as the expression cassette upstream from the expression cassette and/or downstream from the expression cassette. The present inventors have surprisingly shown that by leveraging both antisense and sense oriented miRNA target sequences in the vector it is possible to overcome sense and antisense aberrant transcription/read-through phenomena.

Chromatin insulators are DNA sequences that allow gene expression regulation of the host genome by mediating chromatin loops that physically separate genomic regions in functional domains, where sequences within the same domain interact and regulate each other, while such crosstalk is blocked in regions from different chromatin domains. The present inventors have surprisingly shown that introducing chromatin insulators within the lentiviral backbone can reduce the interactions between the integrated vector enhancers and cellular promoters, thereby improving the safety of the vectors *in vivo.*

In one aspect, the present invention provides a polynucleotide comprising a nucleotide sequence comprising an expression cassette, wherein the nucleotide sequence further comprises:
(a) one or more miRNA target sequences having the same orientation as the expression cassette;
(b) one or more miRNA target sequences having the opposite orientation as the expression cassette; and
(c) one or more insulator elements.

Any suitable expression cassette may be used in the present invention. The expression cassette may comprise or consist of a promoter, an open reading frame, and a 3' regulatory region. The polynucleotide may further comprise a transcription termination site.

The polynucleotide may comprise the one or more miRNA target sequences having the same orientation as the expression cassette in any suitable configuration. In preferred embodiments, the nucleotide sequence comprises one or more miRNA target sequences having the same orientation as the expression cassette, upstream from the expression cassette and/or downstream from the transcription termination site. In some embodiments, the nucleotide sequence comprises two or more, three or more, or four or more miRNA target sequences having the same orientation as the expression cassette, upstream from the expression cassette and/or downstream from the transcription termination site. In some embodiments, the nucleotide sequence comprises one or more miRNA target sequences having the same orientation as the expression cassette, upstream from the expression cassette; and/or one or more miRNA target sequences having the same orientation as the expression cassette, downstream from the transcription termination site. In some embodiments, the nucleotide sequence comprises two or more miRNA target sequences having the same orientation as the expression cassette, upstream from the expression cassette, optionally wherein the two or more miRNA target sequences are two or more copies of the same miRNA target sequence arranged in tandem; and/or two or more miRNA target sequences having the same orientation as the expression cassette, downstream from the transcription termination site, optionally wherein the two or more miRNA target sequences are two or more copies of the same miRNA target sequence arranged in tandem. In some embodiments, the nucleotide sequence comprises one or more miRNA target sequences having the same orientation as the expression cassette, upstream from the expression cassette. In some embodiments, the nucleotide sequence comprises one or more miRNA target sequences having the same orientation as the expression cassette, directly upstream from the expression cassette. In some embodiments, the nucleotide sequence comprises one or more miRNA target sequences having the same orientation as the expression cassette 100 bp or less upstream from the expression cassette. In some embodiments, the nucleotide sequence comprises one or more miRNA target sequences having the same orientation as the expression cassette, downstream from the transcription termination site. In some embodiments, the nucleotide sequence comprises one or more miRNA target sequences having the same orientation as the expression cassette, directly downstream from the transcription termination site. In some embodiments, the nucleotide sequence comprises one or more miRNA target sequences having the same orientation as the expression cassette 100 bp or less downstream from the transcription termination site.

Any suitable miRNA target sequence having the same orientation as the expression cassette may be used in the present invention. In some embodiments, the one or more miRNA target sequences having the same orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, or a combination thereof. In some embodiments, the one or more miRNA target sequences having the same orientation as the expression cassette are miR142-3p target sequences. In some embodiments, the one or more miRNA target sequences having the same orientation as the expression cassette each comprise or consist of a nucleotide sequence selected from the group consisting of SEQ ID NOs: 1 to 10. In some embodiments, the one or more miRNA target sequences having the same orientation as the expression cassette each comprise or consist of the nucleotide sequence of SEQ ID NO: 1.

The polynucleotide may comprise the one or more miRNA target sequences having the opposite orientation as the expression cassette in any suitable configuration. In some embodiments, the nucleotide sequence comprises one or more miRNA target sequences having the opposite orientation as the expression cassette, upstream from the expression cassette and/or downstream from the expression cassette. In some embodiments, the nucleotide sequence comprises two or more, three or more, or four or more miRNA target sequences having the opposite orientation as the expression cassette, upstream from the expression cassette and/or downstream from the expression cassette. In some embodiments, the nucleotide sequence comprises one or more miRNA target sequences having the opposite orientation as the expression cassette, upstream from the expression cassette; and/or one or more miRNA target sequences having the opposite orientation as the expression cassette, downstream from the expression cassette. In some embodiments, the nucleotide sequence comprises two or more miRNA target sequences having the opposite orientation as the expression cassette, upstream from the expression cassette, optionally wherein the two or more miRNA target sequences are two or more copies of the same miRNA target sequence arranged in tandem; and/or two or more miRNA target sequences having the opposite orientation as the expression cassette, downstream from the expression cassette, optionally wherein the two or more miRNA target sequences are two or more copies of the same miRNA target sequence arranged in tandem. In some embodiments, the nucleotide sequence comprises one or more miRNA target sequences having the opposite orientation as the expression cassette, upstream from the expression cassette. In some embodiments, the nucleotide sequence comprises one or more miRNA target sequences having the opposite orientation as the expression cassette, directly upstream from the expression cassette. In some embodiments, the nucleotide sequence comprises one or more miRNA target sequences having the opposite orientation as the expression cassette 100 bp or less upstream from the expression cassette. In some embodiments, the nucleotide sequence comprises one or more miRNA target sequences having the opposite orientation as the expression cassette, downstream from the expression cassette. In some embodiments, the nucleotide sequence comprises one or more miRNA target sequences having the opposite orientation as the expression cassette, directly downstream from the expression cassette. In some embodiments, the nucleotide sequence comprises one or more miRNA target sequences having the opposite orientation as the expression cassette 100 bp or less downstream from the expression cassette.

Any suitable miRNA target sequence having the opposite orientation as the expression cassette may be used in the present invention. In some embodiments, the one or more miRNA target sequences having the opposite orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, or a combination thereof. In some embodiments, wherein the one or more miRNA target sequences having the opposite orientation as the expression cassette are miR223-3p target sequences. In some embodiments, the one or more miRNA target sequences having the opposite orientation as the expression cassette each comprise or consist of a nucleotide sequence selected from the group consisting of SEQ ID NOs: 11 to 20. In some embodiments, the one or more miRNA target sequences having the opposite orientation as the expression cassette each comprise or consist of the nucleotide sequence of SEQ ID NO: 12.

The polynucleotide may comprise the one or more insulator elements in any suitable configuration. In some embodiments, the nucleotide sequence comprises one or more insulator elements upstream from the expression cassette and/or downstream from the expression cassette. In some embodiments, the nucleotide sequence comprises two or more insulator elements upstream from the expression cassette and/or downstream from the expression cassette. In some embodiments, the nucleotide sequence comprises a pair of insulator elements flanking the expression cassette. In some embodiments, the pair of insulator elements is in a divergent orientation. In some embodiments, the pair of insulator elements is in a convergent orientation. In some embodiments, the pair of insulator elements is in a parallel orientation.

Any suitable insulator element may be used in the present invention. In some embodiments, the one or more insulator elements are CTCF-based insulator elements. In some embodiments, the one or more insulator elements each comprise a nucleotide sequence selected from the group consisting of SEQ ID NOs: 23 to 28. In some embodiments, the one or more insulator elements each comprise or consist of a core insulator sequence. In some embodiments, the one or more insulator elements each comprise or consist of a nucleotide sequence selected from the group consisting of SEQ ID NOs: 29 to 36. In some embodiments, the one or more insulator elements each comprise or consist of a full length insulator sequence. In some embodiments, the one or more insulator elements each comprise or consist of a nucleotide sequence selected from the group consisting of SEQ ID NOs: 37 to 90, 102 or 103, or a nucleotide sequence having at least 90% identity thereto.

In some embodiments, the nucleotide sequence further comprises a 3' LTR, optionally wherein the 3' LTR is a self-inactivating (SIN) 3' LTR.

The 3'LTR may comprise one or more of the genetic elements in any suitable configurations. By cloning the genetic elements within the 3'LTR, after reverse transcription, these genetic elements may be copied in the same orientation within the 5'LTR. In some embodiments, the 3' LTR comprises one or more miRNA target sequences having the same orientation as the expression cassette. In preferred embodiments, the 3' LTR does not comprise a miRNA target sequence having the same orientation as the expression cassette. In some embodiments, the 3' LTR comprises one or more miRNA target sequences having the opposite orientation as the expression cassette. In other embodiments, the 3' LTR does not comprise a miRNA target sequence having the opposite orientation as the expression cassette. In some embodiments, the 3' LTR comprises one or more insulator elements. In other embodiments, the 3' LTR does not comprise an insulator element.

In some embodiments, the 3' LTR does not comprise (a) one or more miRNA target sequences having the same orientation as the expression cassette; and the 3' LTR comprises (b) one or more miRNA target sequences having the opposite orientation as the expression cassette and/or (c) one or more insulator elements.

In some embodiments, the 3' LTR comprises (b) one or more miRNA target sequences having the opposite orientation as the expression cassette; and (c) one or more insulator elements.

In some embodiments, the nucleotide sequence further comprises a 5' LTR, optionally wherein the 5' LTR is a self-inactivating (SIN) 5' LTR.

The 5' LTR may comprise one or more of the genetic elements in any suitable configurations. In some embodiments, the 5' LTR comprises one or more miRNA target sequences having the same orientation as the expression cassette. In other embodiments, the 5' LTR does not comprise a miRNA target sequence having the same orientation as the expression cassette. In some embodiments, the 5' LTR comprises one or more miRNA target sequences having the opposite orientation as the expression cassette. In other embodiments, the 5' LTR does not comprise a miRNA target sequence having the opposite orientation as the expression cassette. In some embodiments, the 5' LTR comprises one or more insulator elements. In other embodiments, the 5' LTR does not comprise an insulator element.

In some embodiments, the 5' LTR does not comprise (a) one or more miRNA target sequences having the same orientation as the expression cassette; (b) one or more miRNA target sequences having the opposite orientation as the expression cassette; or (c) one or more insulator elements.

In another aspect, the present invention provides a vector comprising the polynucleotide of the present invention.

The vector may be a viral vector, a plasmid, or a transposon. In some embodiments, the vector is a viral vector selected from the group consisting of a lentiviral vector, a retroviral vector, and an adeno-associated viral (AAV) vector. In some embodiments, the vector is a retroviral vector. In preferred embodiments, the vector is a lentiviral vector.

In another aspect, the present invention provides a viral particle comprising the polynucleotide of the present invention.

The viral particle may be selected from the group consisting of a lentiviral particle, a retroviral particle, and an adeno-associated viral (AAV) particle. In some embodiments, the viral particle is a retroviral particle. In some embodiments, the viral particle is a lentiviral particle.

In another aspect, the present invention provides a retroviral vector comprising from 5' to 3' direction: a 5' LTR; one or more sense-oriented miRNA target sequences; a sense-oriented expression cassette; and a 3' LTR.

The retroviral vector may further comprise any other genetic elements for improving vector safety, such as one or more antisense-oriented miRNA target sequences and/or one or more insulator elements. In some embodiments, the retroviral vector further comprises one or more antisense-oriented miRNA target sequences. In some embodiments, the 3' LTR comprises one or more antisense-oriented miRNA target sequences. In some embodiments, the retroviral vector further comprises one or more insulator elements. In some embodiments, the 3' LTR comprises one or more insulator elements. In some embodiments, the 3' LTR comprises one or more antisense-oriented miRNA target sequences and one or more insulator elements.

In some embodiments, the retroviral vector comprises from 5' to 3' direction: a 5' LTR; one or more sense-oriented miRNA target sequences; a sense-oriented expression cassette; and a 3' LTR, wherein the 3' LTR comprises from 5' to 3' direction: a first insulator element, one or more antisense-oriented miRNA target sequences, and a second insulator element, wherein the first insulator element and the second insulator element are in a divergent orientation. In some embodiments, the first insulator element is an A2 insulator element and the second insulator element is an E2 insulator element. In some embodiments, the first insulator element and the second insulator element each comprise or consist of a full length insulator sequence. In some embodiments, the first insulator element comprises or consists of the nucleotide sequence of SEQ ID NO: 103, or a nucleotide sequence having at least 90% identity thereto, and/or the second insulator element comprises or consists of the nucleotide sequence of SEQ ID NO: 55, or a nucleotide sequence having at least 90% identity thereto. In some embodiments, the first insulator element and the second insulator element each consist of a core insulator sequence. In some embodiments, the first insulator element comprises or consists of the nucleotide sequence of SEQ ID NO: 34 and/or the second insulator element comprises or consists of the nucleotide sequence of SEQ ID NO: 32.

In other embodiments, the retroviral vector comprises from 5' to 3' direction: a 5' LTR; one or more sense-oriented miRNA target sequences; a sense-oriented expression cassette; a first insulator element; and a 3' LTR, wherein the 3' LTR comprises from 5' to 3' direction: one or more antisense-oriented miRNA target sequences, and a second insulator element, wherein the first insulator element and the second insulator element are in a divergent orientation. In some embodiments, the first insulator element is an A2 insulator element and the second insulator element is an E2 insulator element. In some embodiments, the first insulator element comprises or consists of the nucleotide sequence of SEQ ID NO: 103, or a nucleotide sequence having at least 90% identity thereto, and/or the second insulator element comprises or consists of the nucleotide sequence of SEQ ID NO: 55, or a nucleotide sequence having at least 90% identity thereto.

In other embodiments, the retroviral vector comprises from 5' to 3' direction: a 5' LTR; one or more sense-oriented miRNA target sequences; a sense-oriented expression cassette; and a 3' LTR, wherein the 3' LTR comprises from 5' to 3' direction: one or more insulator elements and one or more antisense-oriented miRNA target sequences. In some embodiments, the one or more insulator elements consist of an A1 insulator element and an A2 insulator element in a parallel orientation, wherein the A1 insulator element and the A2 insulator element each comprise or consist of a full length insulator sequence. In some embodiments, the A1 insulator element comprises or consists of the nucleotide sequence of SEQ ID NO: 64, or a nucleotide sequence having at least 90% identity thereto, and/or the A2 insulator element comprises or consists of the nucleotide sequence of SEQ ID NO: 103, or a nucleotide sequence having at least 90% identity thereto. In other embodiments, the one or more insulator elements consist of an A1 insulator element and an A2 insulator element in a parallel orientation, wherein the A1 insulator element and the A2 insulator element each consist of a core insulator sequence. In some embodiments, the A1 insulator element comprises or consists of the nucleotide sequence of SEQ ID NO: 33 and/or the A2 insulator element comprises or consists of the nucleotide sequence of SEQ ID NO: 34. In other embodiments, the one or more insulator elements consist of an A2 insulator element, wherein the A2 insulator element comprises or consists of a full length insulator sequence. In some embodiments, the A2 insulator element comprises or consists of the nucleotide sequence of SEQ ID NO: 103, or a nucleotide sequence having at least 90% identity thereto. In other embodiments, the one or more insulator elements consist of an A2 insulator element, wherein the A2 insulator element consists of a core insulator sequence. In some embodiments, wherein the A2 insulator element comprises or consists of the nucleotide sequence of SEQ ID NO: 34. In other embodiments, the one or more insulator elements consist of an A1 insulator element, wherein the A1 insulator element consists of a core insulator sequence. In some embodiments, wherein the A1 insulator element comprises or consists of the nucleotide sequence of SEQ ID NO: 33. In other embodiments, the one or more insulator elements consist of a B2 insulator element, wherein the B2 insulator element consists of a core insulator sequence. In some embodiments, wherein the A2 insulator element comprises or consists of the nucleotide sequence of SEQ ID NO: 35.

In another aspect, the present invention provides a retroviral vector comprising from 5' to 3' direction: a 5' LTR; an antisense-oriented expression cassette; and a 3' LTR, wherein the retroviral vector further comprises:
(a) one or more antisense-oriented miRNA target sequences;
(b) one or more sense-oriented miRNA target sequences; and
(c) one or more insulator elements.

In preferred embodiments, the retroviral vector comprises one or more antisense-oriented miRNA target sequences upstream from the expression cassette and/or downstream from the expression cassette. In some embodiments, the retroviral vector comprises one or more sense-oriented miRNA target sequences upstream from the expression cassette and/or downstream from the expression cassette; and/or one or more insulator elements, upstream from the expression cassette and/or downstream from the expression cassette. In some embodiments, the 3' LTR does not comprise one or more antisense-oriented miRNA target sequences. In some embodiments, the 3' LTR does not comprise one or more sense-oriented miRNA target sequences.

Any suitable sense-oriented or antisense-oriented miRNA target sequence may be used in the present invention. In some embodiments, the one or more sense-oriented miRNA target sequences comprise or consist of two or more miR142-3p target sequences. In some embodiments, the one or more sense-oriented miRNA target sequences comprise or consist of the nucleotide sequence of SEQ ID NO: 21. In some embodiments, the one or more antisense-oriented miRNA target sequences comprise or consist of two or more miR223-3p target sequences. In some embodiments, the one or more antisense-oriented miRNA target sequences comprise or consist of the nucleotide sequence of SEQ ID NO: 22.

The retroviral vector of the present invention may be derived from any type of retroviral vector. In preferred embodiments, the retroviral vector is a lentiviral vector. In some embodiments, the lentiviral vector is a self-inactivating lentiviral vector. In some embodiments, the 3' LTR is a self-inactivating (SIN) 3' LTR. In some embodiments, the lentiviral vector is an integration-proficient lentiviral vector (IPLV).

The retroviral vector of the present invention may have improved safety (e.g. reduced toxicity). The retroviral vector may be substantially non-genotoxic. The one or more sense-oriented miRNA target sequences and the one or more antisense-oriented miRNA target sequences may avoid and/or reduce aberrant splicing and/or read-through events. The one or more insulator elements may avoid and/or reduce enhancer-mediated gene activation.

In another aspect, the present invention provides a cell comprising the polynucleotide of the present invention, the vector of the present invention, the viral particle of the present invention, or the retroviral vector of the present invention.

In some embodiments, the cell is a hematopoietic stem and/or progenitor cell (HSPC).

In another aspect, the present invention provides a pharmaceutical composition comprising the polynucleotide of the present invention, the vector of the present invention, the viral particle of the present invention, the retroviral vector of the present invention, or the cell of the present invention, in combination with a pharmaceutically acceptable carrier, diluent or excipient.

In another aspect, the present invention provides the polynucleotide of the present invention, the vector of the present invention, the viral particle of the present invention, the retroviral vector of the present invention, the cell of the present invention, or the pharmaceutical composition of the present invention, for use as a medicament.

In another aspect, the present invention provides a method of reducing the genotoxicity of a polynucleotide or vector comprising a nucleotide sequence comprising an expression cassette and a transcription termination site, wherein the method comprises introducing into the nucleotide sequence: (a) one or more miRNA target sequences having the same orientation as the expression cassette; (b) one or more miRNA target sequences having the opposite orientation as the expression cassette; and (c) one or more insulator elements.

In some embodiments, the one or more miRNA target sequences having the same orientation as the expression cassette are introduced upstream from the expression cassette and/or downstream from the transcription termination site.

In another aspect, the present invention provides a method of reducing the genotoxicity of a retroviral vector comprising a sense-oriented expression cassette, wherein the method comprises introducing into the retroviral vector: (a) one or more sense-oriented miRNA target sequences upstream from the sense-oriented expression cassette.

In some embodiments, the method further comprises introducing into the retroviral vector: (b) one or more antisense-oriented miRNA target sequences upstream from the sense-oriented expression cassette and/or downstream from the sense-oriented expression cassette; and/or (c) one or more insulator elements upstream from the sense-oriented expression cassette and/or downstream from the sense-oriented expression cassette.

In another aspect, the present invention provides a method of reducing the genotoxicity of a retroviral vector comprising an antisense-oriented expression cassette, wherein the method comprises introducing into the retroviral vector: (a) one or more antisense-oriented miRNA target sequences upstream from the antisense-oriented expression cassette and/or downstream from the antisense-oriented expression cassette; (b) one or more sense-oriented miRNA target sequences upstream from the antisense-oriented expression cassette and/or downstream from the antisense-oriented expression cassette; and (c) one or more insulator elements upstream from the antisense-oriented expression cassette and/or downstream from the antisense-oriented expression cassette.

In another aspect, the present invention provides an insulator element comprising or consisting of a sequence having at least 95% sequence identity to SEQ ID NO: 103, wherein the insulator element comprises the nucleotides G and C at positions corresponding to nucleotides 45 and 46, respectively, wherein the nucleotides are numbered with reference to SEQ ID NO: 103. In some embodiments, the insulator element comprises SEQ ID NO: 34. In some embodiments, the insulator element comprises or consists of SEQ ID NO: 103.

In another aspect, the present invention provides an insulator element comprising or consisting of a sequence having at least 95% sequence identity to SEQ ID NO: 102, wherein the insulator element comprises the nucleotides G and C at positions corresponding to nucleotides 221 and 222, respectively, wherein the nucleotides are numbered with reference to SEQ ID NO: 102. In some embodiments, the insulator element comprises SEQ ID NO: 30. In some embodiments, the insulator element comprises or consists of SEQ ID NO: 102.

In another aspect, the present invention provides a polynucleotide comprising a nucleotide sequence comprising the insulator element of the present invention. In some embodiments, the nucleotide sequence further comprises an expression cassette and the insulator element flanks the expression cassette. In another aspect, the present invention provides a vector comprising the polynucleotide. In another aspect, the present invention provides a cell comprising the polynucleotide or the vector.

In another aspect, the present invention provides a self-inactivating (SIN) 3' LTR comprising one or more antisense-oriented miRNA target sequences and one or more insulator elements. In another aspect, the present invention provides a self-inactivating (SIN) 3' LTR comprising or consisting of a nucleotide sequence having at least 90% sequence identity to a nucleotide sequence selected from the group consisting of SEQ ID NOs: 104 to 112.

In some embodiments, the SIN 3' LTR comprises an antisense-oriented miRNA target sequence comprising or consisting of the nucleotide sequence of SEQ ID NO: 22. In some embodiments, the SIN 3' LTR comprises an insulator element comprising or consisting of a nucleotide sequence selected from the group consisting of SEQ ID NOs: 29 to 36.

In another aspect, the present invention provides a polynucleotide comprising a nucleotide sequence comprising the SIN 3' LTR of the present invention. In some embodiments, the nucleotide sequence further comprises an expression cassette. In another aspect, the present invention provides a vector comprising the polynucleotide. In another aspect, the present invention provides a cell comprising the polynucleotide or the vector.

### DESCRIPTION OF DRAWINGS

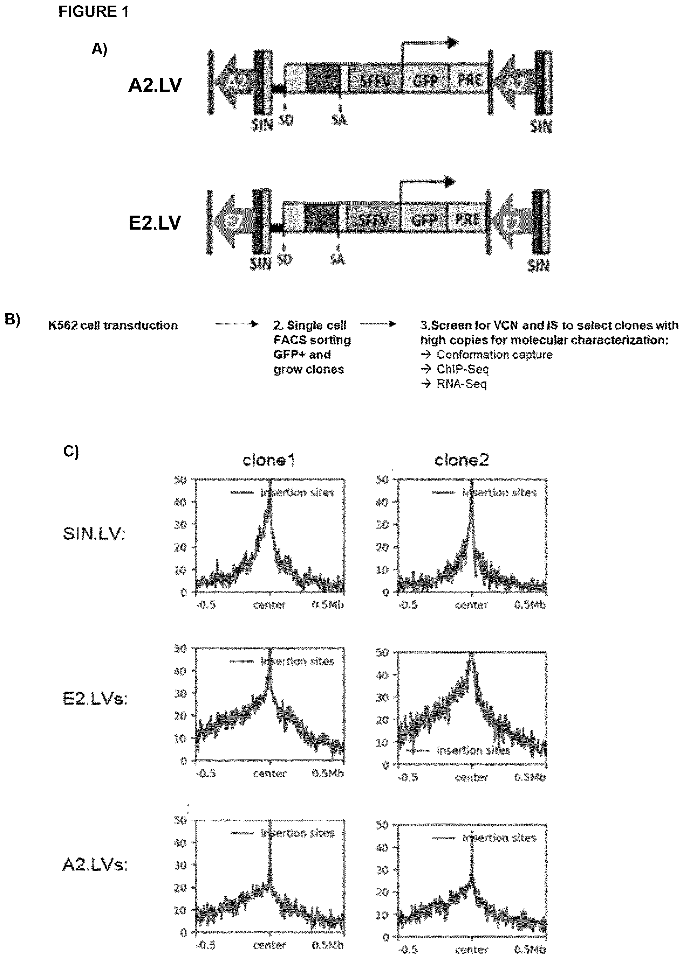

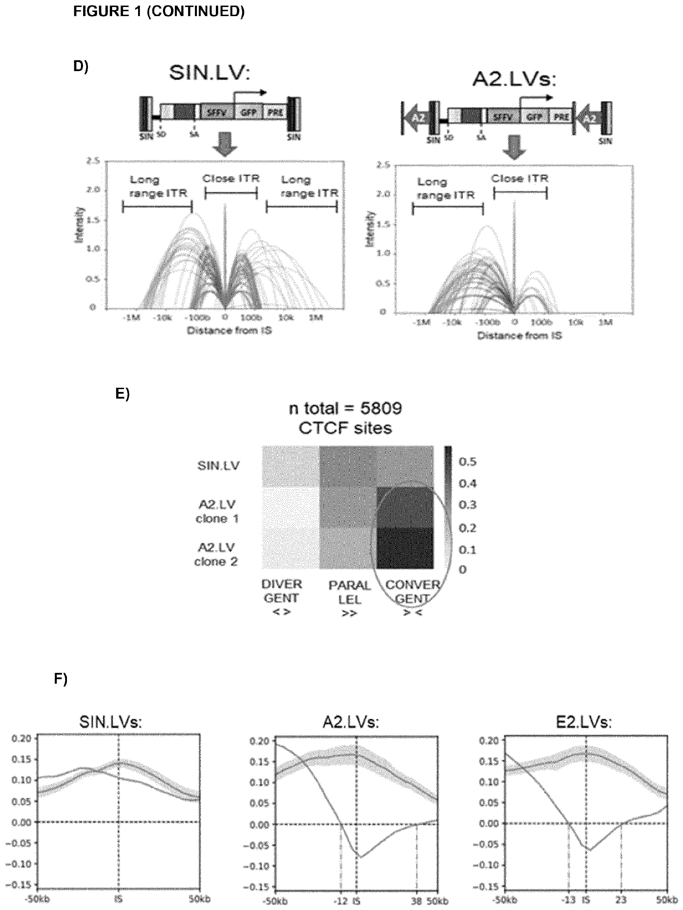

**Figure 1** **- Generation of insulator-harbouring SIN.LVs and *in vitro* characterization**
   **A)** Schematic diagram of SIN.LV vectors comprising chromatin insulators: SIN, self-inactivating LTR; SD, splice donor; SA, splice acceptor; SFFV, SFFV enhancer/promoter; GFP, green fluorescent protein; PRE, posttranscriptional regulatory element; A2, A2 chromatin insulator; E2, E2 chromatin insulator. **B)** Experimental scheme to generate K562 transduced clones and molecular characterisation. **C)** Analysis of LV-mediated interactions with genomic loci ± 0.5Mb from centre. All LV sequences are oriented 5' to 3'. Two independent clones per vector constructs are shown. **D)** LV4C derived interactions (arches) are show for SIN LV (left) and A2 insulated LV (right). **E)** CTCF-enrichment analysis at the LV/genome interaction site show an enrichment of genomic CTCF sites in convergent orientation with the A2 insulator contained in the LV LTR. **F)** Insulation boundary analysis for the different vector types is shown. X-axis indicates distance in Kb; Y-axis indicates the insulation score; a single line indicates the interaction skewing as readout of insulation; the line with confidence limits indicates the distribution at sites without insertion sites.

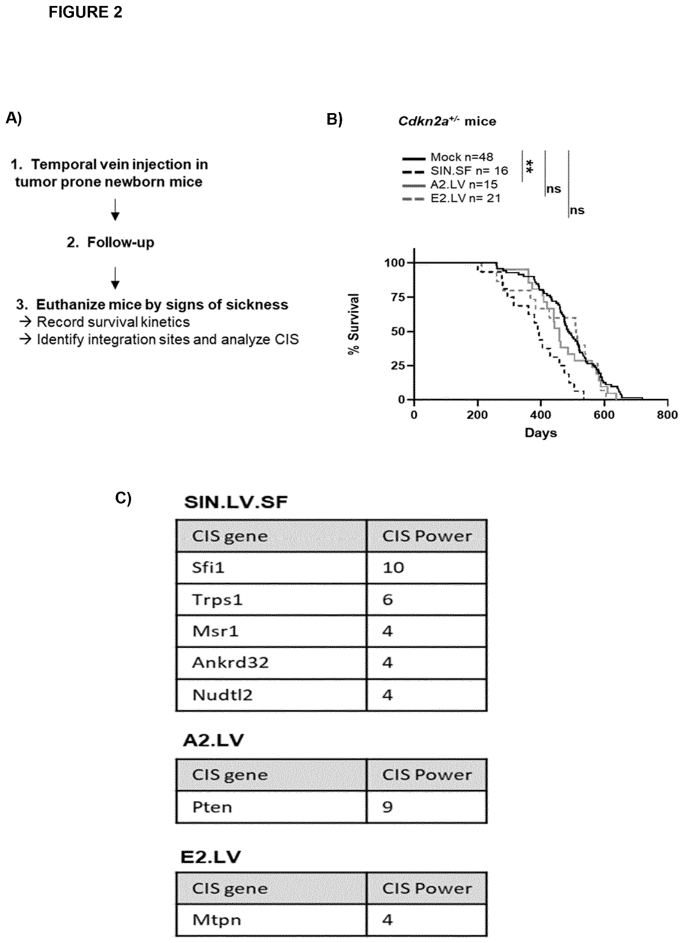

**Figure 2** ***- In vivo* safety study of CI-harbouring SIN.LVs in tumour prone mice**
   **A)** Experimental scheme of the *in vivo* genotoxicity assays with tumour prone *Cdkn2a*^{*+*/*-*} mice. **B)** Survival kinetics of the *Cdkn2a*^{+/*-*} mice treated with SIN.LV (SIN.SF); A2-insulated SIN.LV (A2.LV); E2-insulated SIN.LV (E2.LV) and untreated Mock-controls. **C)** Common Integration Sites (CIS) of the different treatment groups in *Cdkn2a*^{+/-} mice with a CIS power ≥4 are shown.

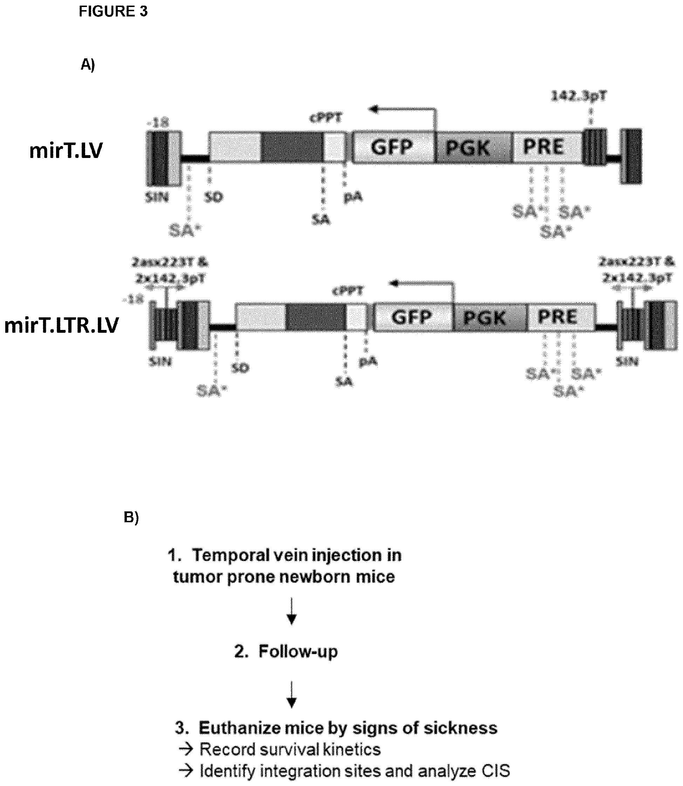

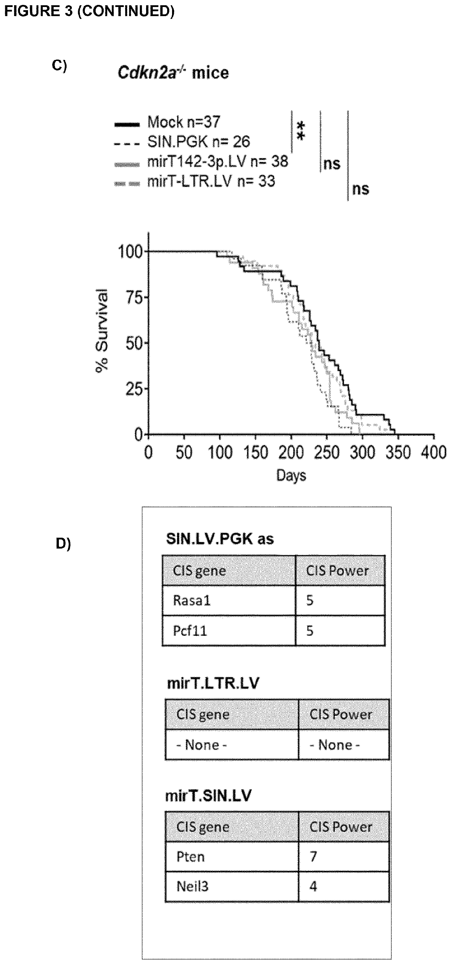

**Figure 3** ***- In vivo* safety evaluation of SIN.LVs with reduced aberrant-splicing/read-through potential**
   **A)** Schematic diagram of miRT-containing SIN.LVs, mirT.LV and mirT.LTR.LV: SIN, self-inactivating LTR; SD, splice donor; SA, splice acceptor; cPPT, central polypurine tract; pA, polyadenylation sequence; GFP, green fluorescent protein; PGK, PGK enhancer-promoter; PRE, posttranscriptional regulatory element; 142.3pT, miR142-3p target sequence; 223T, miR223 target sequence. **B)** Experimental scheme of the *in vivo* genotoxicity assays with tumour prone *Cdkn2a*^{-/-} mice. **C)** Survival kinetics of the *Cdkn2a*^{-/-} mice treated with SIN.PGK; mirT.LV; mirT.LTR.LV and untreated Mock-controls. **D)** Common Integration Sites (CIS) of the different treatment groups in Cdkn2a^{-/-} mice. CIS genes with power ≥4 are shown.

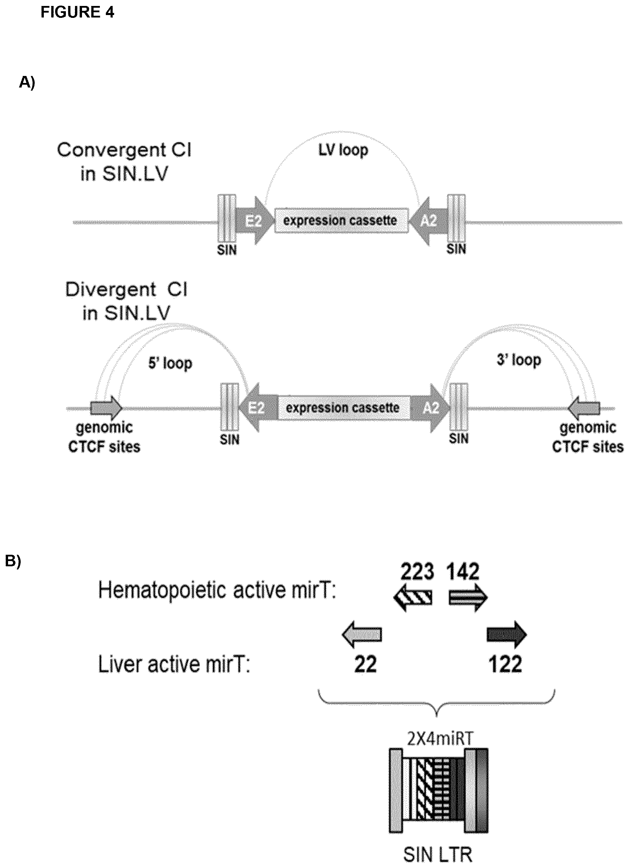

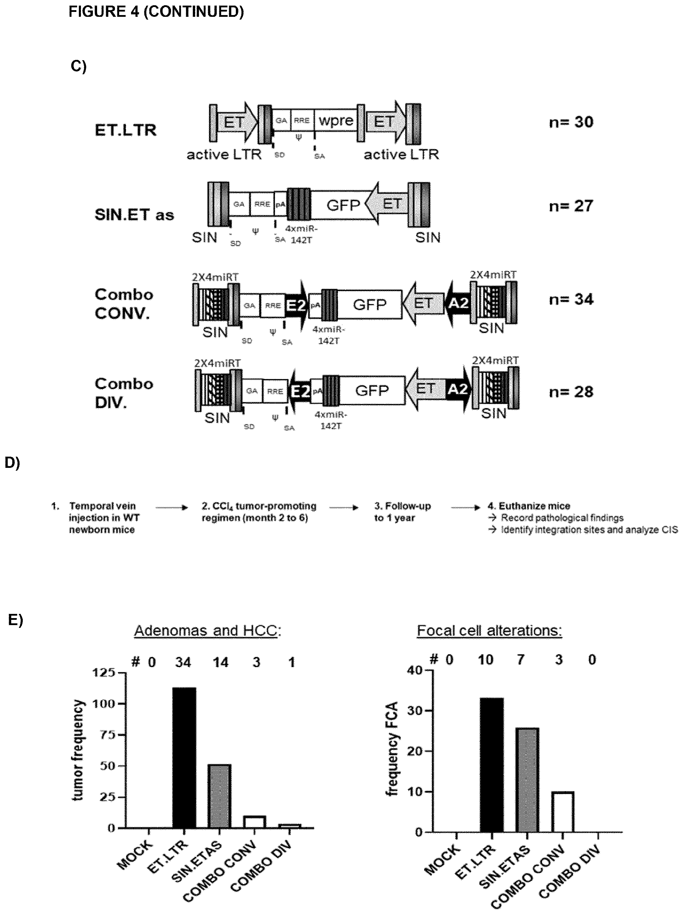

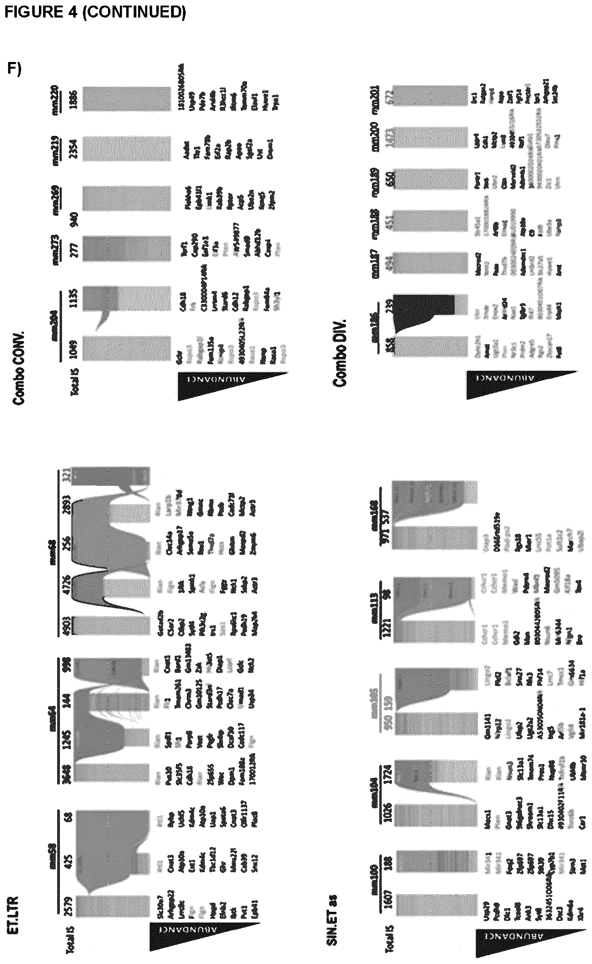

**Figure 4** **- Combination of safety-features to enhance vector safety**
   **A)** Schematic representation of the chromatin loops and insulation regulation by the convergent (CONV) or divergent (DIV) insulator configurations. **B)** Schematic representation of the inclusion of different micro-RNA-target sites (miRT) in the vectors. Two tandem copies of each of the hematopoietic- or liver-specific miRTs are included in the LTRs. Arrows indicate sense and antisense orientation of the miRT comparted to vector backbone transcription. **C)** Schematic diagram of control and Cl- and miRT-containing SIN.LVs, ET.LTR, SIN.ETas, Combo CONV, and Combo DIV: SIN, self-inactivating LTR; ET, ET promoter; SD, splice donor; SA, splice acceptor; GA, truncated gag sequence; RRE, Rev responsive element; WPRE, woodchuck posttranscriptional regulatory element; pA, polyadenylation sequence; GFP, green fluorescent protein; miR142T, miR142 target sequence; A2, A2 chromatin insulator; E2, E2 chromatin insulator. **D)** Experimental scheme of the *in vivo* assay with WT mice treated with cancer-promoting regimen to evaluate the safety of the different test vectors. **E)** Results from histopathology analysis in liver from mice treated with the different vectors. Bars indicate the event frequency calculated as total occurrences per number of mice composing the treatment group. Numbers on top of the bars indicate the absolute number of events. **F)** Integration sites analysis and abundance analysis from healthy liver tissues (first column) and tumours (following columns) of the vector-treated mice. Stacked bars indicate vector integration sites. IS are sorted from most abundant to least abundant integration. Gene list highlights the top abundant IS. Genes known to be genotoxic or that could potentially induce transformation are highlighted. Mouse ID and the total number of insertions is shown at the top of the plot.

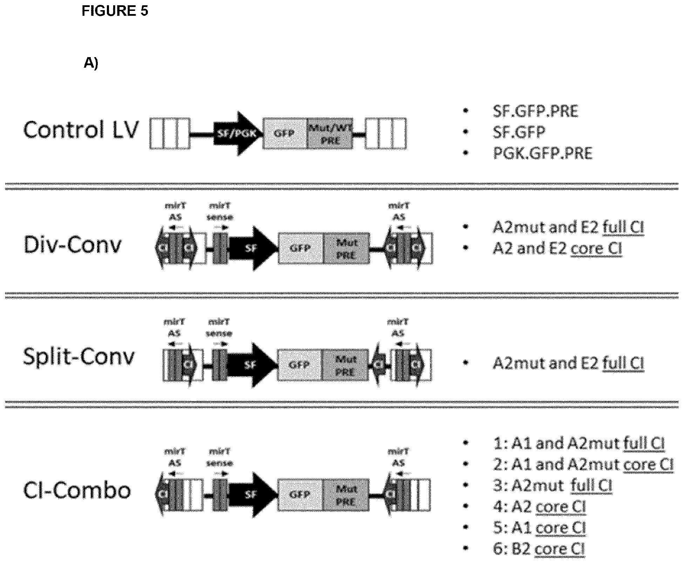

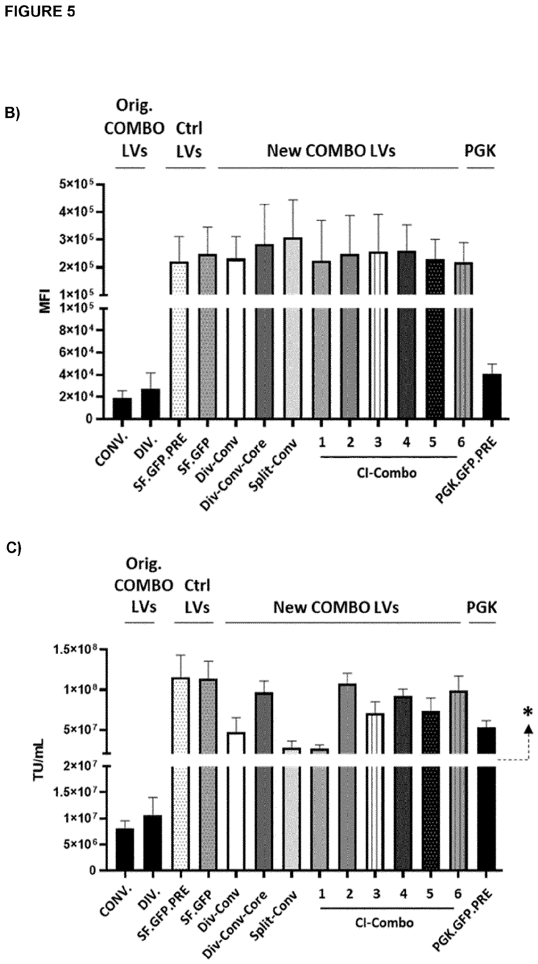

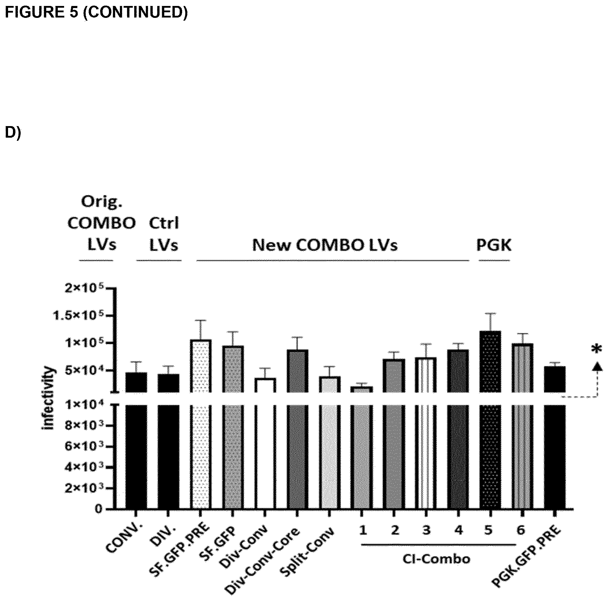

**Figure 5** **- Optimisation of vector designs comprising a combination of safety-features**
   **A)** Schematic diagram of the COMBO-LV designs and control vector tested in the *in vitro* assay: SF, SF enhancer-promoter; GFP, green fluorescent protein; PRE, posttranscriptional regulatory element; Cl, chromatin enhancer; mirT AS, antisense-oriented miRNA target sequence; mirT sense, sense-oriented miRNA target sequence. Control LV is a SIN.LV with SF enhancer-promoter, GFP and mutated PRE. All test vectors contain the novel positioning of hematopoietic-active miRTs (223 and 142-3p) in the LV backbone and a combination of insulator elements. The Div.Conv LV contains E2 and A2mut Cl in divergent and convergent orientations in the LTRs; the Div.Conv.Core LV contains A2 and E2 core sequences in divergent and convergent orientation cloned in the LTR; the Split.Conv LV displays a single internal copy of A2mut and two copies of E2 in the two LTRs. A1.A2 insulator combination placed in parallel orientation in the LTR were tested in full length or core versions. Individual core insulators A1, A2 and B2 were also tested in combination with mirTs. B) Mean fluorescence intensity (MFI) measured at FACS in technical triplicates for each vector preparation for and for each construct. **C)** Non-concentrated vector titre assayed for all tested vectors and controls. **D)** Vector infectivity measured for all test vectors and controls. The "*" indicates a threshold for good-quality lab-grade vector production performances.

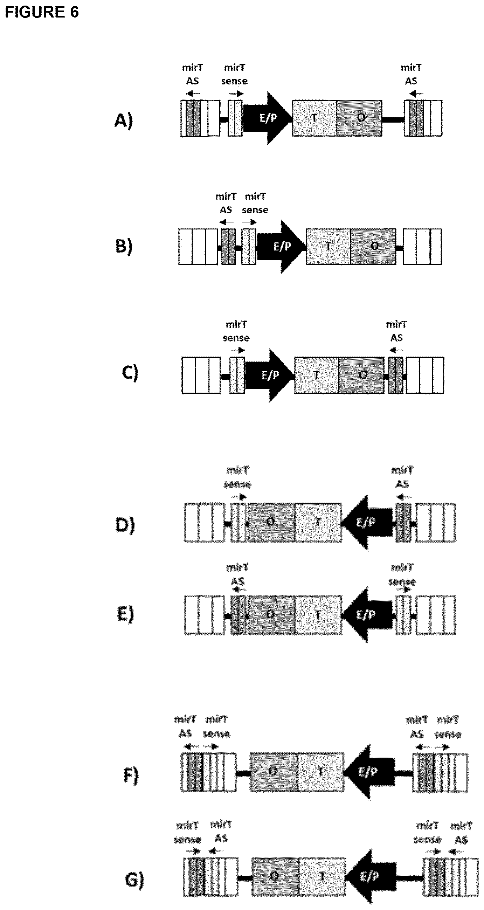

**Figure 6** **- Example configurations of miRNA target sequences (mirT) in lentiviral vector (LV) proviruses**
   **A-C)** For sense-oriented expression cassettes in LVs. Sense-oriented mirT (yellow in scheme): preferred embodiment upstream the expression cassette. Antisense-oriented mirT: no preferred embodiment, can be placed in the LTRs, upstream and/or downstream the expression cassette. **D-G)** For antisense-oriented expression cassettes in LVs. Sense-oriented mirT: no preferred embodiment, can be placed in the LTRs, upstream and/or downstream the expression cassette, in single or combination (convergent and divergent orientation). Antisense-oriented mirT: no preferred embodiment, can be placed in the LTRs, upstream and/or downstream the expression cassette, in single or combination (convergent and divergent orientation). **Figure legend:** E/P: enhancer/promoter; T: transgene; O: other (e.g. WPRE site and/or any other transcriptional and/or post-transcriptional regulatory element); mirT AS: antisense oriented micro-RNA target sites; mirT sense: sense oriented micro-RNA target sites.

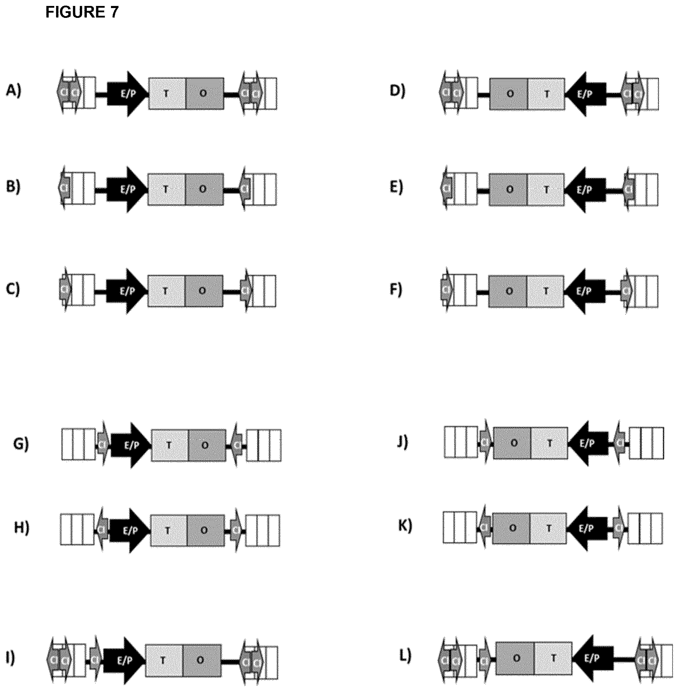

**Figure 7** **- Example configurations of insulator elements in lentiviral vector (LV) proviruses**
   **A-F)** Chromatin insulators within the LTRs for sense- and antisense- oriented expression cassettes. Embodiment within the LTR in combination (preferred embodiment divergent or parallel orientation) or single elements both in sense or antisense orientations. **G-H and J-K)** Chromatin insulators within the backbone for sense- and antisense- oriented expression cassettes. Within the LV backbone preferred embodiment upstream and downstream the expression cassette, in divergent and/or convergent orientation. Embodiment in parallel orientation is also possible. **I, L)** Insulators within LTRs and backbone for sense- and antisense- oriented expression cassettes. Within the LTR or backbone as described above. Preferred embodiment divergent in LTR, sense and/or antisense in the backbone, both upstream and/or downstream the expression cassette is possible. **Figure legend:** E/P: enhancer/promoter; T: transgene; O: other (e.g. WPRE site, polyA site and/ or any other transcriptional and/or post-transcriptional regulatory element); Cl: Chromatin Insulator.

### DETAILED DESCRIPTION

Various preferred features and embodiments of the present invention will now be described by way of non-limiting examples. This disclosure is not limited by the exemplary methods and materials disclosed herein, and any methods and materials similar or equivalent to those described herein can be used in the practice or testing of embodiments of this disclosure.

The skilled person will understand that they can combine all features of the invention disclosed herein without departing from the scope of the invention as disclosed.

It must be noted that as used herein and in the appended claims, the singular forms "a", "an", and "the" include plural referents unless the context clearly dictates otherwise.

The terms "comprising", "comprises" and "comprised of" as used herein are synonymous with "including", "includes", "containing", or "contains", and are inclusive or open-ended and do not exclude additional, non-recited members, elements or steps. The terms "comprising", "comprises" and "comprised of' also include the term "consisting of".

Numeric ranges are inclusive of the numbers defining the range. As used herein the term "about" means approximately, in the region of, roughly, or around. Unless otherwise indicated, any nucleic acid sequences are written left to right in 5' to 3' orientation and amino acid sequences are written left to right in amino to carboxy orientation, respectively.

The publications discussed herein are provided solely for their disclosure prior to the filing date of the present application. Nothing herein is to be construed as an admission that such publications constitute prior art to the claims appended hereto. All publications mentioned in the specification are herein incorporated by reference.

### Polynucleotides

In one aspect, the present invention provides a polynucleotide comprising an expression cassette, wherein the polynucleotide further comprises:
(a) one or more miRNA target sequences having the same orientation as the expression cassette;
(b) one or more miRNA target sequences having the opposite orientation as the expression cassette; and/or
(c) one or more insulator elements.

The polynucleotide of the present invention may be an isolated polynucleotide. The polynucleotide of the present invention may be double or single stranded. The polynucleotide of the invention may comprise or consist of DNA and/or RNA. In some embodiments, the polynucleotide is a DNA polynucleotide. In some embodiments, the polynucleotide is a RNA polynucleotide.

The directionality of the polynucleotide may be used to define the orientation of the nucleotide sequences comprised therein. A DNA polynucleotide has one strand in the 5' to 3' direction with respect to the carbon atoms on the deoxyribose (referred to herein as the "sense" strand) and one complementary strand in the 3' to 5' direction (referred to herein as the "anti-sense strand). A RNA polynucleotide has one strand is in the 5' to 3' direction with respect to the carbon atoms on the ribose.

### Expression cassettes

The polynucleotide of the present invention comprises an expression cassette.

An "expression cassette" may refer to a nucleotide sequence consisting of a gene and one or more regulatory sequences controlling the expression of the gene. An expression cassette typically comprises a promoter or enhancer-promoter, an open reading frame, and a 3' regulatory region.

An expression cassette is transcribed from DNA from 5' to 3'. The orientation of the expression cassette may be used to define the relative orientation of the other elements. In some embodiments, an expression cassette comprises or consists of from 5' to 3' direction: a promoter or enhancer-promoter, an open reading frame, and a 3' regulatory region.

An expression cassette may be sense-oriented or antisense-oriented. In the context of a DNA polynucleotide, a "sense-oriented" expression cassette is transcribed from the sense strand. In the context of a DNA polynucleotide, an "antisense-oriented" expression cassette is transcribed from the antisense strand.

### Promoters and enhancers

A "promoter" may refer to a nucleotide sequence that leads to initiation of transcription of a gene. Promoters are located near the transcription start sites of genes, upstream from the gene. Any promoter may be used in the present invention, the selection of which may be readily made by the skilled person.

In some embodiments, the promoter is a constitutive promoter. A "constitutive promoter" may refer to a promoter which is always active. Suitable constitutive promoters will be known to the skilled person. Example constitutive promoters include the CMV promoter, the EF1a promoter, the CAG promoter, the PGK promoter, the U6 promoter, the T7 promoter, the SV40 promoter, and the Sp6 promoter.

In some embodiments, the promoter is an inducible promoter. An "inducible promoter" may refer to a promoter which is activated in response to specific stimuli (e.g. in response to chemicals, temperature, or light). Suitable inducible promoters will be known to the skilled person. Example inducible promoters include the TRE promoter, the lac promoter, the araBad promoter.

In some embodiments, the promoter is a repressible promoter. A "repressible promoter" may refer to a promoter which is de-activated in response to specific stimuli. Suitable repressible promoters will be known to the skilled person. Example repressible promoters include the trp promoter.

In some embodiments, the promoter is a ubiquitous promoter. As used herein, a "ubiquitous promoter" is a promoter which is active in a wide range of cells and tissues. Suitable ubiquitous promoters will be known to the skilled person and include, e.g. Phosphoglycerate kinase (PGK) promoter, Cytomegalovirus (CMV) promoter, EF1alpha promoter (short), EF1alpha promoter (long), Ubiquitin C (UbiC) promoter, Simian Virus 40 (SV40) enhancer/promoter, U6 promoter, H1 promoter, CAG promoter (CMV enhancer/chicken beta-actin promoter), or comprises a TRE (tetracyclin response element). In some embodiments, the promoter is a PGK promoter.

In some embodiments, the promoter is a tissue-specific promoter. A "tissue-specific promoter" may refer to a promoter which preferentially facilitates expression of a gene in a specific cell and/or tissue. Suitable tissue-specific promoters will be known to the skilled person. In some embodiments, the promoter is a hematopoietic-specific promoter, erythroid-specific promoter, liver-specific promoter, or neuron-specific promoter.

In some embodiments, the promoter is a hematopoietic-specific promoter. In some embodiments, the promoter comprises Universal chromatin opener element (UCOE) enhancer. In some embodiments, the promoter is a Gp91 phox promoter, Wiskott Aldrich-Syndrome (WAS) promoter, Murine-leukemia virus derived MND promoter, Spleen Focus Forming Virus (SFFV) enhancer/promoter, or Tyrosine protein kinase receptor (Tie2) promoter. In some embodiments, the promoter is a SFFV enhancer/promoter.

In some embodiments, the promoter is an erythroid-specific promoter. In some embodiments, the promoter comprises β globin LCR HS2 and HS3 or β globin LCR HS2, HS3, and HS4
In some embodiments, the promoter is a liver-specific promoter. In some embodiments, the promoter is an Enhanced Transthyretin (ET) enhancer/promoter, Albumin promoter (Alb) promoter, or Apolipoprotein (Apo) promoter. In some embodiments, the promoter is an ET promoter.

In some embodiments, the promoter is a neuron-specific promoter. In some embodiments, the promoter is a Nestin promoter or CaMKlla promoter.

In some embodiments, the promoter is a strong promoter. As used herein, a "strong promoter" may refer to a promoter which facilitates high expression of a protein-coding sequence. Genes regulated by strong promoters yield more mRNA and therefore more product protein than genes regulated by weak promoters. Example strong promoters include the spleen focus forming virus (SFFV) promoter.

In some embodiments, the promoter is a moderate promoter. As used herein, a "moderate promoter" may refer to a promoter which facilitates moderate expression of a protein-coding sequence. Example moderate promoters include the PGK promoter.

In some embodiments, the promoter is a viral promoter, e.g. a strong viral promoter. As used herein, a "viral promoter" may refer to a promoter derived from a viral genome. In some embodiments, the promoter is a cellular promoter. As used herein, a "cellular promoter" may refer to a promoter derived from a cell. In some embodiments, the promoter is a bacterial promoter. In some embodiments, the promoter is a eukaryotic promoter. In some embodiments, the promoter is a mammalian promoter.

In some embodiments, the polynucleotide further comprises an enhancer. An "enhancer" may refer to a nucleotide sequence that can be bound by activators to enhance transcription. Enhancers are cis-acting and can be located up to 1 Mbp (1,000,000 bp) away from the promoter, upstream or downstream from the start site. Suitably, the enhancer is upstream from the promoter. Any suitable enhancer may be used, the selection of which may be readily made by the skilled person.

In some embodiments, an expression cassette comprises an enhancer-promoter. In some embodiments, an expression cassette comprises or consists of from 5' to 3' direction: an enhancer-promoter, an open reading frame, and a 3' regulatory region.

In some embodiments, the enhancer-promoter is a strong enhancer-promoter. Example strong enhancer-promoters include the spleen focus forming virus (SFFV) enhancer-promoter. In some embodiments, the enhancer-promoter is a moderate enhancer-promoter. Example moderate enhancer-promoters include the PGK enhancer-promoter. In some embodiments, the enhancer-promoter is a viral enhancer-promoter, e.g. a strong viral enhancer-promoter. In some embodiments, the promoter is a cellular enhancer-promoter. In some embodiments, the promoter is a bacterial enhancer-promoter. In some embodiments, the promoter is a eukaryotic enhancer-promoter. In some embodiments, the promoter is a mammalian enhancer-promoter.

### 5' regulatory region

An expression cassette may further comprise a 5' regulatory region. In some embodiments, an expression cassette comprises or consists of from 5' to 3' direction: a promoter or enhancer-promoter, a 5' regulatory region, an open reading frame, and a 3' regulatory region.

A "5' regulatory region" may refer to a nucleotide sequence that is downstream from the transcription start site and upstream from the start codon which controls expression of the open reading frame. The 5' regulatory region is typically not translated, but forms a complex secondary structure in the mRNA to regulate translation. Any 5' regulatory region may be used, the selection of which may be readily made by the skilled person.

A prokaryotic 5' regulatory region can contains a ribosome binding site (RBS), for example having the sequence AGGAGGT, which is usually 3-10 base pairs upstream from the initiation codon. A eukaryotic 5' regulatory region usually contains a Kozak sequence, for example having the sequence ACCATGG, which contains the initiation codon. A eukaryotic 5' regulatory region may also contain cis-acting regulatory elements.

### Open reading frame

An "open reading frame" may refer to a nucleotide sequence between a start codon (e.g. ATG) and a stop codon (e.g. TAA, TAG, or TGA). An open reading frame typically encodes a polypeptide (i.e. a protein-coding sequence) or a functional RNA (i.e. a RNA-coding sequence). Any open reading frame may be used, the selection of which may be readily made by the skilled person.

In some embodiments, the open reading frame is a protein-coding sequence. A "protein-coding sequence" or "transgene" may encode any polypeptide of interest. In some embodiments, the polypeptide is a therapeutic polypeptide. A protein-coding sequence may further encode a signal peptide and/or a protein tag. For example, a protein-coding sequence may encode from 5' to 3' direction: optionally a signal peptide; a polypeptide; and optionally a protein tag. Suitable signal peptides and protein tags will be known to the skilled person.

For a protein-coding polynucleotide, numerous different polynucleotides can encode the same polypeptide as a result of the degeneracy of the genetic code. For example, Nucleotide substitutions may be made that do not affect the polypeptide sequence encoded by the protein coding sequence to reflect the codon usage of any particular host organism in which the polypeptides of the invention are to be expressed.

The protein-coding sequence may be codon-optimised. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells (e.g. humans), as well as for a variety of other organisms.

In some embodiments, the open reading frame is a RNA-coding sequence. A "RNA-coding sequence" may encode any RNA of interest. For example, a miRNA, a siRNA, a piRNA, a snoRNA, a snRNA, an exRNA, a scaRNA, a transfer RNA (tRNA), a small hairpin RNA (shRNA), or a ribosomal RNA (rRNA).

### 3' regulatory region

A "3' regulatory region" may refer to a nucleotide sequence that is downstream from the stop codon and upstream from the transcription termination which controls expression of the open reading frame. The 3' regulatory region is typically not translated, but contains regulatory regions that post-transcriptionally influence gene expression.

Any 3' regulatory region may be used, the selection of which may be readily made by the skilled person. For example, a 3' regulatory region may include one or more regulatory elements. For example, a 3' regulatory region may include one or more post-transcriptional regulatory elements, an AU-rich element, one or more miRNA target sequences, and/or a polyadenylation signal.

In some embodiments, a 3' regulatory region comprises one or more post-transcriptional regulatory elements. A "post-transcriptional regulatory element" may control gene expression at the RNA level (i.e. post-transcription). Suitable post-transcriptional regulatory element will be known to the skilled person. Example post-transcriptional regulatory elements include a Woodchuck Hepatitis Virus Post-transcriptional Regulatory Element (WPRE). In some embodiments, a 3' regulatory region comprises a WPRE devoid of the X-protein ORF.

In some embodiments, a 3' regulatory region comprises an AU-rich element. An "AU-rich element" is typically from 50 to 150 bp in length and includes many copies of the sequence ATTTA.

In some embodiments, a 3' regulatory region comprises one or more miRNA target sequences. The one or more miRNA target sequences may be used to control expression of the open reading frame, for example to de-target the expression of the open reading frame from specific cells and/or tissues. In preferred embodiments, a 3' regulatory region does not comprise one or more miRNA target sequences.

In some embodiments, a 3' regulatory region comprises a polyadenylation sequence. A polyadenylation sequence may regulate polyadenylation of the transcribed mRNA. A polyadenylation sequence typically comprises a polyadenylation signal, e.g. having the sequence AATAAA, a polyadenylation site and a GT-rich downstream element. Suitable polyadenylation sequences will be known to the skilled person. Example polyadenylation sequences include the early SV40 polyadenylation sequence, the human growth hormone (hGH) polyadenylation sequence, the bovine growth hormone (bGH) polyadenylation sequence, and the rbGlob polyadenylation sequence.

In some embodiments, a 3' regulatory region comprises or consists of from 5' to 3' direction: one or more post-transcriptional regulatory elements and a polyadenylation sequence.

In some embodiments, a 3' regulatory region does not comprise a polyadenylation sequence. In some embodiments, a 3' regulatory region comprises or consists of one or more post-transcriptional regulatory elements.

### Transcription termination site

The polynucleotide of the present invention may further comprise a transcription termination site. The transcription termination site may be downstream from the expression cassette.

A "transcription termination site" or "transcription terminator" may refer to a nucleic acid sequence that marks the end of a gene or operon during transcription. A transcription termination site may mediate transcriptional termination by providing signals in the newly synthesized transcript RNA that trigger processes which release the transcript RNA from the transcriptional complex. For example, in eukaryotic transcription of mRNAs, terminator signals are recognised by protein factors that are associated with the RNA polymerase II and which trigger the termination process. These proteins factors then recruit other proteins to the site to cleave the transcript, freeing the mRNA from the transcription complex, and carrying out polyadenylation (see e.g. Proudfoot, N.J., 2016. Science, 352(6291), p.aad9926).

The transcription termination site may be 10kb or less downstream from the expression cassette. In some embodiments, the transcription termination site is 5000 bp or less, 4000 bp or less, 3000 bp or less, 2500 bp or less, 2000 bp or less, 1500 bp or less, 1000 bp or less, 500 bp or less, 400 bp or less, 300 bp or less, 200 bp or less, 100 bp or less, 90 bp or less, 80 bp or less, 70 bp or less, 60 bp or less, 50 bp or less, 40 bp or less, 30 bp or less, 20 bp or less, 10 bp, 5 bp or less, 4 bp or less, 3 bp or less, or 2 bp or less, downstream from the expression cassette. In some embodiments, the transcription termination site is immediately downstream from the expression cassette (i.e. there are no nucleotides between the expression cassette and the transcription termination site).

In some embodiments, the polynucleotide of the present invention comprises from 5' to 3' direction: an expression cassette and a transcription termination site. The polynucleotide may comprise further elements between the expression cassette and the transcription termination site, such as one or more miRNA target sequences, one or more insulator elements, and/or one or more further regulatory elements.

### microRNA (miRNA) target sequences

The polynucleotide of the present invention comprises one or more miRNA target sequences.

A "miRNA target sequence" may refer to a nucleotide sequence to which a naturally-occurring miRNA binds (see e.g. Geisler, A. and Fechner, H., 2016. World journal of experimental medicine, 6(2), pp.37-54). MicroRNAs (miRNAs) are post-transcriptional regulators of gene expression. miRNA target sequences may be provided using prediction tools and/or by experimental validation (see e.g. Riffo-Campos, Á.L., et al., 2016. International journal of molecular sciences, 17(12), p.1987; and Riolo, G., et al., 2020. Methods and protocols, 4(1), p.1). The miRBase database is a searchable database of published miRNA sequences and annotation (see Griffiths-Jones, S., et al., 2007. Nucleic acids research, 36(suppl_1), pp.D154-D158).

Suitably, the miRNA target sequences are human miRNA target sequences. Example human miRNA target sequences include let-7a-1, let-7a-3, let-7a-2, let-7c, let-7f-2, let-7f-1, mir-143, let-7g, mir-21, let-7b, let-7e, mir-98, mir-26a-2, mir-145, mir-26a-1, mir-99a, mir-26b, let-7i, mir-148a, mir-30a, mir-27b, mir-125b-1, mir-1-2, mir-103a-2, mir-103a-1, mir-107, mir-29a, mir-19b-2, mir-30d, mir-125b-2, mir-19b-1, mir-29c, mir-23b, mir-30e, mir-19a, mir-16-1, mir-16-2, mir-199a-2, mir-23a, mir-100, mir-199a-1, mir-17, mir-199b, mir-191, mir-92a-1, mir-27a, mir-29b-2, mir-29b-1, mir-106a, mir-10b, mir-20a, mir-1246, mir-92a-2, mir-1-1, mir-205, mir-15b, mir-101-2, mir-101-1, mir-378a, mir-23c, mir-140, mir-320a, mir-320b-2, mir-10a, mir-320b-1, mir-378i, mir-378c, mir-151a, mir-200c, mir-378d-2, mir-378f, mir-320e, let-7d, mir-378g, mir-92b, mir-320d-1, mir-126, mir-221, mir-320c-2, mir-186, mir-15a, mir-125a, mir-378b, mir-451a, mir-200b, mir-22, mir-378h, mir-335, mir-24-2, mir-24-1, mir-423, mir-424, mir-7-1, mir-142, mir-28, mir-181a-1, mir-181a-2, mir-320c-1, mir-222, mir-630, mir-375, mir-378d-1, mir-7-3, mir-7-2, mir-30c-2, mir-30c-1, mir-378e, mir-25, mir-18a, mir-320d-2, mir-18b, mir-148b, mir-151b, mir-146a, mir-200a, mir-152, mir-30b, mir-146b, mir-195, mir-93, mir-4429, mir-9985, mir-185, mir-106b, mir-130a, mir-99b, mir-141, mir-196b, mir-196a-1, mir-182, mir-374a, mir-31, mir-1234, mir-196a-2, mir-122, mir-218-1, mir-425, mir-374b, mir-218-2, mir-9-1, mir-9-2, mir-9-3, mir-223, mir-365b, mir-365a, mir-8485, mir-4449, mir-340, mir-1260b, mir-1260a, mir-181d, mir-181b-2, mir-181b-1, mir-4264, mir-4443, mir-130b, mir-361, mir-4289, mir-7851, mir-1290, mir-888, mir-192, mir-574, mir-342, mir-363, mir-132, mir-150, mir-128-1, mir-128-2, mir-215, mir-193b, mir-4521, mir-486-1, mir-486-2, mir-144, mir-3159, mir-135a-1, mir-7110, mir-514b, mir-20b, mir-133a-1, mir-133a-2, mir-193a, mir-203a, mir-660, mir-211, mir-183, mir-96, mir-8081, mir-214, mir-3192, mir-4454, mir-12136, mir-135a-2, mir-484, mir-514a-2, mir-514a-3, mir-514a-1, mir-216a, and mir-9902-1. Suitably, the miRNA target sequences are -5p or -3p miRNA target sequences.

miRNAs are usually transcribed from polymerase-II promoters, generating a so-called primary miRNA transcript (pri-miRNA). From the pri-miRNA, a stem loop of about 60 nucleotides in length, called miRNA precursor (pre-miRNA), is excised leaving a 5' phosphate and a 2 bp long, 3' overhang. The pre-miRNA is then actively transported from the nucleus to the cytoplasm. Dicer performs a double strand cut at the other end of the stem loop, generating a 19-24 bp duplex, which is composed of the mature miRNA and the opposite strand of the duplex, called miRNA*. One strand of the duplex is selectively loaded into the RNA-induced silencing complex (RISC), and accumulates as the mature microRNA. This strand is usually the one whose 5' end is less tightly paired to its complement. However, there are some miRNAs that support accumulation of both duplex strands to similar extent.

Once loaded into RISC, the guide strand of the mature microRNA interacts with mRNA target sequences. If the whole guide strand sequence is perfectly complementary to the mRNA target, the mRNA is endonucleolytically cleaved. If only the seed sequence (i.e. nucleotides 2-8 counted from the 5' end of the miRNA) is perfectly complementary to the target mRNA, RNAi may act through alternative mechanisms leading to translational repression.

A miRNA target sequence may be fully or partially complementary to the corresponding miRNA. The term "fully complementary", as used herein, may mean that the target sequence has a nucleic acid sequence which is 100% complementary to the sequence of the miRNA which recognises it. The term "partially complementary", as used herein, may mean that the target sequence is only in part complementary to the sequence of the miRNA which recognises it, whereby the partially complementary sequence is still recognised by the miRNA. In other words, a partially complementary target sequence in the context of the present invention is effective in recognising the corresponding miRNA. Suitably, a partially complementary miRNA target sequence may be fully complementary to the miRNA seed sequence.

A miRNA target sequence which suppresses transgene expression in specific cells and/or tissues will be known to the skilled person. Determining a miRNA with the desired expression profile may be achieved using techniques known to those skilled in the art. For example, a mammalian microRNA expression atlas is described in Landgraf, P., et al., 2007. Cell, 129(7), pp. 1401-1414 and the distribution of miRNA expression across human tissues is described in Ludwig, N., et al., 2016. Nucleic acids research, 44(8), pp.3865-3877. Once a miRNA has been identified, the corresponding target sequence can readily be determined using, for example, a microRNA database, such as miRBase (see Griffiths-Jones, S., et al., 2007. Nucleic acids research, 36(suppl_1), pp.D154-D158).

Examples of miRNAs specific to or enriched in cells and/or tissues are provided in the table below (see e.g. Dhungel, B., et al., 2018. Molecules, 23(7), p.1500; Geisler, A. and Fechner, H., 2016. World journal of experimental medicine, 6(2), pp.37-54; and Kelly, E.J. and Russell, S.J., 2009. Molecular Therapy, 17(3), pp.409-416).

| **Cell/Tissue type** | **miRNA** |
|---|---|
| Eye | miR124, miR204, miR181 |
| Heart | miR1, miR206, miR126, miR134, miR133, miR208, miR302 |
| Brain/Nervous system | miR338, miR219, miR124a, miR9, miR218, miR7, miR128, miR125, miR138, miR132, miR212, miR137, miR31, miR127, miR143, miR346, miR708 |
| Kidney | miR10a, miR192, miR204, miR194, miR215, miR216 |
| Liver | miR22, miR122, miR192, miR92a, miR483 |
| Lung | miR126, mir200c, miR141 |
| Hematopoietic and pluripotent cells | miR223, miR142, miR126, miR130a, miR302, miR292 |
| Pancreas | miR216, miR217, mir375, mir200c |
| Spleen | miR142, miR150 |
| Muscle | miR133a, miR1, miR206, miR134, miR193a, miR128a |
| Immune system | miR150, miR181a, miR155, miR142 |

The polynucleotide may comprise more than one miRNA target sequence, which may or may not be different. The polynucleotide may, for example, comprise at least 2, at least 3, at least 4, at least 5, at least 6, at least 7, or at least 8 miRNA target sequences. The miRNA target sequences may be the same or different.

The miRNA target sequences may be in any orientation relative to the expression cassette. A miRNA target sequence may be a sense-oriented miRNA target sequence or an antisense-oriented miRNA target sequence.

A "sense-oriented" miRNA target sequence may refer to a nucleotide sequence to which a miRNA binds, e.g. a sense-oriented miRNA target sequence may be a reverse complement of a miRNA. When a sense strand comprising a sense-oriented miRNA sequence is transcribed, the resulting mRNA may be degraded.

An "antisense-oriented" miRNA target sequence may refer to a nucleotide sequence to which a miRNA binds the reverse complement thereof, e.g. an antisense-oriented miRNA target sequence may be identical to a miRNA (except U may be replaced by T). When a sense strand comprising an antisense-oriented miRNA sequence is transcribed, the mRNA is not degraded, however, when an antisense strand comprising an antisense-oriented miRNA sequence is transcribed, the resulting mRNA may be degraded.

### miRNA target sequences outside expression cassette

The polynucleotide of the present invention may comprise one or more miRNA target sequences upstream from the expression cassette and/or downstream from the expression cassette (e.g. an expression cassette comprising a promoter, an open reading frame, and a 3' regulatory region).

miRNA target sequences upstream from the expression cassette and/or downstream from the expression cassette may improve safety of the polynucleotide by avoiding and/or reducing aberrant transcription, aberrant splicing, and/or read-through events. The miRNA target sequences may specifically degrade aberrant transcripts. The miRNA target sequences may be any distance upstream from the expression cassette and/or downstream from the expression cassette, for example 5000 bp or less, 4000 bp or less, 3000 bp or less, 2000 bp or less, 1000 bp or less, 500 bp or less, 400 bp or less, 300 bp or less, 200 bp or less, or 100 bp or less upstream from the expression cassette and/or downstream from the expression cassette.

The miRNA target sequences may be directly upstream and/or directly downstream from the expression cassette. As used herein, "directly upstream" or "directly downstream" may mean that there are no further regulatory elements between the elements. In some embodiments, the miRNA target sequences are 100 bp or less, 90 bp or less, 80 bp or less, 70 bp or less, 60 bp or less, 50 bp or less, 40 bp or less, 30 bp or less, 20 bp or less, or 10 bp or less upstream from the expression cassette and/or downstream from the expression cassette.

The miRNA target sequence may be downstream from the transcription termination site. In some embodiments, the polynucleotide comprises one or more miRNA target sequences downstream from the transcription termination site. The miRNA target sequences may be any distance downstream from the transcription termination site, for example 5000 bp or less, 4000 bp or less, 3000 bp or less, 2000 bp or less, or 1000 bp or less, or 500 bp or less, 400 bp or less, 300 bp or less, 200 bp or less, or 100 bp or less downstream from the transcription termination site.

In some embodiments, the polynucleotide comprises one or more miRNA target sequences directly downstream from the transcription termination site. In some embodiments, the miRNA target sequences are 100 bp or less, 90 bp or less, 80 bp or less, 70 bp or less, 60 bp or less, 50 bp or less, 40 bp or less, 30 bp or less, 20 bp or less, or 10 bp or less downstream from the transcription termination site.

Suitably, the miRNA target sequences are active in target cells allowing selective degradation, through the miRNA pathway, of aberrant transcripts.

In some embodiments, the miRNA target sequences suppress transgene expression in hematopoietic lineages. Suitable miRNA target sequences for suppressing transgene expression in hematopoietic lineages include miR223 target sequences and mir142 target sequences. In some embodiments, the miRNA target sequences are mir223-3p target sequences. In some embodiments, the miRNA target sequences are miR142-3p target sequences.

In some embodiments, the miRNA target sequences suppress transgene expression in liver. Suitable miRNA target sequences for suppressing transgene expression in liver include mir122 target sequences, miR22 target sequences and mir126 target sequences. In some embodiments, the miRNA target sequences are miR122-5p target sequences. In some embodiments, the miRNA target sequences are mir22-3p target sequences. In some embodiments, the miRNA target sequences are mir126-3p target sequences.

In some embodiments, the miRNA target sequences suppress transgene expression in neurons. Suitable miRNA target sequences for suppressing transgene expression in neurons include miR124 target sequences. In some embodiments, the miRNA target sequences are miR124-3p target sequences.

In some embodiments, the miRNA target sequences suppress transgene expression in muscle. Suitable miRNA target sequences for suppressing transgene expression in muscle include miR133a and miR133b target sequences. In some embodiments, the miRNA target sequences are miR133a-3p target sequences. In some embodiments, the miRNA target sequences are miR133b target sequences.

In some embodiments, the miRNA target sequences suppress transgene expression in lung. Suitable miRNA target sequences for suppressing transgene expression in lung include let7f and miR7 target sequences. In some embodiments, the miRNA target sequences are let7f-5p target sequences. In some embodiments, the miRNA target sequences are miR7-5p target sequences.

In some embodiments, the miRNA target sequences suppress transgene expression in multiple tissues. Suitable miRNA target sequences for suppressing transgene expression in multiple tissues include miR22 target sequences and. In some embodiments, the miRNA target sequences are mir22-3p target sequences.

Example sense-oriented miRNA target sequences are provided by SEQ ID NOs: 1 to 10. Example antisense-oriented miRNA target sequences are provided by SEQ ID NOs: 11 to 20.
TCCATAAAGTAGGAAACACTACA
   mirT 142-3p sense-oriented (SEQ ID NO: 1)
GGGGTATTTGACAAACTGACA
   mirT 223-3p sense-oriented (SEQ ID NO: 2)
ACAAACACCATTGTCACACTCCA
   mirT 122-5p sense-oriented (SEQ ID NO: 3)
ACAGTTCTTCAACTGGCAGCTT
   mirT 22-3p sense-oriented (SEQ ID NO: 4)
CGCATTATTACTCACGGTACGA
   mirT 126-3p sense-oriented (SEQ ID NO: 5)
GGCATTCACCGCGTGCCTTA
   mirT 124-3p sense-oriented (SEQ ID NO: 6)
CAGCTGGTTGAAGGGGACCAAA
   mirT 133a-3p sense-oriented (SEQ ID NO: 7)
TAGCTGGTTGAAGGGGACCAAA
   mirT 133b sense-oriented (SEQ ID NO: 8)
AACTATACAATCTACTACCTCA
   let 7f-5p sense-oriented (SEQ ID NO: 9)
ACAACAAAATCACTAGTCTTCCA
   mirT 7-5p sense-oriented (SEQ ID NO: 10)
TGTAGTGTTTCCTACTTTATGGA
   mirT 142-3p antisense-oriented (SEQ ID NO: 11)
TGTCAGTTTGTCAAATACCCC
   mirT 223-3p antisense-oriented (SEQ ID NO: 12)
TGGAGTGTGACAATGGTGTTTGT
   mirT 122-5p antisense-oriented (SEQ ID NO: 13)
AAGCTGCCAGTTGAAGAACTGT
   mirT 22-3p antisense-oriented (SEQ ID NO: 14)
TCGTACCGTGAGTAATAATGCG
   mirT 126-3p antisense-oriented (SEQ ID NO: 15)
TAAGGCACGCGGTGAATGCC
   mirT 124-3p antisense-oriented (SEQ ID NO: 16)
TTTGGTCCCCTTCAACCAGCTG
   mirT 133a-3p antisense-oriented (SEQ ID NO: 17)
TTTGGTCCCCTTCAACCAGCTA
   mirT 133b antisense-oriented (SEQ ID NO: 18)
T GAGGTAGTAGAT TGTATAGT T
   let 7f-5p antisense-oriented (SEQ ID NO: 19)
TGGAAGACTAGTGATTTTGTTGT
   mirT 7-5p antisense-oriented (SEQ ID NO: 20)

Variant miRNA target sequences may have one or two nucleotide variations (e.g. substitutions, insertions, additions or deletions) whilst retaining the ability to bind the corresponding miRNA. As described above, a partially complementary target sequence may be effective in recognising the corresponding miRNA. Suitably, a variant miRNA target sequence may be fully complementary to the miRNA seed sequence (i.e. nucleotides 2-8 counted from the 5' end of the miRNA).

Including more than one copy of a miRNA target sequence may increase the effectiveness of the miRNA target sequence. Also, different miRNA target sequences can be combined. The polynucleotide may, for example, comprise 1, 2, 3, 4, 5, 6, 7, 8 or more copies of the same or different miRNA target sequences. In some embodiments, the polynucleotide comprises at least 2 copies of each miRNA target sequence. In some embodiments, the polynucleotide comprises from 2 to 4 copies of each miRNA target sequence.

Copies of miRNA target sequences may be arranged in tandem, but other arrangements are envisaged. Copies of miRNA target sequences may be separated by a spacer sequence. A spacer sequence may comprise any suitable number of nucleotide bases, for example, at least one, at least two, at least three, at least four or at least five nucleotide bases. Suitably, the polynucleotide comprises at least 2 copies of each miRNA target sequence arranged in tandem. Suitably, the polynucleotide comprises from 2 to 4 copies of each miRNA target sequence arranged in tandem.

Example tandem miRNA target sequences are provided by SEQ ID NOs: 21 and 22.
TCCATAAAGTAGGAAACACTACACGATTCCATAAAGTAGGAAACACTACA
   2x mirT 142-3p sense-oriented (SEQ ID NO: 21)
TGTCAGTTTGTCAAATACCCCATCGTGTCAGTTTGTCAAATACCCC
   2x mirT 223-3p antisense-oriented (SEQ ID NO: 22)

The one or more miRNA target sequences may have any orientation relative to the expression cassette. The polynucleotide of the present invention may comprise: (a) one or more miRNA target sequences having the same orientation as the expression cassette, upstream from the expression cassette and/or downstream from the transcription termination site; and/or (b) one or more miRNA target sequences having the opposite orientation as the expression cassette, upstream from the expression cassette and/or downstream from the expression cassette.

In some embodiments, the polynucleotide comprises: (a) one or more miRNA target sequences having the same orientation as the expression cassette, upstream from the expression cassette and/or downstream from the transcription termination site; and (b) one or more miRNA target sequences having the opposite orientation as the expression cassette, upstream from the expression cassette and/or downstream from the expression cassette.

### miRNA target sequences inside expression cassette

The polynucleotide of the present invention may further comprise one or more miRNA target sequences within the expression cassette (e.g. an expression cassette comprising a promoter, an open reading frame, and a 3' regulatory region). The one or more miRNA target sequences preferably have the same orientation as the expression cassette. For example, the 3' regulatory region may comprise one or more miRNA target sequences having the same orientation as the expression cassette.

Expression of the open reading frame may be regulated by one or more endogenous miRNAs using one or more corresponding miRNA target sequences within the expression cassette. Using this method, one or more miRNAs endogenously expressed in a cell may prevent or reduce transgene expression in that cell by interacting with the corresponding miRNA target sequence positioned within the expression cassette (see e.g. Brown, B.D. and Naldini, L., 2009. Nature Reviews Genetics, 10(8), pp.578-585). Suitable miRNA target sequences which suppress transgene expression in specific cells are known.

The one or more miRNA target sequence may suppress transgene expression in hematopoietic-lineage and/or antigen-presenting cells. By preventing transgene expression in antigen-presenting cells, while permitting high levels of expression in other cells, miRNA regulation may enable strong and stable gene transfer in the absence of an immune response. Suitable miRNA target sequences for suppressing transgene expression in hematopoietic-lineage cells and/or antigen-presenting cells include miR223 target sequences and mir142 target sequences. Other miRNA target sequences which suppress transgene expression in hematopoietic-lineage cells and/or antigen-presenting cells are known in the art (see e.g. Ghafouri-Fard, S., et al., 2021. Non-coding RNA research, 6(1), pp.8-14). Further miRNA target sequences that suppress transgene expression in hematopoietic-lineage cells and/or antigen-presenting cells can be identified by any suitable method, for example miRNA expression analysis as described in Monticelli, S., et al., 2005. Genome biology, 6(8), pp.1-15.

Including more than one copy of a miRNA target sequence may increase the effectiveness of the miRNA target sequence. Also, different miRNA target sequences can be combined. The polynucleotide may, for example, comprise 1, 2, 3, 4, 5, 6, 7, 8 or more copies of the same or different miRNA target sequences. In some embodiments, the polynucleotide comprises at least 2 copies of each miRNA target sequence. In some embodiments, the polynucleotide comprises from 2 to 4 copies of each miRNA target sequence.

Copies of miRNA target sequences may be arranged in tandem, but other arrangements are envisaged. Copies of miRNA target sequences may be separated by a spacer sequence. A spacer sequence may comprise any suitable number of nucleotide bases, for example, at least one, at least two, at least three, at least four or at least five nucleotide bases. Suitably, the polynucleotide comprises at least 2 copies of each miRNA target sequence arranged in tandem. Suitably, the polynucleotide comprises from 2 to 4 copies of each miRNA target sequence arranged in tandem.

In some embodiments, the polynucleotide of the present invention does not comprise one or more miRNA target sequences within the expression cassette. In some embodiments, the polynucleotide of the present invention does not comprise one or more miRNA target sequences having the same orientation as the expression cassette within the expression cassette.

In some embodiments, the polynucleotide of the present invention does not comprise one or more miRNA target sequences within the 3' regulatory region. In some embodiments, the polynucleotide of the present invention does not comprise one or more miRNA target sequences having the same orientation as the expression cassette within the 3' regulatory region.

### Same orientation miRNA target sequences

In some embodiments, the polynucleotide of the present invention comprises one or more miRNA target sequences having the same orientation as the expression cassette.

The one or more miRNA target sequences having the same orientation as the expression cassette may be upstream from the expression cassette and/or downstream from the transcription termination site.

In some embodiments, the polynucleotide comprises from 5' to 3' direction: one or more miRNA target sequences having the same orientation as the expression cassette; and the expression cassette. In some embodiments, the polynucleotide comprises from 5' to 3' direction: the expression cassette; and one or more miRNA target sequences having the same orientation as the expression cassette. In some embodiments, the polynucleotide comprises from 5' to 3' direction: one or more miRNA target sequences having the same orientation as the expression cassette; the expression cassette; and one or more miRNA target sequences having the same orientation as the expression cassette.

The polynucleotide may comprise two or more, three or more, four or more, five or more, six or more, seven or more, or eight or more miRNA target sequences having the same orientation as the expression cassette, upstream from the expression cassette and/or downstream from the transcription termination site.

In some embodiments, the polynucleotide comprises two or more miRNA target sequences having the same orientation as the expression cassette, upstream from the expression cassette; and/or two or more miRNA target sequences having the same orientation as the expression cassette, downstream from the transcription termination site. In some embodiments, the polynucleotide comprises two or more miRNA target sequences having the same orientation as the expression cassette, upstream from the expression cassette. In some embodiments, the polynucleotide comprises two or more miRNA target sequences having the same orientation as the expression cassette, downstream from the transcription termination site.

In some embodiments, the polynucleotide comprises two miRNA target sequences having the same orientation as the expression cassette, upstream from the expression cassette; and/or two miRNA target sequences having the same orientation as the expression cassette, downstream from the transcription termination site. In some embodiments, the polynucleotide comprises two miRNA target sequences having the same orientation as the expression cassette, upstream from the expression cassette. In some embodiments, the polynucleotide comprises two miRNA target sequences having the same orientation as the expression cassette, downstream from the transcription termination site.

The miRNA target sequences having the same orientation as the expression cassette may be 5000 bp or less, 4000 bp or less, 3000 bp or less, 2000 bp or less, 1000 bp or less, 500 bp or less, 400 bp or less, 300 bp or less, 200 bp or less, or 100 bp or less upstream from the expression cassette and/or downstream from the transcription termination site.

The miRNA target sequences having the same orientation as the expression cassette may be directly upstream from the expression cassette and/or directly downstream from the transcription termination site. In some embodiments, the miRNA target sequences having the same orientation as the expression cassette and upstream from the expression cassette are 100 bp or less, 90 bp or less, 80 bp or less, 70 bp or less, 60 bp or less, 50 bp or less, 40 bp or less, 30 bp or less, 20 bp or less, or 10 bp or less upstream from the expression cassette and/or the miRNA target sequences having the same orientation as the expression cassette and downstream from the transcription termination site are 100 bp or less, 90 bp or less, 80 bp or less, 70 bp or less, 60 bp or less, 50 bp or less, 40 bp or less, 30 bp or less, 20 bp or less, or 10 bp or less downstream from the transcription termination site.

The miRNA target sequences having the same orientation as the expression cassette may be 5000 bp or less, 4000 bp or less, 3000 bp or less, 2000 bp or less, 1000 bp or less, 500 bp or less, 400 bp or less, 300 bp or less, 200 bp or less, or 100 bp or less upstream from the expression cassette. In some embodiments, the polynucleotide comprises one or more miRNA target sequences having the same orientation as the expression cassette, directly upstream from the expression cassette. In some embodiments, the polynucleotide comprises one or more miRNA target sequences having the same orientation as the expression cassette 100 bp, 90 bp or less, 80 bp or less, 70 bp or less, 60 bp or less, 50 bp or less, 40 bp or less, 30 bp or less, 20 bp or less, or 10 bp or less upstream from the expression cassette.

The miRNA target sequences having the same orientation as the expression cassette may be 5000 bp or less, 4000 bp or less, 3000 bp or less, 2000 bp or less, 1000 bp or less, 500 bp or less, 400 bp or less, 300 bp or less, 200 bp or less, or 100 bp or less downstream from the transcription termination site. In some embodiments, the polynucleotide comprises one or more miRNA target sequences having the same orientation as the expression cassette, directly downstream from the transcription termination site. In some embodiments, the polynucleotide comprises one or more miRNA target sequences having the same orientation as the expression cassette 100 bp, 90 bp or less, 80 bp or less, 70 bp or less, 60 bp or less, 50 bp or less, 40 bp or less, 30 bp or less, 20 bp or less, or 10 bp or less downstream from the transcription termination site.

In some embodiments, the miRNA target sequences having the same orientation as the expression cassette suppress transgene expression in hematopoietic lineages and/or liver.

In some embodiments, the miRNA target sequences having the same orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, and a combination thereof. In some embodiments, the miRNA target sequences having the same orientation as the expression cassette are selected from miR223 target sequences, miR142 target sequences, miR22 target sequences, and miR122 target sequences.

In some embodiments, the miRNA target sequences having the same orientation as the expression cassette comprise or consist of a nucleotide sequence selected from the group consisting of SEQ ID NOs: 1 to 20, or a variant thereof having one or two nucleotide variations. In some embodiments, the miRNA target sequences having the same orientation as the expression cassette comprise or consist of a nucleotide sequence selected from the group consisting of SEQ ID NOs: 1 to 20.

In some embodiments, the miRNA target sequences having the same orientation as the expression cassette comprise or consist of a nucleotide sequence selected from the group consisting of SEQ ID NOs: 1 to 10, or a variant thereof having one or two nucleotide variations. In some embodiments, the miRNA target sequences having the same orientation as the expression cassette comprise or consist of a nucleotide sequence selected from the group consisting of SEQ ID NOs: 1 to 10.

In some embodiments, the miRNA target sequences having the same orientation as the expression cassette suppress transgene expression in hematopoietic lineages.

In some embodiments, the miRNA target sequences having the same orientation as the expression cassette are miR142 target sequences. In some embodiments, the miRNA target sequences having the same orientation as the expression cassette are miR142-3p target sequences.

In some embodiments, the miRNA target sequences having the same orientation as the expression cassette comprise or consist of a nucleotide sequence selected from the group consisting of SEQ ID NOs: 1 or 11, or a variant thereof having one or two nucleotide variations. In some embodiments, the miRNA target sequences having the same orientation as the expression cassette comprise or consist of a nucleotide sequence selected from the group consisting of SEQ ID NOs: 1 or 11.

In some embodiments, the miRNA target sequences having the same orientation as the expression cassette comprise or consist of the nucleotide sequence of SEQ ID NO: 1, or a variant thereof having one or two nucleotide variations. In some embodiments, the miRNA target sequences having the same orientation as the expression cassette comprise or consist of the nucleotide sequence of SEQ ID NO: 1.

In some embodiments, the miRNA target sequences having the same orientation as the expression cassette comprise or consist of the nucleotide sequence of SEQ ID NO: 21, or a variant thereof having one to four nucleotide variations. In some embodiments, the miRNA target sequences having the same orientation as the expression cassette comprise or consist of the nucleotide sequence of SEQ ID NO: 21, or a variant thereof having one or two nucleotide variations. In some embodiments, the miRNA target sequences having the same orientation as the expression cassette comprise or consist of the nucleotide sequence of SEQ ID NO: 21.

### Opposite orientation miRNA target sequences

In some embodiments, the polynucleotide of the present invention comprises one or more miRNA target sequences having the opposite orientation as the expression cassette.

The one or more miRNA target sequences having the opposite orientation as the expression cassette may be upstream from the expression cassette and/or downstream from the expression cassette.

In some embodiments, the polynucleotide comprises from 5' to 3' direction: one or more miRNA target sequences having the opposite orientation as the expression cassette; and the expression cassette. In some embodiments, the polynucleotide comprises from 5' to 3' direction: the expression cassette; and one or more miRNA target sequences having the opposite orientation as the expression cassette. In some embodiments, the polynucleotide comprises from 5' to 3' direction: one or more miRNA target sequences having the opposite orientation as the expression cassette; the expression cassette; and one or more miRNA target sequences having the opposite orientation as the expression cassette.

The polynucleotide may comprise two or more, three or more, four or more, five or more, six or more, seven or more, or eight or more miRNA target sequences having the opposite orientation as the expression cassette, upstream from the expression cassette and/or downstream from the expression cassette.

In some embodiments, the polynucleotide comprises two or more miRNA target sequences having the opposite orientation as the expression cassette, upstream from the expression cassette; and/or two or more miRNA target sequences having the opposite orientation as the expression cassette, downstream from the expression cassette. In some embodiments, the polynucleotide comprises two or more miRNA target sequences having the opposite orientation as the expression cassette, upstream from the expression cassette. In some embodiments, the polynucleotide comprises two or more miRNA target sequences having the opposite orientation as the expression cassette, downstream from the expression cassette.

In some embodiments, the polynucleotide comprises two miRNA target sequences having the opposite orientation as the expression cassette, upstream from the expression cassette; and/or two miRNA target sequences having the opposite orientation as the expression cassette, downstream from the expression cassette. In some embodiments, the polynucleotide comprises two miRNA target sequences having the opposite orientation as the expression cassette, upstream from the expression cassette. In some embodiments, the polynucleotide comprises two miRNA target sequences having the opposite orientation as the expression cassette, downstream from the expression cassette.

The miRNA target sequences having the opposite orientation as the expression cassette may be 5000 bp or less, 4000 bp or less, 3000 bp or less, 2000 bp or less, 1000 bp or less, 500 bp or less, 400 bp or less, 300 bp or less, 200 bp or less, or 100 bp or less upstream from the expression cassette and/or downstream from the expression cassette.

The miRNA target sequences having the opposite orientation as the expression cassette may be directly upstream and/or directly downstream from the expression cassette. In some embodiments, the miRNA target sequences having the opposite orientation as the expression cassette are 100 bp or less, 90 bp or less, 80 bp or less, 70 bp or less, 60 bp or less, 50 bp or less, 40 bp or less, 30 bp or less, 20 bp or less, or 10 bp or less upstream from the expression cassette and/or downstream from the expression cassette.

The miRNA target sequences having the opposite orientation as the expression cassette may be 5000 bp or less, 4000 bp or less, 3000 bp or less, 2000 bp or less, 1000 bp or less, 500 bp or less, 400 bp or less, 300 bp or less, 200 bp or less, or 100 bp or less upstream from the expression cassette. In some embodiments, the polynucleotide comprises one or more miRNA target sequences having the opposite orientation as the expression cassette, directly upstream from the expression cassette. In some embodiments, the polynucleotide comprises one or more miRNA target sequences having the opposite orientation as the expression cassette 100 bp, 90 bp or less, 80 bp or less, 70 bp or less, 60 bp or less, 50 bp or less, 40 bp or less, 30 bp or less, 20 bp or less, or 10 bp or less upstream from the expression cassette.

The miRNA target sequences having the opposite orientation as the expression cassette may be 5000 bp or less, 4000 bp or less, 3000 bp or less, 2000 bp or less, 1000 bp or less, 500 bp or less, 400 bp or less, 300 bp or less, 200 bp or less, or 100 bp or less downstream from the expression cassette. In some embodiments, the polynucleotide comprises one or more miRNA target sequences having the opposite orientation as the expression cassette, directly downstream from the expression cassette. In some embodiments, the polynucleotide comprises one or more miRNA target sequences having the opposite orientation as the expression cassette 100 bp, 90 bp or less, 80 bp or less, 70 bp or less, 60 bp or less, 50 bp or less, 40 bp or less, 30 bp or less, 20 bp or less, or 10 bp or less downstream from the expression cassette.

Alternatively, or additionally, the polynucleotide may comprise one or more miRNA target sequences having the opposite orientation as the expression cassette within the expression cassette (e.g. within the 3' regulatory region). In some embodiments, the polynucleotide comprises one or more miRNA target sequences having the opposite orientation as the expression cassette within the expression cassette. In some embodiments, the one or more miRNA target sequences having the opposite orientation as the expression cassette are within the 3' regulatory region.

In some embodiments, the miRNA target sequences having the opposite orientation as the expression cassette suppress transgene expression in hematopoietic lineages and/or liver.

In some embodiments, the miRNA target sequences having the opposite orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, and a combination thereof. In some embodiments, the miRNA target sequences having the opposite orientation as the expression cassette are selected from miR223 target sequences, miR142 target sequences, miR22 target sequences, and miR122 target sequences.

In some embodiments, the miRNA target sequences having the opposite orientation as the expression cassette comprise or consist of a nucleotide sequence selected from the group consisting of SEQ ID NOs: 1 to 20, or a variant thereof having one or two nucleotide variations. In some embodiments, the miRNA target sequences having the opposite orientation as the expression cassette comprise or consist of a nucleotide sequence selected from the group consisting of SEQ ID NOs: 1 to 20.

In some embodiments, the miRNA target sequences having the opposite orientation as the expression cassette comprise or consist of a nucleotide sequence selected from the group consisting of SEQ ID NOs: 11 to 20, or a variant thereof having one or two nucleotide variations. In some embodiments, the miRNA target sequences having the opposite orientation as the expression cassette comprise or consist of a nucleotide sequence selected from the group consisting of SEQ ID NOs: 11 to 20.

In some embodiments, the miRNA target sequences having the opposite orientation as the expression cassette suppress transgene expression in hematopoietic lineages.

In some embodiments, the miRNA target sequences having the opposite orientation as the expression cassette are miR223 target sequences. In some embodiments, the miRNA target sequences having the opposite orientation as the expression cassette are miR223-3p target sequences.

In some embodiments, the miRNA target sequences having the opposite orientation as the expression cassette comprise or consist of a nucleotide sequence selected from the group consisting of SEQ ID NOs: 2 or 12, or a variant thereof having one or two nucleotide variations. In some embodiments, the miRNA target sequences having the opposite orientation as the expression cassette comprise or consist of a nucleotide sequence selected from the group consisting of SEQ ID NOs: 2 or 12.

In some embodiments, the miRNA target sequences having the opposite orientation as the expression cassette comprise or consist of the nucleotide sequence of SEQ ID NO: 12, or a variant thereof having one or two nucleotide variations. In some embodiments, the miRNA target sequences having the opposite orientation as the expression cassette comprise or consist of the nucleotide sequence of SEQ ID NO: 12.

In some embodiments, the miRNA target sequences having the opposite orientation as the expression cassette comprise or consist of the nucleotide sequence of SEQ ID NO: 22, or a variant thereof having one to four nucleotide variations. In some embodiments, the miRNA target sequences having the opposite orientation as the expression cassette comprise or consist of the nucleotide sequence of SEQ ID NO: 22, or a variant thereof having one or two nucleotide variations. In some embodiments, the miRNA target sequences having the opposite orientation as the expression cassette comprise or consist of the nucleotide sequence of SEQ ID NO: 22.

### Insulator elements

The polynucleotide of the present invention may comprise one or more insulator elements.

The one or more insulator elements may be upstream from the expression cassette and/or downstream from the expression cassette.

In some embodiments, the polynucleotide comprises from 5' to 3' direction: an insulator element; and the expression cassette. In some embodiments, the polynucleotide comprises from 5' to 3' direction: the expression cassette; and an insulator element. In some embodiments, the polynucleotide comprises from 5' to 3' direction: an insulator element; the expression cassette; and an insulator element.

An "insulator element", "chromatin insulator", or "chromatin insulator element" may refer to a genetic element that functions to prevent activation of a promoter by nearby heterologous enhancers and/or functions as a barrier against repressive effects of neighbouring heterochromatin (see e.g. Valenzuela, L. and Kamakaka, R.T., 2006. Annu. Rev. Genet., 40, pp.107-138). Insulator elements may, for example, be used in integrating vectors to reduce the interactions between the integrated vector enhancers and cellular promoters. In a genomic context, insulator elements allow gene expression regulation of the host genome by mediating chromatin loops that physically separate genomic regions in functional domains, where sequences within the same domain interact and regulate each other, while such crosstalk is blocked in regions from different chromatin domains.

An insulator element may bind to an insulator-binding protein (IBP). Examples of known insulator-binding proteins include CTCF, Su(Hw), Ibf1, Ibf2, ZIPIC, Cp190, Mod(mdg4), and BEAF-32 (see e.g. Ozdemir, I. and Gambetta, M.C., 2019. Genes, 10(10), p.767). Suitably, an insulator element binds to a human IBP, such as CTCF.

The insulator elements may be in any orientation relative to the expression cassette. An insulator element may be a forward insulator element or a reverse insulator element. A "forward" insulator element may refer to a nucleotide sequence to which an insulator-binding protein binds. A "reverse" insulator element may refer to a nucleotide sequence to which an insulator-binding protein binds the reverse-complement thereof. Both forward and reverse insulator elements may function to prevent activation of a promoter by nearby heterologous enhancers and/or as a barrier against repressive effects of neighbouring heterochromatin. Insulator elements may improve safety of the polynucleotide by avoiding and/or reducing enhancer-mediated gene activation.

### CTCF-binding motifs

In some embodiments, an insulator element binds CCCTC-binding factor (CTCF).

The best characterized insulator protein described to date is the CCCTC-binding factor (CTCF), which is one of the master regulators of chromatin structure and which is involved in long-range interactions via chromatin loops formation. Such loops are formed by the action of Cohesin that binds to DNA and extrude chromatin through a loop until a pair of convergent CTCF sites is found. By virtue of this ability, CTCF-bound insulators can block interactions between regulatory elements like enhancers and promoters.

Suitably, an insulator element comprises a CTCF-binding motif. Example CTCF-binding motifs are provided in SEQ ID NOs: 23 to 28. Suitably, an insulator element comprises a nucleotide sequence selected from the group consisting of SEQ ID NOs: 23 to 28, or a nucleotide sequence having one or two nucleotide variations. In some embodiments, an insulator element comprises a nucleotide sequence selected from the group consisting of SEQ ID NOs: 23 to 28, or a nucleotide sequence having one nucleotide variation. In some embodiments, an insulator element comprises a nucleotide sequence selected from the group consisting of SEQ ID NOs: 23 to 28.
CACCAGGTGGCGCT
   Example CTCF-binding motif A (SEQ ID NO: 23)
CCACCAGGGGGAGC
   Example CTCF-binding motif B (SEQ ID NO: 24)
TCAGTAGAGGGCGC
   Example CTCF-binding motif C (SEQ ID NO: 25)
CCACTAGGGGGCAG
   Example CTCF-binding motif D (SEQ ID NO: 26)
CAGCAGAGGGCGCT
   Example CTCF-binding motif E (SEQ ID NO: 27)
CAGTAGAGGGCGCT
   Example CTCF-binding motif F (SEQ ID NO: 28)

In some embodiments, an insulator element comprises a nucleotide sequence selected from the group consisting of SEQ ID NOs: 23, 24, and 27, or a nucleotide sequence having one or two nucleotide variations. In some embodiments, an insulator element comprises a nucleotide sequence selected from the group consisting of SEQ ID NOs: 23, 24, and 27, or a nucleotide sequence having one nucleotide variation. In some embodiments, an insulator element comprises a nucleotide sequence selected from the group consisting of SEQ ID NOs: 23, 24, and 27.

In some embodiments, an insulator element comprises the nucleotide sequence of SEQ ID NO: 23, or a nucleotide sequence having one or two nucleotide variations. In some embodiments, an insulator element comprises the nucleotide sequence of SEQ ID NO: 23, or a nucleotide sequence having one nucleotide variation. In some embodiments, an insulator element comprises the nucleotide sequence of SEQ ID NO: 23.

In some embodiments, an insulator element comprises the nucleotide sequence of SEQ ID NO: 24, or a nucleotide sequence having one or two nucleotide variations. In some embodiments, an insulator element comprises the nucleotide sequence of SEQ ID NO: 24, or a nucleotide sequence having one nucleotide variation. In some embodiments, an insulator element comprises the nucleotide sequence of SEQ ID NO: 24.

In some embodiments, an insulator element comprises the nucleotide sequence of SEQ ID NO: 27, or a nucleotide sequence having one or two nucleotide variations. In some embodiments, an insulator element comprises the nucleotide sequence of SEQ ID NO: 27, **or a nucleotide** sequence having one nucleotide variation. In some embodiments, an insulator element comprises the nucleotide sequence of SEQ ID NO: 27.

### Core insulator sequences

Suitably, an insulator element comprises a core insulator sequence. As used herein, a "core insulator sequence" may refer to a nucleic acid sequence comprising a binding motif and minimal flanking sequences. The flanking sequences may each independently comprise from 5 to 20 nucleotides, from 10 to 20 nucleotides, or from 10 to 15 nucleotides. A core insulator sequence may have any suitable length, for example a total length of from 30 to 50 nucleotides, from 35 to 45 nucleotides, or from 38 to 42 nucleotides.

In some embodiments, an insulator element consists of a core insulator sequence. Using insulator elements consisting of core insulator sequences may allow the polynucleotide length to be minimised.

Example forward core insulator sequence are provided in SEQ ID NOs: 29 to 32. Example reverse core insulator sequence are provided in SEQ ID NOs: 33 to 36.
TCTGCTTGATGTGGACACCAGGTGGCGCTGGACATCAGAT
   Example core A1 insulator sequence (forward) (SEQ ID NO: 29)
GCACAGACAAATTACCACCAGGTGGCGCTCAGAGTCTGCG
   Example core A2 insulator sequence (forward) (SEQ ID NO: 30)
AGCGTTTGCTTCTGGCCACCAGGGGGAGCTGCTGGGACGCAG
   Example core B2 insulator sequence (forward) (SEQ ID NO: 31)
ATCAAACCAGGAAACAGCAGAGGGCGCTGGTGCTGTGCT
   Example core E2 insulator sequence (forward) (SEQ ID NO: 32)
ATCTGATGTCCAGCGCCACCTGGTGTCCACATCAAGCAGA
   Example core A1 insulator sequence (reverse) (SEQ ID NO: 33)
CGCAGACTCTGAGCGCCACCTGGTGGTAATTTGTCTGTGC
   Example core A2 insulator sequence (reverse) (SEQ ID NO: 34)
CTGCGTCCCAGCAGCTCCCCCTGGTGGCCAGAAGCAAACGCT
   Example core B2 insulator sequence (reverse) (SEQ ID NO: 35)
AGCACAGCACCAGCGCCCTCTGCTGTTTCCTGGTTTGAT
   Example core E2 insulator sequence (reverse) (SEQ ID NO: 36)

In some embodiments, an insulator element comprises or consists of a nucleotide sequence selected from the group consisting of SEQ ID NOs: 29 to 36, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise a CTCF-binding motif (e.g. any of SEQ ID NOs: 23 to 28) and one or more variation is made in the flanking sequences.

In some embodiments, an insulator element comprises or consists of a nucleotide sequence selected from the group consisting of SEQ ID NOs: 29 to 36.

### Full-length insulator sequences

In some embodiments, an insulator element comprises or consists of one or more full-length insulator sequences. As used herein, a "full-length insulator sequence" may refer to a nucleic acid sequence comprising a binding motif and flanking sequences. The flanking sequences may each independently comprise from 21 to 500 nucleotides, from 30 to 400 nucleotides, from 40 to 300 nucleotides, or from 50 to 250 nucleotides. A full-length insulator sequence may have any suitable length, for example a total length of from 100 to 1000 nucleotides, from 150 to 800 nucleotides, or from 200 to 600 nucleotides.

In some embodiments, an insulator element consists of one or more full-length insulator sequence.

Example (forward) full-length insulator sequence are provided in SEQ ID NOs: 37 to 63 and 102. Example (reverse) full-length insulator sequence are provided in SEQ ID NOs: 64 to 90 and 103. 
CTGGTTCTACTCATTACATTCCAATCGTGGCATATCCTCTAAACTTTCTTTTCCCTTCATAA
ATCCTCTTTCTTTTTTTTCCCCCTCACAGTTTTCCTGAACAGGTTGACTATTAATTGTGTCT
GCTTGATGTGGACACCAGGTGGCGCTGGACATCAGATTTGGAGAGGCAGTTGTCTAGGGAAC
CGGGCTCTGTGCCAGCGCAGGAGGCAGGCTGGCTCTCCTATTCCAGGGATGCTCATCCAGGA
AGGAAAGGTTGCATGCTGGACACACTAACCTTGAAGAATTCTTCTGTCTCTCTCGTCATTTA
GAAAGGAAGG
 Example A1 insulator sequence (forward) (SEQ ID NO: 37) 
ACAATGGCTGGCCCATAGTAAATGCCGTGTTAGTGTGTTAGTTGCTGTTCTTCCACGTCAGA
AGAGGCACAGACAAATTACCACCAGGTGGCGCTCAGAGTCTGCGGAGGCATCACAACAGCCC
TGAATTTGAATCCTGCTCTGCCACTGCCTAGTTGAGACCTTTTACTACCTGACTAGCTGAGA
CATTTACGACATTTACTGGCTCTAGGACTCATTTTATTCATTTCATTACTTTTTTTTTCTTT
GAGACGGAATCTCGCTCT
 Example A2 insulator sequence (forward) (SEQ ID NO: 38) 
AGGGGTTGGTCTCCCTATGTTCCCCAGGCTGGTCTCCAACCCCTGGGCTCAAGCAATGCTCC
TGCCTCAACCACCCAAAGTGCTGAGATTACAGGTGTGAACCACTGCGCCCGGCTTCAGAGGA
GTTTTGATGCACCAGGTGGCGCTGGTAATTTAAGGTTCTCTCGTGAAATTGGTCTTTTCACC
 
TGGCCAGCATCTTATTCCTTCTTCTGGTAACAGCATCCCATTGTTCTTTGTTGAACCACCTC
TCCC
 Example A3 insulator sequence (forward) (SEQ ID NO: 39) 
GAACTCCTGACCCCTCACAAAGTGCTGGGATTACAAGCGTGAGCCACCGCGCCCGGCCAGAA
CTTTTGAAAGGAAATTACAAAGGTAGTTATTGGATAAAAGCGTGATAGAGTAGTTAGCACC
AGGTGGCGCTAAAGGACTGAAATTGGAAACGGCTACGGTATTTGGTCCTCTTACCTGAGGGC
CGGGGAAGGGCGATGCTCCTGGTTTAATGGCTGTGTTACAGGAAGGAAGGACAAGCA
 Example A4 insulator sequence (forward) (SEQ ID NO: 40) 
TCCCACTCCATCACCTTCAAGAATGTTCAAAATCCAGAGATTCTAGGATTCTGTTTCCCAGG
ACTTGGACGACTCCGTGTCTCCATAGCTCCACCAGGTGGCGCTGCCGGCCTCGTGACCACT
TGGAAAAAGCAGTGTCACCAGAACGCTGCGGAGACCTGCCAGCAGGGGTCACACTGAGGTTG
CCTCGTGGTGTCCCTGCCTCTGCAGGTGACCTATGAAATTCCTGGCAGGCCCAGCACAGTGG
CTCACGCCTGTAATCCCAGCACTTTGGGTGGCCGAGGCGGGTGGATCACCTGAGGTCAGGAG
TTCGAGACCAGCCTGGCCAACATGGTGAAACCCCGTCTCTAC
 Example A5 insulator sequence (forward) (SEQ ID NO: 41) 
AGGCATGACTGGAAGAACAAATCACATTTCTCTGTGTACTTAGGGGAGAAAGAATTAATTC
AAATGCAGGGTCTGCCGCTTCAGGTCCATTTACGGAAGCCGCAATCCTGCACTATGACCACC
AGGGGGAGCGCTAGGCCCATCCCTGCACAGGTAGCTATTTTAGAGGTTGCTTCTAGCCTGG
AGGAAGAAACGAAAGAAATAAAAGACTATGATACTTTTTTTTTTTAACCGGACCAACTTATA
AATTATTTGTAAGTCCAATAAAAGGCAATTATCAACATTTCCTACTATTTCAGGAGTAATAT
ATTCTATGTTAGAAAAAGATGCACTTTATTCCATGGCT
 Example B1 insulator sequence (forward) (SEQ ID NO: 42) 
AAGAGCCCCAAGAAGTGGATGCGACGTGTGTGCCCCTGGCCTGTCCCTCATCACTCATCTGA
CACATCACTCATATTTGCGGAGGAGCCTTGGTCTGAAAACTATTCCGGAATGGGAATCCAGG
GGTAGGAGATGCTTTGGCTCTAGATGCAAGACATGAAGCGTTTGCTTCTGGCCACCAGGGGG
AGCTGCTGGGACGCAGACGGACTCCAAGGACTGGAGCCAGAAGCCTGGTGTTGCCATGAGGG
GTCTGATCTGCACACCTGTCTCAGGACCTAAACGTCAGGCCAGGCCTCCAAGGGCCTATTTG
CAGGAGAGCCCTG
 Example B2 insulator sequence (forward) (SEQ ID NO: 43) 
CCATCCACCTGCTCTTCATTCTCCTCCCCTGCTTTTCCCAGCTTCTGGTAACCCCATTCCGC
CCTCTATTGGAGGGTTGTGTTTTGATTGGCTGCGAAATGCACAGTGTGGCTTCTCTTCTCCC
AGGAGTGGGAGGGGCCACCTCTAGGGTCCACCAGGGGGAGCTGCCCTGCAGCCCTGGGCTCC
CATCACTCTGGTCCATCCCCTCTCCTCTCCCTCCTGAGCAAATGTGCTAGACAAGACTGTTC
CAGGTATGGGCCAGCCAGATGGCAGCATGGTGGTGAGGGTGCAGATCTGCTGGAAGCAAGAG
AATGTGGACTTCATGGGTTGTGGAGG
 Example B3 insulator sequence (forward) (SEQ ID NO: 44) 
CTCCCGTGTGGTACCTGAGGCCGGCTCCTGTGGCTCTGAGGGGTCTGCAGCACCCCCTTAC
ATCTGTCCACAGAAGGGCTGGGGAGCAGCTTTCCTGTCCCTCCTGTGAGTGGCCACCAGGGG
GAGCGTGGACACAGCTGCCCGTGCAGTGACCACCTGCCCCCCACTCCCGCTACTCCAGCAGC
AGCGGCTCCAGCCCTGGACACCCTCCCTGCCCCCACCAGCCTGGTCCTGAGCCAGGTGACCT
CCTCCAGCATCC
 Example B4 insulator sequence (forward) (SEQ ID NO: 45) 
GCGGAAACTGAACCAAAAGACTCTAAGAGAGGAAGGATAGTGACTTTTAGGCTGCATGGCT
GGGGACAGTGATGGTGGCGTCTTCCTCCTCCTTTGGTTTCTCCGCTGCTTCCTCCCACATCT
TTGACACTGGACAGTCACGCTGAGCTCGTTTCTGGTCTTGCCACCAGGGGGAGCCAGAGCTC
ATATTTCTGGCCCCACTGCAGGAAGCCAGACCTGCTGGGGCAAGCCAGGGGCTTCCCACCCG
GTCACTGGCACAAATCCAGGACCCCAGTTCAGAGGCAGCAGAGTGGTACAAGCAGCCCTGT
 Example B5 insulator sequence (forward) (SEQ ID NO: 46) 
GTCTGAATGGTGGCCGTAGTTTGCAGAGCCCTGGTTTCTTCTTGCCTCTCAGCTTCCAACTT
CCCCGTGAGTGCCTGCTCCTTGATGGACTGGACTCTAAGCCCTTCTTTGCAGCAAGCACGAT
ATCAAGCTTTGTCAGTAGAGGGCGCCGGAGGGACACTGTGGAGGAAGGGGCCTTTTCATGGT
CCACAGAGCTCTGTTGTGCAATTTCTTGTTCCTGTTGCATCTTCTCTTAGGGTATGAACGCG
GGGGGACATCCTCTGGGGCTTTTCCTCAGCTGTGCACCCAGAATGCATGGTCCCTCGACCAC
CTCATAGCCCATCCT
 Example C1 insulator sequence (forward) (SEQ ID NO: 47) 
GTTTCGCATCCACCTTTCATTGCTTGCTCTGTGATAATGGAGAGGACCCTGTAAACGTGTC
CGCCTTGCCACTTTGTGCAATATTTAGCTCCTTCAGTAGAGGGCGCTGCAGGAACATGTCAG
GAGGGGGCCTTCTCTTCCGGATTCTGGATTCTGGTATACACTTGGAGCGTGGCATTTGGGGG
ACAGCCACTGGTGCGCAGCCCCATCAAGTTTTGGTGGCATCCCTGTGGACCATATTCTATTA
ATCTTCAGTGGTACCCCTGTGGCAGATCCCCAGTGATTCTTATGGGCACTCAGGGCTAAC
 Example C3 insulator sequence (forward) (SEQ ID NO: 48) 
AGAAGCACTGCCTGGTAGGATTTCGTAGGAGGAATTCAATCATCAAGTGGATGTGGATTGG
AAATAGGAAGACATTAGATTAGCGTTTCTTTCAACCCAGTAAGTCTGTGAGTGTCCACGCCA
CTTATCAATGCATTCTCAGCTCCAAAGGCATCCTCCTTTAACTGCTCTGTGATAATGGAGAT
GGGCTTTGTAAATATTTCTTACCAGCTGGCATAATGTAAATCCTTGTCAGTAGAGGGCGCTA
GAGAGAGAGAGACCAGAGGAGAAAAAAGTTTTATCTTCCAGATTCTGGGGTGCCTCATCTTT
TTGCCTCCTCCAGAGCACAGCTTGCTTTCTCTGTTGCTGGGCTCCTATGCACATGCTTTCTT
TTGAGCTAGGCCCA
 Example C5 insulator sequence (forward) (SEQ ID NO: 49) 
TCTTTTGCAATGCTCTTTGGGAAATTATATTAGCCTAATTACTAATTTCCTGGCCCTCAAGG
TGACTTGCTTGAACTTGCCACCTTCGCCACTAGGGGCAGCATTGGTTTACACAGGGTGAAC
AAACGTTCAATTCTAAACTAATATTCTTTGGTGGAAAGTGTGTTCATTTTGGTTTGTTTTG
AAGAAGTCACTTCATACCTTTGAACTGGGGTTAAGCTGGTTAACTCCCAAAATTCCACCAGT
TCCAATATCCTATGGAAAAACCCCAAAACCACT
 Example D1 insulator sequence (forward) (SEQ ID NO: 50) 
GAGACCCTCCACCCCCTACCACAGGGAGGCCTCAGGGATGCGCCCTATGGCCAGAGTGAGGG
GGAGAGGGCCCTTGAGGGCCACTTTCCACCTTTGGTGTCCTTGACTGGCATGTCCTGCCCTG
TTCTCCCGTCGCCACTAGGGGGCAGCCACGCAGCAGGAATTTTACGCCAAGAACTCGCGCTG
GACCGAAGGCCTCATCTCGGCCTCCAAGGCTGTGGGCTGGGGAGCCACACAGCTGGTGTAGG
TTGCCCTGGGTGGGGGGGGCAGGGGGCTGCTTCCTGCCAGTTGGAGCAGTTTGGGGTTCAA
CAG
 Example D3 insulator sequence (forward) (SEQ ID NO: 51) 
GGCGTGTTTGATTTGCTTTTCTCTAAAGCACTGCTCTGTAAGTTCCTATGGGGGGGCGGGC
AGAGCTGCTAGAAATACAGAAGCATAAGAGAGTAAATAATGGTACCTTCATATGCAAATGTG
CTTTTCAGATTGGATCTGTCTGTGCTTGTTTTGAGCTATACCCGGTAAGGCTCCCTCCAGAA
ACAGAATTCTTGTTTAGCTCCTGGGAGTGTGCAGAATCCACAACAGCCACTAGGGGGCAGGA
GGCATGTGTCATATATACCGGGCAGTGAGAAAGAGTTAATGCAATTAACTAAGGGGCACTAT
TTTGTACCCTCAGGCTTGAGAGGCCGCCAAGCTACTCCAGCCTCATACAAGTGGGGAAAGAC
TTTGACGGAGTTTAGGGTGGACTGGCAGGCTCTCAACCATAGCAGGAGCACTCAAATTTCTC
AGCTTATGTGGCAGGCCTCATT
 Example D4 insulator sequence (forward) (SEQ ID NO: 52) 
GTTTTCCCCAGTCCTCTTCCCTTCTCCACTGGTTGGGTAGCTCCGTATCTTCCTCTTGCATG
TCAGGGTGGCGGCCACTAGGGGGCAGCAGGCACCGTGGTAACGAATGGGCTGCAGCTTTGGC
ACTTGCGCTCGGGGACTTGGCCTGGCTTGGAGGAGCTCTGCTCCATCCTTCAAGATGACTTA
CGTTGGAGGATGGCGATTCGAGCACCTCTTCACCCCCTTTTTCTCCAGTTTAGGGAAAGGGG
 Example D5 insulator sequence (forward) (SEQ ID NO: 53) 
GCCACCTTTGGTCTTGACATTCACTAAGGAGTGGGCCTGGAATAAAACCAGAAATCCCCATT
CTCAGTCTGCCCCAAGCCCTCCAATTCCGAGGCGCTGTATGTATAAGCTCGGGCAGGCAAA
AGTCGACTGTGAGAACACGCCAGCAGAGGGCGCTGTGGCCCCATCAGTCCCTGCCCACTGAA
CTCTCCAGAAGGAAAAGCGGCAAGGATGCAAACAAGAAAATCAGACCAGACTGGGAGTCTAG
ACCCTGCGTCCCACGCGGTCCCACCATGGCTTCCTCTTTGGAATTTTCAGGGGGACCCAGGA
AGACTGACACCGCTGAT
 Example E1 insulator sequence (forward) (SEQ ID NO: 54) 
TTTGATTTCCTTCACTCTGGAATATACTACAGAATGTACTAATATAAATGTGCTTGCCTATG
TCTAATCTAGAATATAATTATCCTGAACTCTACATGGTATTTTAGTCTAGAAAGTACTGTAG
CTTCTCTGAGTTCTAAATCAAACCAGGAAACAGCAGAGGGCGCTGGTGCTGTGCTTTTGCAA
GTGAGATGTGTGCTCATTTCAAGGGTCCACAAAATTTTCTGGCATGCTGTTTCCATGGAGAG
GAAAGTAGGTGTTTTCTTTTTCTTTTTTTTTTTTCAGTCAAGCCACCTGCAAACT
 Example E2 insulator sequence (forward) (SEQ ID NO: 55) 
CACCCCCTTACTCCACTCAACCCCTCATTCCTTTAGGCAAGGTATTAAGAGCCCTATGCAAA
GGCAATCTTCAAGTATGCGTTGGCAGAAAAACAACCAGTACAAATTGAGGCATAATGTAAAA
CCGTTAGGCTGCTTTTCACCCAGCAGAGGGCGCTAAACAGCTGTGCCCAAGCCTCTGATTCA
ACAAAGCAAACGAGGGCTGGTGAAGCAAGGGAAAGTCAGTTCAGACGCAAAAGCAGCTCTAC
AATTGTCTCCCTTAATCTCCTAGTCAGTTCAGAAAGGCAGAGATTTATTGTTTAGTTCCACA
GAGAGAGACTGACCTGCAAATAATCCAAGTGAAGAATATTTAGGATCATTTCAGACCATTTA
AGCCAGCC
 Example E3 insulator sequence (forward) (SEQ ID NO: 56) 
TCAAAAGAATGTGCCAATGCATATTTCCTAACCCACTGTTATTAATAAGTATTAACCAACAA
CATTGCAACATATAGTTTTAGTGTGATTCCTTTCATTCAGTGTTTTTCAAGAGTTTTAAAAC
AACACCAGCTTTCCAATTGTACTGAATTTGTTTTTTTCCCTTTCATCACCACAAAAATCCAG
TTTTGCCCAGTATATGGGGAAACCATTCTTACTGAACCAGCAGAGGGCGCTGATGCACTAGT
GATGTCCGATATGCCTAAGACTTAAAACATATAGAAACATATTTATGAGAATGGAGAAGAGT
GAGAGAAGAAAACCTTCAGAATCCCTTTTTAAAGGGAATTGTTAAAAATTCTATAGAAAGGA
CGTTCCCTAATATGACATTAAATGTGAGCAGGATTAAATAGGTACTATTCAAAGTAATATTT
ACAGTAAGAAGTTTCAATGTCAACACAAATTCCA
 Example E4 insulator sequence (forward) (SEQ ID NO: 57) 
ACACTTGGGCTGAGAGTGGTGGAGTCAAGAGGTCGGGTTGGGAAGTGCTAAATTTAAAATGC
CCATTATATATCGTCTAAGGGTCTGAATGCAGGCGCCCAGGAACTCAGGGGAGAGGGTGGGA
TGGAAAGAAAAATCCTGGCAGTCAGTCAGTGAACACTGGTCCCCAGAGCCTGCCCGGATTTG
AGTTACTGTCAGTATTTGGGTTCAAACCCTAAAAGCTGAGGAGCTGATCCTGACCTGGAGAT
CCGTTTCAGCAGGGATGTGTCTGTCTCCGTGCTGACTCTTAGTGAGGTTTAGAATAGTGTGA
CAAACTCAGAGTTACAGCAGAGGGCGCTGTCAATCTAAAGTCCTAAAGCGCAGGAAAGGTTT
GTGATATGCTGGGTGGATTTCAGCTTAAAACATAGTAAAATAATTGAGAAAGGGTCTACACC
ATATCTTGGCTTATAACCTGAAATAAAACAGAATCTGTAATATTTTACCTTGAAATATCACT
GTGTCCTGAAATGCA
 Example E5 insulator sequence (forward) (SEQ ID NO: 58) 
GAACAGAAGTGTGGGGATGGGGAGCCAAGCCTCTGCTTTGGGGCCACTTGGATGGACCT
GTGGCCCCTAGCGCCTGAGCGCTCCGCCTGTGTGCATCCTGCCTGGCAGCAATCGGTGGTCA
CCTCCGTTGTCCAGCAGCTTCCCGCGGTAGATCTTGTCCTCCACCACGTCGGTCCAGTAGAG
GGCGCTCTGGCTGAGGTGGAAGTCCAGGGCGATGGTGTTGCGCAGGCCGGGCACCAGGACGC
TGTAGTCTCCTTTGTGAAGATCGATGCGCCGGATTTCATGGCGGTTGGAGAAAATGATGAAC
GGCTTGAAGG
 Example F1 insulator sequence (forward) (SEQ ID NO: 59) 
TGCCTTTCAGCTCCAAATCTACCCTTATATTACCTGTTCTGAAATAACAGACAAGACTCTTT
GAAGTCTTTCCTCTGTAGTAAGCATGATGCTAAGTTTGTCAGTAGAGGGCGCTGGAGGGCCG
TGGCAGGAGGAAGGAGTTTCCTTTCACGGTTCCAGTAAGTTGCAGGTTTTTTGCTTCTTGCT
 
CTTTCTGCGTTGCTGCCAAACTGCACTGTCCCAGCCATGCACCCAGACTGTGCTGCCTGACT
TCCTGCAAACTCAAGGCCCCATCTTAGCTCAGTAATCACCTCACTGTGGCCC
 Example F2 insulator sequence (forward) (SEQ ID NO: 60) 
CAATCTCACCCAACCCTTTCATTTTACAGGAGACTAAAGTCCCGAGGATAAGTTAAATGAGG
CTAGTGGCAGAGGCAGAATGGTCGATTTCTTAACATCTCAGCTCCCCGGCTCTGCCATCCTG
TGAGTTTCTCCTTCACAGGTCGATGTTAAGTTTTGCCAGTAGAGGGCGCTGGCGGGACACGG
TGGGAGGCGGGAACTCCTCTTTCTGCCTCCAGGGTGCAGTGTGCTGCTGCTGCTGCGTGTTC
CTTTGCTGCTGCTGCAGGGTCTGTTCAGGGATGCTCTGCCCCAGCGGTGCACCCAGAGTTCC
TCAGTGGCTGACC
 Example F3 insulator sequence (forward) (SEQ ID NO: 61) 
TCGGACATTTCCCTGTCTCTTAAACCCAGTTTTGCCGCTTTGTCCTGTCTTGGATTCCGCAC
GCTGCACAAAAAATGGCCAGTAGAGGGCGCTGCTGGCTTACTTTTAGAAAACTGCTCACAG
CCTGATTTCAGCACTTTCAAAAACATGGAAATTATCTTAATTCAGTCAAAGCAATCAGAACG
AGAAAACTGTCTTAAATTCATCAAAGTTGTTTCTTTGTGTCTTTTTCTATTTCGTTTCCCCA
AAGGCATCAATA
 Example F4 insulator sequence (forward) (SEQ ID NO: 62) 
TTTCCCTACTTCTCCCACCATTTTAAAAACAAGGAAACGAAGCCTAGATTTGCAAGAATAAA
AGGTTTGGGTTTATTTACCTGTTGGACTCTATACCAGTTATTGATTTATTGCCTCTCACCTC
TGAATTCACCTTATTTGACTGCTTTGCGAAAATAAATCTGAACCTTTTCAAGAATTTGCCGT
TGGCACCGAAGTTTTGCCAGTAGAGGGCGCTCGAGAAACATTGCAGGAGAAAGAGTTTTGCC
TCCGGCTCGGTGGAGTCTCAGCGGGGTTGGCAGGGGCGCGTGGTTTCGCTGGCCCCCAGCGC
CTTTGGCTCACAGGTTCTCCAGGGCCCTCCTGCTGCCATACACAGCCAGCAACACTCAGTGG
CCAGTGGCTTTCCCCTGCAACCTCCTAAGGTGGTTTGTAGCCAAGGCGCGGTAGTGAGACAT
CTTCCCATAAACAGCTTTCCCCAGCACCCTACAGGACTGATTTCTAGCAAGTTTCACCATTA
TGGCATCACAGCAACTTGTCCGCCGTTCAGTAGCCATGACTGAGCCA
 Example F5 insulator sequence (forward) (SEQ ID NO: 63) 
CCTTCCTTTCTAAATGACGAGAGAGACAGAAGAATTCTTCAAGGTTAGTGTGTCCAGCATGC
AACCTTTCCTTCCTGGATGAGCATCCCTGGAATAGGAGAGCCAGCCTGCCTCCTGCGCTGGC
ACAGAGCCCGGTTCCCTAGACAACTGCCTCTCCAAATCTGATGTCCAGCGCCACCTGGTGTC
CACATCAAGCAGACACAATTAATAGTCAACCTGTTCAGGAAAACTGTGAGGGGGAAAAAAAA
GAAAGAGGATTTATGAAGGGAAAAGAAAGTTTAGAGGATATGCCACGATTGGAATGTAATGA
GTAGAACCAG
 Example A1 insulator sequence (reverse) (SEQ ID NO: 64) 
AGAGCGAGATTCCGTCTCAAAGAAAAAAAAAGTAATGAAATGAATAAAATGAGTCCTAGAGC
CAGTAAATGTCGTAAATGTCTCAGCTAGTCAGGTAGTAAAAGGTCTCAACTAGGCAGTGGCA
GAGCAGGATTCAAATTCAGGGCTGTTGTGATGCCTCCGCAGACTCTGAGCGCCACCTGGTGG
TAATTTGTCTGTGCCTCTTCTGACGTGGAAGAACAGCAACTAACACACTAACACGGCATTTA
CTATGGGCCAGCCATTGT
 Example A2 insulator sequence (reverse) (SEQ ID NO: 65) 
GGGAGAGGTGGTTCAACAAAGAACAATGGATGCTGTTACCAGAAGAAGGAATAAGATGCTG
GCCAGGTGAAAAGACCAATTTCACGAGAGAACCTTAAATTACCAGCGCCACCTGGTGCATCA
AAACTCCTCTGAAGCCGGGCGCAGTGGTTCACACCTGTAATCTCAGCACTTTGGGTGGTTGA
GGCAGGAGCATTGCTTGAGCCCAGGGGTTGGAGACCAGCCTGGGGAACATAGGGAGACCAAC
CCCT
 Example A3 insulator sequence (reverse) (SEQ ID NO: 66) 
TGCTTGTCCTTCCTTCCTGTAACACAGCCATTAAACCAGGAGCATCGCCCTTCCCCGGCCCT
CAGGTAAGAGGACCAAATACCGTAGCCGTTTCCAATTTCAGTCCTTTAGCGCCACCTGGTGC
TAACTACTCTATCACGCTTTTATCCAATAACTACCTTTGTAAATTTCCTTTCAAAAGTTCTG
GCCGGGCGCGGTGGCTCACGCTTGTAATCCCAGCACTTTGTGAGGGGTCAGGAGTTC
 Example A4 insulator sequence (reverse) (SEQ ID NO: 67) 
GTAGAGACGGGGTTTCACCATGTTGGCCAGGCTGGTCTCGAACTCCTGACCTCAGGTGATCC
ACCCGCCTCGGCCACCCAAAGTGCTGGGATTACAGGCGTGAGCCACTGTGCTGGGCCTGCCA
GGAATTTCATAGGTCACCTGCAGAGGCAGGGACACCACGAGGCAACCTCAGTGTGACCCCTG
CTGGCAGGTCTCCGCAGCGTTCTGGTGACACTGCTTTTTCCAAGTGGTCACGAGGCCCGGCA
GCGCCACCTGGTGGAGCTATGGAGACACGGAGTCGTCCAAGTCCTGGGAAACAGAATCCTAG
AATCTCTGGATTTTGAACATTCTTGAAGGTGATGGAGTGGGA
 Example A5 insulator sequence (reverse) (SEQ ID NO: 68) 
AGCCATGGAATAAAGTGCATCTTTTTCTAACATAGAATATATTACTCCTGAAATAGTAGGAA
ATGTTGATAATTGCCTTTTATTGGACTTACAAATAATTTATAAGTTGGTCCGGTTAAAAAAA
AAAAGTATCATAGTCTTTTATTTCTTTCGTTTCTTCCTCCAGGCTAGAAGCAACCTCTAAAA
ATAGCTACCTGTGCAGGGATGGGCCTAGCGCTCCCCCTGGTGGTCATAGTGCAGGATTGCGG
CTTCCGTAAATGGACCTGAAGCGGCAGACCCTGCATTTGAATTAATTCTTTCTCCCCTAAGT
ACACAGAGAAATGTGATTTGTTCTTCCCAGTCATGCCT
 Example B1 insulator sequence (reverse) (SEQ ID NO: 69) 
CAGGGCTCTCCTGCAAATAGGCCCTTGGAGGCCTGGCCTGACGTTTAGGTCCTGAGACAGGT
GTGCAGATCAGACCCCTCATGGCAACACCAGGCTTCTGGCTCCAGTCCTTGGAGTCCGTCTG
CGTCCCAGCAGCTCCCCCTGGTGGCCAGAAGCAAACGCTTCATGTCTTGCATCTAGAGCCAA
AGCATCTCCTACCCCTGGATTCCCATTCCGGAATAGTTTTCAGACCAAGGCTCCTCCGCAAA
TATGAGTGATGTGTCAGATGAGTGATGAGGGACAGGCCAGGGGCACACACGTCGCATCCACT
TCTTGGGGCTCTT
 Example B2 insulator sequence (reverse) (SEQ ID NO: 70) 
CCTCCACAACCCATGAAGTCCACATTCTCTTGCTTCCAGCAGATCTGCACCCTCACCACCAT
GCTGCCATCTGGCTGGCCCATACCTGGAACAGTCTTGTCTAGCACATTTGCTCAGGAGGGAG
AGGAGAGGGGATGGACCAGAGTGATGGGAGCCCAGGGCTGCAGGGCAGCTCCCCCTGGTGGA
CCCTAGAGGTGGCCCCTCCCACTCCTGGGAGAAGAGAAGCCACACTGTGCATTTCGCAGCCA
ATCAAAACACAACCCTCCAATAGAGGGCGGAATGGGGTTACCAGAAGCTGGGAAAAGCAGGG
GAGGAGAATGAAGAGCAGGTGGATGG
 Example B3 insulator sequence (reverse) (SEQ ID NO: 71) 
GGATGCTGGAGGAGGTCACCTGGCTCAGGACCAGGCTGGTGGGGCAGGGAGGGTGTCCAGG
GCTGGAGCCGCTGCTGCTGGAGTAGCGGGAGTGGGGGCAGGTGGTCACTGCACGGGCAGCT
GTGTCCACGCTCCCCCTGGTGGCCACTCACAGGAGGGACAGGAAAGCTGCTCCCCAGCCCTT
CTGTGGACAGATGTAAGGGGGTGCTGCAGACCCCCTCAGAGCCACAGGAGCCGGCCTCAGGT
ACCACACGGGAG
 Example B4 insulator sequence (reverse) (SEQ ID NO: 72) 
ACAGGGCTGCTTGTACCACTCTGCTGCCTCTGAACTGGGGTCCTGGATTTGTGCCAGTGACC
GGGTGGGAAGCCCCTGGCTTGCCCCAGCAGGTCTGGCTTCCTGCAGTGGGGCCAGAAATATG
AGCTCTGGCTCCCCCTGGTGGCAAGACCAGAAACGAGCTCAGCGTGACTGTCCAGTGTCAAA
GATGTGGGAGGAAGCAGCGGAGAAACCAAAGGAGGAGGAAGACGCCACCATCACTGTCCCCA
GCCATGCAGCCTAAAAGTCACTATCCCTTCCTCTCTTAGAGTCTTTTGGTTCAGTTTCCGC
 Example B5 insulator sequence (reverse) (SEQ ID NO: 73) 
AGGATGGGCTATGAGGTGGTCGAGGGACCATGCATTCTGGGTGCACAGCTGAGGAAAAGCCC
CAGAGGATGTCCCCCCGCGTTCATACCCTAAGAGAAGATGCAACAGGAACAAGAAATTGCAC
AACAGAGCTCTGTGGACCATGAAAAGGCCCCTTCCTCCACAGTGTCCCTCCGGCGCCCTCTA
CTGACAAAGCTTGATATCGTGCTTGCTGCAAAGAAGGGCTTAGAGTCCAGTCCATCAAGGAG
CAGGCACTCACGGGGAAGTTGGAAGCTGAGAGGCAAGAAGAAACCAGGGCTCTGCAAACTAC
GGCCACCATTCAGAC
 Example C1 insulator sequence (reverse) (SEQ ID NO: 74) 
GTTAGCCCTGAGTGCCCATAAGAATCACTGGGATCTGCCACAGGGGTACCACTGAAGATTA
ATAGAATATGGTCCACAGGGATGCCACCAAAACTTGATGGGGCTGCGCACCAGTGGCTGTCC
CCCAAATGCCACGCTCCAAGTGTATACCAGAATCCAGAATCCGGAAGAGAAGGCCCCCTCCT
GACATGTTCCTGCAGCGCCCTCTACTGAAGGAGCTAAATATTGCACAAAGTGGCAAGGCGGA
CACGTTTACAGGGTCCCTCTCCATTATCACAGAGCAAGCAATGAAAGGTGGATGCGAAAC
 Example C3 insulator sequence (reverse) (SEQ ID NO: 75) 
TGGGCCTAGCTCAAAAGAAAGCATGTGCATAGGAGCCCAGCAACAGAGAAAGCAAGCTGTGC
TCTGGAGGAGGCAAAAAGATGAGGCACCCCAGAATCTGGAAGATAAAACTTTTTTCTCCTCT
GGTCTCTCTCTCTCTAGCGCCCTCTACTGACAAGGATTTACATTATGCCAGCTGGTAAGAAA
TATTTACAAAGCCCATCTCCATTATCACAGAGCAGTTAAAGGAGGATGCCTTTGGAGCTGAG
AATGCATTGATAAGTGGCGTGGACACTCACAGACTTACTGGGTTGAAAGAAACGCTAATCTA
ATGTCTTCCTATTTCCAATCCACATCCCACTTGATGATTGAATTCCTCCTACGAAATCCTAC
CAGGCAGTGCTTCT
 Example C5 insulator sequence (reverse) (SEQ ID NO: 76) 
AGTGGTTTTGGGGTTTTCCATAGGATATTGGAACTGGTGGAATTTTGGGAGTTAACCAGCT
TAACCCCAGTTCAAAGGTATGAAGTGACTTCTTCAAAACAAACCAAAATGAACACACTTTCC
CACCAAAGAATATTAGTTTAGAATTGAACGTTTGTTCACCCTGTGTAAACCAATGCTGCCCC
CTAGTGGCGAAGGTGGCAAGTTCAAGCAAGTCACCTTGAGGGCCAGGAAATTAGTAATTAGG
CTAATATAATTTCCCAAAGAGCATTGCAAAAGA
 Example D1 insulator sequence (reverse) (SEQ ID NO: 77) 
CTGTTGAACCCCAAACTGCTCCAACTGGCAGGAAGCAGCCCCCTGCCCCCCCCACCCAGGG
CAACCTACACCAGCTGTGTGGCTCCCCAGCCCACAGCCTTGGAGGCCGAGATGAGGCCTTCG
GTCCAGCGCGAGTTCTTGGCGTAAAATTCCTGCTGCGTGGCTGCCCCCTAGTGGCGACGGGA
GAACAGGGCAGGACATGCCAGTCAAGGACACCAAAGGTGGAAAGTGGCCCTCAAGGCCCTC
TCCCCCTCACTCTGGCCATAGGGCGCATCCCTGAGGCCTCCCTGTGGTAGGGGGTGGAGGGT
CTC
 Example D3 insulator sequence (reverse) (SEQ ID NO: 78) 
AATGAGGCCTGCCACATAAGCTGAGAAATTTGAGTGCTCCTGCTATGGTTGAGAGCCTGCCA
GTCCACCCTAAACTCCGTCAAAGTCTTTCCCCACTTGTATGAGGCTGGAGTAGCTTGGCGGC
CTCTCAAGCCTGAGGGTACAAAATAGTGCCCCTTAGTTAATTGCATTAACTCTTTCTCACTG
CCCGGTATATATGACACATGCCTCCTGCCCCCTAGTGGCTGTTGTGGATTCTGCACACTCCC
AGGAGCTAAACAAGAATTCTGTTTCTGGAGGGAGCCTTACCGGGTATAGCTCAAAACAAGCA
CAGACAGATCCAATCTGAAAAGCACATTTGCATATGAAGGTACCATTATTTACTCTCTTATG
CTTCTGTATTTCTAGCAGCTCTGCCCGCCCCCCCCATAGGAACTTACAGAGCAGTGCTTTAG
AGAAAAGCAAATCAAACACGCC
 Example D4 insulator sequence (reverse) (SEQ ID NO: 79) 
CCCCTTTCCCTAAACTGGAGAAAAAGGGGGTGAAGAGGTGCTCGAATCGCCATCCTCCAACG
TAAGTCATCTTGAAGGATGGAGCAGAGCTCCTCCAAGCCAGGCCAAGTCCCCGAGCGCAAGT
GCCAAAGCTGCAGCCCATTCGTTACCACGGTGCCTGCTGCCCCCTAGTGGCCGCCACCCTGA
CATGCAAGAGGAAGATACGGAGCTACCCAACCAGTGGAGAAGGAAGAGGACTGGGGAAAAC
 Example D5 insulator sequence (reverse) (SEQ ID NO: 80) 
ATCAGCGGTGTCAGTCTTCCTGGGTCCCCCTGAAAATTCCAAAGAGGAAGCCATGGTGGGAC
CGCGTGGGACGCAGGGTCTAGACTCCCAGTCTGGTCTGATTTTCTTGTTTGCATCCTTGCCG
CTTTTCCTTCTGGAGAGTTCAGTGGGCAGGGACTGATGGGGCCACAGCGCCCTCTGCTGGCG
TGTTCTCACAGTCGACTTTTGCCTGCCCGAGCTTATACATACAGCGCCCTCGGAATTGGAGG
GCTTGGGGCAGACTGAGAATGGGGATTTCTGGTTTTATTCCAGGCCCACTCCTTAGTGAATG
TCAAGACCAAAGGTGGC
 Example E1 insulator sequence (reverse) (SEQ ID NO: 81) 
AGTTTGCAGGTGGCTTGACTGAAAAAAAAAAAGAAAAAGAAAACACCTACTTTCCTCTCCAT
GGAAACAGCATGCCAGAAAATTTTGTGGACCCTTGAAATGAGCACACATCTCACTTGCAAAA
GCACAGCACCAGCGCCCTCTGCTGTTTCCTGGTTTGATTTAGAACTCAGAGAAGCTACAGTA
CTTTCTAGACTAAAATACCATGTAGAGTTCAGGATAATTATATTCTAGATTAGACATAGGCA
AGCACATTTATATTAGTACATTCTGTAGTATATTCCAGAGTGAAGGAAATCAAA
 Example E2 insulator sequence (reverse) (SEQ ID NO: 82) 
GGCTGGCTTAAATGGTCTGAAATGATCCTAAATATTCTTCACTTGGATTATTTGCAGGTCAG
TCTCTCTCTGTGGAACTAAACAATAAATCTCTGCCTTTCTGAACTGACTAGGAGATTAAGGG
AGACAATTGTAGAGCTGCTTTTGCGTCTGAACTGACTTTCCCTTGCTTCACCAGCCCTCGTT
TGCTTTGTTGAATCAGAGGCTTGGGCACAGCTGTTTAGCGCCCTCTGCTGGGTGAAAAGCAG
CCTAACGGTTTTACATTATGCCTCAATTTGTACTGGTTGTTTTTCTGCCAACGCATACTTGA
AGATTGCCTTTGCATAGGGCTCTTAATACCTTGCCTAAAGGAATGAGGGGTTGAGTGGAGTA
AGGGGGTG
 Example E3 insulator sequence (reverse) (SEQ ID NO: 83) 
TGGAATTTGTGTTGACATTGAAACTTCTTACTGTAAATATTACTTTGAATAGTACCTATTTA
ATCCTGCTCACATTTAATGTCATATTAGGGAACGTCCTTTCTATAGAATTTTTAACAATTCC
CTTTAAAAAGGGATTCTGAAGGTTTTCTTCTCTCACTCTTCTCCATTCTCATAAATATGTTT
CTATATGTTTTAAGTCTTAGGCATATCGGACATCACTAGTGCATCAGCGCCCTCTGCTGGTT
CAGTAAGAATGGTTTCCCCATATACTGGGCAAAACTGGATTTTTGTGGTGATGAAAGGAAA
AAAACAAATTCAGTACAATTGGAAAGCTGGTGTTGTTTTAAAACTCTTGAAAAACACTGAAT
GAAAGGAATCACACTAAAACTATATGTTGCAATGTTGTTGGTTAATACTTATTAATAACAGT
GGGTTAGGAAATATGCATTGGCACATTCTTTTGA
 Example E4 insulator sequence (reverse) (SEQ ID NO: 84) 
TGCATTTCAGGACACAGTGATATTTCAAGGTAAAATATTACAGATTCTGTTTTATTTCAGGT
TATAAGCCAAGATATGGTGTAGACCCTTTCTCAATTATTTTACTATGTTTTAAGCTGAAATC
CACCCAGCATATCACAAACCTTTCCTGCGCTTTAGGACTTTAGATTGACAGCGCCCTCTGCT
GTAACTCTGAGTTTGTCACACTATTCTAAACCTCACTAAGAGTCAGCACGGAGACAGACACA
TCCCTGCTGAAACGGATCTCCAGGTCAGGATCAGCTCCTCAGCTTTTAGGGTTTGAACCCAA
ATACTGACAGTAACTCAAATCCGGGCAGGCTCTGGGGACCAGTGTTCACTGACTGACTGCCA
GGATTTTTCTTTCCATCCCACCCTCTCCCCTGAGTTCCTGGGCGCCTGCATTCAGACCCTTA
GACGATATATAATGGGCATTTTAAATTTAGCACTTCCCAACCCGACCTCTTGACTCCACCAC
TCTCAGCCCAAGTGT
 Example E5 insulator sequence (reverse) (SEQ ID NO: 85) 
CCTTCAAGCCGTTCATCATTTTCTCCAACCGCCATGAAATCCGGCGCATCGATCTTCACAAA
GGAGACTACAGCGTCCTGGTGCCCGGCCTGCGCAACACCATCGCCCTGGACTTCCACCTCAG
CCAGAGCGCCCTCTACTGGACCGACGTGGTGGAGGACAAGATCTACCGCGGGAAGCTGCTGG
ACAACGGAGGTGACCACCGATTGCTGCCAGGCAGGATGCACACAGGCGGAGCGCTCAGGCGC
TAGGGGCCACAGGTCCCATCCAAGTGGCCCCAAAGCAGAGGCTTGGCTCCCCCATCCCCCAC
ACTTCTGTTC
 Example F1 insulator sequence (reverse) (SEQ ID NO: 86) 
GGGCCACAGTGAGGTGATTACTGAGCTAAGATGGGGCCTTGAGTTTGCAGGAAGTCAGGCAG
CACAGTCTGGGTGCATGGCTGGGACAGTGCAGTTTGGCAGCAACGCAGAAAGAGCAAGAAGC
AAAAAACCTGCAACTTACTGGAACCGTGAAAGGAAACTCCTTCCTCCTGCCACGGCCCTCCA
GCGCCCTCTACTGACAAACTTAGCATCATGCTTACTACAGAGGAAAGACTTCAAAGAGTCTT
GTCTGTTATTTCAGAACAGGTAATATAAGGGTAGATTTGGAGCTGAAAGGCA
 Example F2 insulator sequence (reverse) (SEQ ID NO: 87) 
GGTCAGCCACTGAGGAACTCTGGGTGCACCGCTGGGGCAGAGCATCCCTGAACAGACCCTGC
AGCAGCAGCAAAGGAACACGCAGCAGCAGCAGCACACTGCACCCTGGAGGCAGAAAGAGGAG
TTCCCGCCTCCCACCGTGTCCCGCCAGCGCCCTCTACTGGCAAAACTTAACATCGACCTGTG
AAGGAGAAACTCACAGGATGGCAGAGCCGGGGAGCTGAGATGTTAAGAAATCGACCATTCTG
 
CCTCTGCCACTAGCCTCATTTAACTTATCCTCGGACTTTAGTCTCCTGTAAAATGAAAGGG
TTGGGTGAGATTG
 Example F3 insulator sequence (reverse) (SEQ ID NO: 88) 
TATTGATGCCTTTGGGAAACGAAATAGAAAAGACACAAAGAAACAACTTTGATGAATTTA
AGACAGTTTTCTCGTTCTGATTGCTTTGACTGAATTAAGATAATTTCCATGTTTTTGAAAGT
GCTGAAATCAGGCTGTGAGCAGTTTTCTAAAAAGTAAGCCAGCAGCGCCCTCTACTGGCCAT
TTTTTGTGCAGCGTGCGGAATCCAAGACAGGACAAAGCGGCAAAACTGGGTTTAAGAGACAG
GGAAATGTCCGA
 Example F4 insulator sequence (reverse) (SEQ ID NO: 89) 
TGGCTCAGTCATGGCTACTGAACGGCGGACAAGTTGCTGTGATGCCATAATGGTGAAACTTG
CTAGAAATCAGTCCTGTAGGGTGCTGGGAAAGCTGTTTATGGAAGATGTCTCACTACCGC
GCCTTGGCTACAAACCACCTTAGGAGGTTGCAGGGAAAGCCACTGGCCACTGAGTGTTGCT
GGCTGTGTATGGCAGCAGGAGGGCCCTGGAGAACCTGTGAGCCAAAGGCGCTGGGGGCCAGC
GAAACCACGCGCCCCTGCCAACCCCGCTGAGACTCCACCGAGCCGGAGGCAAAACTCTTTCT
CCTGCAATGTTTCTCGAGCGCCCTCTACTGGCAAAACTTCGGTGCCAACGGCAAATTCTTGA
AAAGGTTCAGATTTATTTTCGCAAAGCAGTCAAATAAGGTGAATTCAGAGGTGAGAGGCAAT
AAATCAATAACTGGTATAGAGTCCAACAGGTAAATAAACCCAAACCTTTTATTCTTGCAAAT
CTAGGCTTCGTTTCCTTGTTTTTAAAATGGTGGAGAAGTAGGGAAA
 Example F5 insulator sequence (reverse) (SEQ ID NO: 90)

In some embodiments, an insulator element comprises or consists of a nucleotide sequence selected from the group consisting of SEQ ID NOs: 37 to 90, 102 or 103, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. In some embodiments, an insulator element comprises or consists of a nucleotide sequence selected from the group consisting of SEQ ID NOs: 37 to 90, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise a CTCF-binding motif (e.g. any of SEQ ID NOs: 23 to 28) and one or more variation is made in the flanking sequences.

In some embodiments, an insulator element comprises or consists of a nucleotide sequence selected from the group consisting of SEQ ID NOs: 37 to 90, 102 or 103.

In some embodiments, an insulator element comprises or consists of a nucleotide sequence selected from the group consisting of SEQ ID NOs: 37, 38, 43, 55, 64, 65, 70, 82, 102 or 103 or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. In some embodiments, an insulator element comprises or consists of a nucleotide sequence selected from the group consisting of SEQ ID NOs: 37, 38, 43, 55, 64, 65, 70, 82, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise a CTCF-binding motif (e.g. any of SEQ ID NOs: 23 to 28) and one or more variation is made in the flanking sequences.

In some embodiments, an insulator element comprises or consists of a nucleotide sequence selected from the group consisting of SEQ ID NOs: 37, 38, 43, 55, 64, 65, 70, 82, 102 or 103.

### A 1 insulator elements

In some embodiments, the polynucleotide of the present invention comprises one or more A1 insulator element. An A1 insulator element may comprise or consist of a core A1 insulator sequence.

In some embodiments, an A1 insulator element comprises or consists of the nucleotide sequence of SEQ ID NO: 29 or 33, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences.

In some embodiments, an A1 insulator element comprises or consists of the nucleotide sequence of SEQ ID NO: 29 or 33.

In some embodiments, an A1 insulator element comprises or consists of the nucleotide sequence of SEQ ID NO: 37 or 64, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise CTCF-binding motif A (e.g. SEQ ID NO: 23) and one or more variation is made in the flanking sequences.

In some embodiments, an A1 insulator element comprises or consists of the nucleotide sequence of SEQ ID NO: 37 or 64.

In some embodiments, the polynucleotide of the present invention comprises one or more core A1 insulator sequence. In some embodiments, a core A1 insulator sequence consists of the nucleotide sequence of SEQ ID NO: 29 or 33, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences. In some embodiments, a core A1 insulator sequence consists of the nucleotide sequence of SEQ ID NO: 29 or 33.

In some embodiments, the polynucleotide of the present invention comprises one or more full-length A1 insulator sequence. In some embodiments, a full-length A1 insulator sequence consists of the nucleotide sequence of SEQ ID NO: 37 or 64, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences. In some embodiments, a full-length A1 insulator sequence consists of the nucleotide sequence of SEQ ID NO: 37 or 64.

### A2 insulator elements

In some embodiments, the polynucleotide of the present invention comprises one or more A2 insulator element. An A2 insulator element may comprise or consist of a core A2 insulator sequence.

In some embodiments, an A2 insulator element comprises or consists of the nucleotide sequence of SEQ ID NO: 30 or 34, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences.

In some embodiments, an A2 insulator element comprises or consists of the nucleotide sequence of SEQ ID NO: 30 or 34.

In some embodiments, an A2 insulator element comprises or consists of the nucleotide sequence of SEQ ID NO: 38 or 65, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise CTCF-binding motif A (e.g. SEQ ID NO: 23) and one or more variation is made in the flanking sequences.

In some embodiments, an A2 insulator element comprises or consists of the nucleotide sequence of SEQ ID NO: 38 or 65.

In some embodiments, the polynucleotide of the present invention comprises one or more core A2 insulator sequence. In some embodiments, a core A2 insulator sequence consists of the nucleotide sequence of SEQ ID NO: 30 or 34, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences. In some embodiments, a core A2 insulator sequence consists of the nucleotide sequence of SEQ ID NO: 30 or 34.

In some embodiments, the polynucleotide of the present invention comprises one or more full-length A2 insulator sequence. In some embodiments, a full-length A2 insulator sequence consists of the nucleotide sequence of SEQ ID NO: 38 or 65, or variants thereof having at least 80.0%, at least 82.5%, at least 85.0%, at least 87.5%, at least 90.0%, at least 92.5%, at least 95.0%, or at least 97.5% sequence identity thereto. In some embodiments, a full-length A2 insulator sequence consists of the nucleotide sequence of SEQ ID NO: 38 or 65. In some embodiments, a full-length A2 insulator sequence consists of the nucleotide sequence of SEQ ID NO: 102 or 103, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences. In some embodiments, a full-length A2 insulator sequence consists of the nucleotide sequence of SEQ ID NO: 102 or 103.

In some embodiments, the polynucleotide of the present invention comprises one or more core A2 insulator sequence described below in the section entitled "Variant A2 insulator elements".

### B2 insulator elements

In some embodiments, the polynucleotide of the present invention comprises one or more B2 insulator element. A B2 insulator element may comprise or consist of a core B2 insulator sequence.

In some embodiments, a B2 insulator element comprises or consists of the nucleotide sequence of SEQ ID NO: 31 or 35, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif B (e.g. SEQ ID NO: 24) and one or more variation is made in the flanking sequences.

In some embodiments, a B2 insulator element comprises or consists of the nucleotide sequence of SEQ ID NO: 31 or 35.

In some embodiments, a B2 insulator element comprises or consists of the nucleotide sequence of SEQ ID NO: 43 or 70, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise CTCF-binding motif B (e.g. SEQ ID NO: 24) and one or more variation is made in the flanking sequences.

In some embodiments, a B2 insulator element comprises or consists of the nucleotide sequence of SEQ ID NO: 43 or 70.

In some embodiments, the polynucleotide of the present invention comprises one or more core B2 insulator sequence. In some embodiments, a core B2 insulator sequence consists of the nucleotide sequence of SEQ ID NO: 31 or 35, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif B (e.g. SEQ ID NO: 24) and one or more variation is made in the flanking sequences. In some embodiments, a core B2 insulator sequence consists of the nucleotide sequence of SEQ ID NO: 31 or 35.

In some embodiments, the polynucleotide of the present invention comprises one or more full-length B2 insulator sequence. In some embodiments, a full-length B2 insulator sequence consists of the nucleotide sequence of SEQ ID NO: 43 or 70, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif B (e.g. SEQ ID NO: 24) and one or more variation is made in the flanking sequences. In some embodiments, a full-length B2 insulator sequence consists of the nucleotide sequence of SEQ ID NO: 43 or 70.

### E2 insulator elements

In some embodiments, the polynucleotide of the present invention comprises one or more E2 insulator element. An E2 insulator element may comprise or consist of a core E2 insulator sequence.

In some embodiments, an E2 insulator element comprises or consists of the nucleotide sequence of SEQ ID NO: 32 or 36, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif E (e.g. SEQ ID NO: 27) and one or more variation is made in the flanking sequences.

In some embodiments, an E2 insulator element comprises or consists of the nucleotide sequence of SEQ ID NO: 32 or 36.

In some embodiments, an E2 insulator element comprises or consists of the nucleotide sequence of SEQ ID NO: 55 or 82, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise CTCF-binding motif E (e.g. SEQ ID NO: 27) and one or more variation is made in the flanking sequences.

In some embodiments, an E2 insulator element comprises or consists of the nucleotide sequence of SEQ ID NO: 55 or 82.

In some embodiments, the polynucleotide of the present invention comprises one or more core E2 insulator sequence. In some embodiments, a core E2 insulator sequence consists of the nucleotide sequence of SEQ ID NO: 32 or 36, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif E (e.g. SEQ ID NO: 27) and one or more variation is made in the flanking sequences. In some embodiments, a core E2 insulator sequence consists of the nucleotide sequence of SEQ ID NO: 32 or 36.

In some embodiments, the polynucleotide of the present invention comprises one or more full-length E2 insulator sequence. In some embodiments, a full-length E2 insulator sequence consists of the nucleotide sequence of SEQ ID NO: 55 or 82, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif E (e.g. SEQ ID NO: 27) and one or more variation is made in the flanking sequences. In some embodiments, a full-length E2 insulator sequence consists of the nucleotide sequence of SEQ ID NO: 55 or 82.

### Insulator element pairs

In some embodiments, the polynucleotide comprises a pair of insulator elements flanking the expression cassette. Suitably, the polynucleotide comprises from 5' to 3' direction: a first insulator element, an expression cassette, and a second insulator element.

A pair of insulator elements may be used to flank the expression cassette, thereby generating a functionally independent domain, protected from position effects. Regulatory interactions can occur with the domain but the insulator elements may block external regulatory signals.

The pair of insulator elements may have the same or different orientations. A pair of insulator elements may be arranged in different orientations, such as a convergent orientation, a parallel orientation or a divergent orientation (see e.g. Ghirlando, R. and Felsenfeld, G., 2016. Genes & development, 30(8), pp.881-891).

In some embodiments, the pair of insulator elements is in a convergent orientation. As used herein, a "convergent orientation" may refer to a forward-reverse pair of insulator elements, e.g. in which the first insulator element is in a forward orientation and the second insulator element is in a reverse orientation.

In some embodiments, the pair of insulator elements is in a parallel orientation. As used herein, a "parallel orientation" or "tandem orientation" may refer to a forward-forward pair of insulator elements, e.g. in which the first insulator element is in a forward orientation and the second insulator element is in a forward orientation; or a reverse-reverse pair of insulator elements, e.g. in which the first insulator element is in a reverse orientation and the second insulator element is in a reverse orientation.

In some embodiments, the pair of insulator elements is in a divergent orientation. As used herein, a "divergent orientation" may refer to a reverse-forward pair of insulator elements, e.g. in which the first insulator element is in a reverse orientation and the second insulator element is in a forward orientation.

The pair of insulator elements may both have the same or different sequences. Suitably, both of the insulator elements binds CTCF. Suitably, both of the insulator elements comprises a CTCF-binding motif.

### A 1-A 1 insulator pairs

In some embodiments, the polynucleotide comprises an A1-A1 insulator element pair flanking the expression cassette. In some embodiments, the A1 insulator elements are in a parallel orientation.

In some embodiments, the A1 insulator elements each comprise or consist of a core insulator sequence. In some embodiments, the A1 insulator elements each consist of a core insulator sequence. In some embodiments, the A1 insulator elements each comprise or consists of the nucleotide sequence of SEQ ID NO: 33, or a nucleotide sequence having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences. In some embodiments, the A1 insulator elements each comprise or consist of the nucleotide sequence of SEQ ID NO: 33.

### A2-A2 insulator pairs

In some embodiments, the polynucleotide comprises an A2-A2 insulator element pair flanking the expression cassette. In some embodiments, the A2 insulator elements are in a parallel orientation.

In some embodiments, the A2 insulator elements each comprise or consist of a core insulator sequence. In some embodiments, the A2 insulator elements each consist of a core insulator sequence. In some embodiments, the A2 insulator elements each comprise or consists of the nucleotide sequence of SEQ ID NO: 34, or a nucleotide sequence having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences. In some embodiments, the A2 insulator elements each comprise or consist of the nucleotide sequence of SEQ ID NO: 34.

In some embodiments, the A2 insulator elements each comprise or consist of a full length insulator sequence. In some embodiments, the A2 insulator elements each comprise or consist of the nucleotide sequence of SEQ ID NO: 103, or a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise CTCF-binding motif A (e.g. SEQ ID NO: 23) and one or more variation is made in the flanking sequences. In some embodiments, the A2 insulator elements each comprise or consist of the nucleotide sequence of SEQ ID NO: 103.

### B2-B2 insulator pairs

In some embodiments, the polynucleotide comprises an B2-B2 insulator element pair flanking the expression cassette. In some embodiments, the B2 insulator elements are in a parallel orientation.

In some embodiments, the B2 insulator elements each comprise or consist of a core insulator sequence. In some embodiments, the B2 insulator elements each consist of a core insulator sequence. In some embodiments, the B2 insulator elements each comprise or consists of the nucleotide sequence of SEQ ID NO: 35, or a nucleotide sequence having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif B (e.g. SEQ ID NO: 24) and one or more variation is made in the flanking sequences. In some embodiments, the B2 insulator elements each comprise or consist of the nucleotide sequence of SEQ ID NO: 35.

### A 1-A2 insulator pairs

In some embodiments, the polynucleotide comprises an A1-A2 insulator element pair flanking the expression cassette. In some embodiments, the first insulator element is an A1 insulator element and the second insulator element is an A2 insulator element. In some embodiments, the A1 insulator element and the A2 insulator element are in a parallel orientation.

In some embodiments, the A1 insulator element and the A2 insulator element each comprise or consist of a core insulator sequence. In some embodiments, the A1 insulator element and the A2 insulator element each consist of a core insulator sequence. In some embodiments, the A1 insulator element comprises or consists of the nucleotide sequence of SEQ ID NO: 33, or a nucleotide sequence having at least 80.0%, at least 82.5%, at least 85.0%, at least 87.5%, at least 90.0%, at least 92.5%, at least 95.0%, or at least 97.5% sequence identity thereto; and/or the A2 insulator element comprises or consists of the nucleotide sequence of SEQ ID NO: 34, or a nucleotide sequence having at least 80.0%, at least 82.5%, at least 85.0%, at least 87.5%, at least 90.0%, at least 92.5%, at least 95.0%, or at least 97.5% sequence identity thereto. Suitably, the variants comprise CTCF-binding motif A (e.g. SEQ ID NO: 23) and one or more variation is made in the flanking sequences. In some embodiments, the A1 insulator element comprises or consists of the nucleotide sequence of SEQ ID NO: 33; and/or the A2 insulator element comprises or consists of the nucleotide sequence of SEQ ID NO: 34.

In some embodiments, the A1 insulator element and the A2 insulator element each comprise or consist of a full length insulator sequence. In some embodiments, the A1 insulator element comprises or consists of the nucleotide sequence of SEQ ID NO: 64, or a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto; and/or the A2 insulator element comprises or consists of the nucleotide sequence of SEQ ID NO: 103, or a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise CTCF-binding motif A (e.g. SEQ ID NO: 23) and one or more variation is made in the flanking sequences. In some embodiments, the A1 insulator element comprises or consists of the nucleotide sequence of SEQ ID NO: 64; and/or the A2 insulator element comprises or consists of the nucleotide sequence of SEQ ID NO: 103.

### A2-E2 insulator pairs

In some embodiments, the polynucleotide comprises an A2-E2 insulator element pair flanking the expression cassette. In some embodiments, the first insulator element is an A2 insulator element and the second insulator element is an E2 insulator element. In some embodiments, the A2 insulator element and the E2 insulator element are in a divergent orientation.

In some embodiments, the A2 insulator element and the E2 insulator element each comprise or consist of a core insulator sequence. In some embodiments, the A2 insulator element and the E2 insulator element each consist of a core insulator sequence. In some embodiments, the A2 insulator element comprises or consists of the nucleotide sequence of SEQ ID NO: 34, or a nucleotide sequence having at least 80.0%, at least 82.5%, at least 85.0%, at least 87.5%, at least 90.0%, at least 92.5%, at least 95.0%, or at least 97.5% sequence identity thereto; and/or the E2 insulator element comprises or consists of the nucleotide sequence of SEQ ID NO: 32, or a nucleotide sequence having at least 80.0%, at least 82.5%, at least 85.0%, at least 87.5%, at least 90.0%, at least 92.5%, at least 95.0%, or at least 97.5% sequence identity thereto. Suitably, the A2 insulator element variants comprise CTCF-binding motif A (e.g. SEQ ID NO: 23) and one or more variation is made in the flanking sequences. Suitably, the E2 insulator element variants comprise CTCF-binding motif E (e.g. SEQ ID NO: 27) and one or more variation is made in the flanking sequences. In some embodiments, the A2 insulator element comprises or consists of the nucleotide sequence of SEQ ID NO: 34; and/or the E2 insulator element comprises or consists of the nucleotide sequence of SEQ ID NO: 32.

In some embodiments, the A2 insulator element and the E2 insulator element each comprise or consist of a full length insulator sequence. In some embodiments, the A2 insulator element comprises or consists of the nucleotide sequence of SEQ ID NO: 103, or a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto; and/or the E2 insulator element comprises or consists of the nucleotide sequence of SEQ ID NO: 55, or a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the A2 insulator element variants comprise CTCF-binding motif A (e.g. SEQ ID NO: 23) and one or more variation is made in the flanking sequences. Suitably, the E2 insulator element variants comprise CTCF-binding motif E (e.g. SEQ ID NO: 27) and one or more variation is made in the flanking sequences. In some embodiments, the A2 insulator element comprises or consists of the nucleotide sequence of SEQ ID NO: 103; and/or the E2 insulator element comprises or consists of the nucleotide sequence of SEQ ID NO: 55.

### Multiple insulator elements

The polynucleotide may comprise any number of insulator elements in any arrangement provided that at least one of the insulator elements functions to prevent activation of a promoter by nearby heterologous enhancers and/or functions as a barrier against repressive effects of neighbouring heterochromatin.

In some embodiments, the polynucleotide comprises one or more insulator elements. In some embodiments, the polynucleotide comprises one insulator element. A single insulator element may form a pair of insulator elements with an element from another polynucleotide, for example following integration of the polynucleotide in a genomic context. For example, the insulator elements may be upstream from the expression cassette or downstream from the expression cassette. Alternatively, if the polypeptide is a retrovirus genome and the insulator element is in the 3' LTR of the retrovirus genome, it may be copied to the 5' LTR during reverse transcription, thereby providing a pair of insulator elements. For example, the insulator element may be in a 3' LTR.

In some embodiments, the polynucleotide comprises two or more insulator elements. In some embodiments, the polynucleotide comprises two insulator elements. The two or more insulator elements may form a pair of insulator elements flanking the expression cassette. For example, one of the insulator elements may be upstream from the expression cassette and one of the insulator elements may be downstream from the expression cassette. Alternatively, each of the insulator elements may form a pair of insulator elements with an insulator element from another polynucleotide. For example, both of the insulator elements may be upstream from the expression cassette or both of the insulator elements may be downstream from the expression cassette. Alternatively, if the polynucleotide is a retrovirus genome and at least one of the insulator elements is in the 3' LTR of the retrovirus genome, at least one of the insulator elements may be copied to the 5' LTR during reverse transcription, thereby providing a pair of insulator elements. For example, one of the insulator elements may be downstream of the expression cassette and upstream of a 3' LTR and one of the insulator elements may be in a 3' LTR. For example, both of the insulator elements may be in a 3' LTR (e.g. in parallel or divergent orientation).

In some embodiments, the polynucleotide comprises three or more insulator elements. In some embodiments, the polynucleotide comprises three insulator elements. At least two of the insulator elements may form a pair of insulator elements flanking the expression cassette. For example, at least one of the insulator elements may be upstream from the expression cassette and at least one of the insulator elements may be downstream from the expression cassette. Alternatively, each of the insulator elements may form a pair of insulator elements with an insulator element from another polynucleotide. For example, all of the insulator elements may be upstream from the expression cassette or all of the insulator elements may be downstream from the expression cassette. Alternatively, if the polynucleotide is a retrovirus genome and at least one of the insulator elements is in the 3' LTR of the retrovirus genome, at least one of the insulator elements may be copied to the 5' LTR during reverse transcription, thereby providing a pair of insulator elements. For example, at least one of the insulator elements may be present in downstream of the expression cassette and upstream of a 3' LTR and at least one of the insulator elements may present in a 3' LTR. For example, one of the insulator elements may be present downstream of the expression cassette and upstream of a 3' LTR and two of the insulator elements may present in a 3' LTR (e.g. in parallel or divergent orientation). For example, one of the insulator elements may present in a 5' LTR, one of the insulator elements may be present downstream of the expression cassette and upstream of a 3' LTR, and one of the insulator elements may present in a 3' LTR.

In some embodiments, the polynucleotide comprises four or more insulator elements. In some embodiments, the polynucleotide comprises four insulator elements. For example, two of the insulator elements may present in a 5' LTR and two of the insulator elements may present in a 3' LTR.

The insulator elements may each have the same or different sequences. Suitably, each of the insulator elements binds CTCF. Suitably, each of the insulator elements comprises a CTCF-binding motif.

### Example polynucleotides

In some embodiments, the polynucleotide comprises: (a) one or more miRNA target sequences having the same orientation as the expression cassette, upstream from the expression cassette and/or downstream from the transcription termination site; and (b) one or more miRNA target sequences having the opposite orientation as the expression cassette, upstream from the expression cassette and/or downstream from the expression cassette.

In some embodiments, the polynucleotide comprises: (a) one or more miRNA target sequences having the same orientation as the expression cassette, upstream from the expression cassette and/or downstream from the transcription termination site; and (c) one or more insulator elements, upstream from the expression cassette and/or downstream from the expression cassette.

In some embodiments, the polynucleotide comprises: (b) one or more miRNA target sequences having the opposite orientation as the expression cassette, upstream from the expression cassette and/or downstream from the expression cassette; and (c) one or more insulator elements, upstream from the expression cassette and/or downstream from the expression cassette.

In preferred embodiments, the polynucleotide comprises: (a) one or more miRNA target sequences having the same orientation as the expression cassette, upstream from the expression cassette and/or downstream from the transcription termination site; (b) one or more miRNA target sequences having the opposite orientation as the expression cassette, upstream from the expression cassette and/or downstream from the expression cassette; and (c) one or more insulator elements, upstream from the expression cassette and/or downstream from the expression cassette.

In some embodiments, the polynucleotide comprises: (a) (i) one or more miRNA target sequences having the same orientation as the expression cassette, upstream from the expression cassette; and (ii) optionally, one or more miRNA target sequences having the same orientation as the expression cassette, downstream from the transcription termination site; and (b) (i) one or more miRNA target sequences having the opposite orientation as the expression cassette, upstream from the expression cassette; and (ii) one or more miRNA target sequences having the opposite orientation as the expression cassette, downstream from the expression cassette.

In some embodiments, the polynucleotide comprises: (a) (i) one or more miRNA target sequences having the same orientation as the expression cassette, upstream from the expression cassette; and (ii) optionally, one or more miRNA target sequences having the same orientation as the expression cassette, downstream from the transcription termination site; and (c) (i) one or more insulator elements upstream from the expression cassette; and (ii) one or more insulator elements downstream from the expression cassette.

In some embodiments, the polynucleotide comprises: (b) (i) one or more miRNA target sequences having the opposite orientation as the expression cassette, upstream from the expression cassette; and (ii) one or more miRNA target sequences having the opposite orientation as the expression cassette, downstream from the expression cassette; and (c) (i) one or more insulator elements upstream from the expression cassette; and (ii) one or more insulator elements downstream from the expression cassette.

In some embodiments, the polynucleotide comprises: (a) (i) one or more miRNA target sequences having the same orientation as the expression cassette, upstream from the expression cassette; and (ii) optionally, one or more miRNA target sequences having the same orientation as the expression cassette, downstream from the transcription termination site; (b) (i) one or more miRNA target sequences having the opposite orientation as the expression cassette, upstream from the expression cassette; and (ii) one or more miRNA target sequences having the opposite orientation as the expression cassette, downstream from the expression cassette; and (c) (i) one or more insulator elements upstream from the expression cassette; and (ii) one or more insulator elements downstream from the expression cassette.

In some embodiments, the polynucleotide comprises from 5' to 3' direction: one or more miRNA target sequences having the same orientation as the expression cassette and/or one or more miRNA target sequences having the opposite orientation as the expression cassette; one or more insulator elements; an expression cassette; one or more insulator elements; and one or more miRNA target sequences having the opposite orientation as the expression cassette.

In some embodiments, the polynucleotide comprises from 5' to 3' direction: one or more insulator elements; one or more miRNA target sequences having the same orientation as the expression cassette and/or one or more miRNA target sequences having the opposite orientation as the expression cassette; an expression cassette; one or more miRNA target sequences having the same orientation as the expression cassette; and one or more insulator elements.

In some embodiments, the polynucleotide comprises from 5' to 3' direction: one or more insulator elements; one or more miRNA target sequences having the same orientation as the expression cassette and/or one or more miRNA target sequences having the opposite orientation as the expression cassette; an expression cassette; one or more insulator elements; and one or more miRNA target sequences having the opposite orientation as the expression cassette.

In some embodiments, the polynucleotide comprises from 5' to 3' direction: one or more miRNA target sequences having the same orientation as the expression cassette and/or one or more miRNA target sequences having the opposite orientation as the expression cassette; one or more insulator elements; an expression cassette; one or more miRNA target sequences having the opposite orientation as the expression cassette; and one or more insulator elements.

The one or more insulator elements and one or more miRNA target sequences having the opposite orientation as the expression cassette that are downstream from the expression cassette may be upstream and/or downstream from the transcription termination site.

For example, the polynucleotide may comprise: (i) one or more insulator elements and one or more miRNA target sequences having the opposite orientation as the expression cassette downstream from the expression cassette and upstream from the transcription termination site; and/or (ii) one or more insulator elements and one or more miRNA target sequences having the opposite orientation as the expression cassette downstream from the transcription termination site.

In some embodiments, the polynucleotide further comprises one or more miRNA target sequences having the same orientation as the expression cassette downstream from the transcription termination site.

### LTRs

In some embodiments, the polynucleotide comprises one or more long terminal repeat (LTR). In some embodiments, the polynucleotide comprises a 5' LTR and a 3' LTR. In some embodiments, the polynucleotide comprises from 5' to 3' direction: a 5' LTR, an expression cassette, and a 3' LTR.

A "long terminal repeat" may refer to a nucleotide sequence that forms a retrotransposon, an endogenous retrovirus, or a retroviral provirus. All retroviral genomes are flanked by LTRs, while there are some retrotransposons without LTRs.

A lentivirus genome is typically flanked at both ends by LTRs. The LTRs are responsible for integration and transcription. LTRs may also serve as enhancer-promoter sequences and may contain signals for transcription termination and polyadenylation. The LTRs themselves are identical or near-identical sequences that can typically be divided into three regions: U3, R and U5. LTRs may be naturally-occurring or may be modified. For example, U3 and U5 modifications are described in Iwakuma, T., et al., 1999. Virology, 261(1), pp.120-132. In some embodiments, the 3' LTR and the 5' LTR are identical.

Any LTR sequences may be used, the selection of which may be readily made by the skilled person. Suitable LTR sequences will be well known to those of skill in the art (see e.g. Frech, K., et al., 1996. Virology, 224(1), pp.256-267).

An example LTR sequence is provided as SEQ ID NO: 91. In some embodiments, a LTR comprises or consists of a nucleotide sequence which is at least 70% identical to SEQ ID NO: 91 or a fragment thereof. Suitably, a LTR comprises or consists of a nucleotide sequence which is at least 75%, at least 80%, at least 85%, at least 90%, at least 95%, at least 96%, at least 97%, at least 98%, or at least 99% identical to SEQ ID NO: 91 or a fragment thereof. In some embodiments, a LTR comprises or consists of the nucleotide sequence SEQ ID NO: 91 or a fragment thereof.

TGGAAGGGCTAATTCACTCCCAACGAAGACAAGATCTGCTTTTTGCTTGTACTGGGTCTCTC
TGGTTAGACCAGATCTGAGCCTGGGAGCTCTCTGGCTAACTAGGGAACCCACTGCTTAAGCC
TCAATAAAGCTTGCCTTGAGTGCTTCAAGTAGTGTGTGCCCGTCTGTTGTGTGACTCTGGTA
ACTAGAGATCCCTCAGACCCTTTTAGTCAGTGTGGAAAATCTCTAGCAG

Example LTR (SEQ ID NO: 91)

In some embodiments, the polynucleotide comprises one or more self-inactivating long terminal repeat (SIN-LTR). A "SIN-LTR" may comprise a deletion that abolishes transcription of the full-length virus after it has incorporated into a host cell. For example, a 3' SIN-LTR may comprise a deletion in the U3 region removing the promoter/enhancer elements (see e.g. Zufferey, R., et al., 1998. Journal of virology, 72(12), pp.9873-9880). This deletion is copied into the 5' LTR after reverse transcription, thereby making the gene expression in target cells dependent on an internal promoter of choice.

Any SIN-LTR sequences may be used, the selection of which may be readily made by the skilled person. Suitable SIN-LTR sequences will be well known to those of skill in the art (see e.g. Zufferey, R., et al., 1998. Journal of virology, 72(12), pp.9873-9880 and Miyoshi, H., et al., 1998. Journal of virology, 72(10), pp.8150-8157).

### 3'LTR

In some embodiments, the polynucleotide comprises a 3' LTR. In some embodiments, the polynucleotide comprises from 5' to 3' direction: an expression cassette, and a 3' LTR.

The expression cassette and the 3' LTR may overlap (see e.g. Guntaka, R.V., 1993. Microbiological reviews, 57(3), pp.511-521). The 3' LTR may comprise part of the 3' regulatory region, e.g. comprising regulatory sequences, such as a polyadenylations sequence. The 3' LTR may comprise a transcription termination site.

In some embodiments, it is preferred for the one or more miRNA target sequences and/or the one or more insulator elements to be present in the 3' LTR, because the 3' LTR may be copied to the 5' LTR during reverse transcription, e.g. thereby providing multiple copies of the one or more miRNA target sequences and/or a pair of insulator element flanking the expression cassette.

In some embodiments, the 3' LTR is a SIN-LTR. In some embodiments, the 3' LTR is any described in the section below entitled "SIN 3' LTRs".

In some embodiments, the 3' LTR comprises one or more miRNA target sequences having the same orientation as the expression cassette. In some embodiments, the 3' LTR does not comprise a miRNA target sequence having the same orientation as the expression cassette. This is preferred when the expression cassette is sense-oriented.

In some embodiments, the 3' LTR comprises one or more miRNA target sequences having the opposite orientation as the expression cassette. In some embodiments, the 3' LTR does not comprise a miRNA target sequence having the opposite orientation as the expression cassette.

In some embodiments, the 3' LTR comprises one or more miRNA target sequences having the same orientation as the expression cassette and one or more miRNA target sequences having the opposite orientation as the expression cassette.

In some embodiments, the 3' LTR comprises one or more insulator elements. In some embodiments, the 3' LTR does not comprise an insulator element.

In some embodiments, the 3' LTR comprises one or more miRNA target sequences and one or more insulator elements.

In some embodiments, the 3' LTR comprises one or more miRNA target sequences having the same orientation as the expression cassette and one or more insulator elements. In some embodiments, the 3' LTR does not comprise one or more miRNA target sequences having the opposite orientation as the expression cassette.

In some embodiments, the 3' LTR comprises one or more miRNA target sequences having the opposite orientation as the expression cassette and one or more insulator elements. In some embodiments, the 3' LTR does not comprise one or more miRNA target sequences having the same orientation as the expression cassette.

In some embodiments, the 3' LTR comprises one or more miRNA target sequences having the same orientation as the expression cassette, one or more miRNA target sequences having the opposite orientation as the expression cassette, and one or more insulator elements.

### 5'LTR

In some embodiments, the polynucleotide comprises a 5' LTR. In some embodiments, the polynucleotide comprises from 5' to 3' direction: a 5' LTR, and an expression cassette.

In some embodiments, the one or more miRNA target sequences and/or the one or more insulator elements are not present in the 5' LTR, since a 3' LTR comprising said sequences may be copied to the 5' LTR during reverse transcription.

In some embodiments, the 5' LTR is a SIN-LTR. An example 5'LTR sequence is provided as SEQ ID NO: 92. In some embodiments, a 5'LTR comprises or consists of a nucleotide sequence which is at least 70% identical to SEQ ID NO: 92 or a fragment thereof. Suitably, a 5'LTR comprises or consists of a nucleotide sequence which is at least 75%, at least 80%, at least 85%, at least 90%, at least 95%, at least 96%, at least 97%, at least 98%, or at least 99% identical to SEQ ID NO: 92 or a fragment thereof. In some embodiments, a 5'LTR comprises or consists of the nucleotide sequence SEQ ID NO: 92 or a fragment thereof.

GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAG

Example 5'LTR (SEQ ID NO: 92)

In some embodiments, the 5 LTR comprises one or more miRNA target sequences having the same orientation as the expression cassette. In some embodiments, the 5' LTR does not comprise a miRNA target sequence having the same orientation as the expression cassette.

In some embodiments, the 5' LTR comprises one or more miRNA target sequences having the opposite orientation as the expression cassette. In some embodiments, the 5' LTR does not comprise a miRNA target sequence having the opposite orientation as the expression cassette.

In some embodiments, the 5' LTR comprises one or more miRNA target sequences having the same orientation as the expression cassette and one or more miRNA target sequences having the opposite orientation as the expression cassette.

In some embodiments, the 5' LTR comprises one or more insulator elements. In some embodiments, the 5' LTR does not comprise an insulator element.

In some embodiments, the 5' LTR comprises one or more miRNA target sequences and one or more insulator elements.

In some embodiments, the 5' LTR comprises one or more miRNA target sequences having the same orientation as the expression cassette and one or more insulator elements. In some embodiments, the 5' LTR does not comprise a miRNA target sequence having the opposite orientation as the expression cassette.

In some embodiments, the 5' LTR comprises one or more miRNA target sequences having the opposite orientation as the expression cassette and one or more insulator elements. In some embodiments, the 5' LTR does not comprise a miRNA target sequence having the same orientation as the expression cassette.

In some embodiments, the 5' LTR comprises one or more miRNA target sequences having the same orientation as the expression cassette, one or more miRNA target sequences having the opposite orientation as the expression cassette, and one or more insulator elements.

### Vectors

In one aspect, the present invention provides a vector comprising the polynucleotide of the invention.

A "vector" is a tool that allows or facilitates the transfer of an entity from one environment to another. In accordance with the invention, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (DNA or RNA) within the cell, facilitating the replication of the vector comprising a segment of nucleic acid, or facilitating the expression of the protein encoded by a segment of nucleic acid.

Examples of vectors used in recombinant nucleic acid techniques include, but are not limited to, plasmids, chromosomes, artificial chromosomes, transposons and viruses. The vector may be single stranded or double stranded. The vector may comprise or consist of DNA and/or RNA. In some embodiments, the vector is a DNA vector. In some embodiments, the vector is a RNA vector. The vector may be linear or circular. The vector may also be, for example, a naked nucleic acid (e.g. DNA). In its simplest form, the vector may itself be a nucleotide of interest.

The term "vector" includes an expression vector i.e. a construct capable of *in vivo, in vitro,* or ex *vivo* expression. Expression may be controlled by a vector sequence, or, for example in the case of insertion at a target site, expression may be controlled by a target sequence. A vector may be integrated or tethered to the cell's DNA.

In some embodiments, the vector is a plasmid, a transposon or a viral vector. In some embodiments, the vector is an integrating vector. Integrating vectors, such as lentivirus and gammaretrovirus, fuse a fragment of their genetic material into the host cell genome. In some embodiments, the vector is an integrating viral vector.

### Viral vectors

In some embodiments, the vector is a viral vector. The vector of the present invention may be in the form of a viral vector particle.

Viral vectors include but are not limited to a lentiviral vector, a retroviral vector, an adenovirus vector, an adeno-associated viral (AAV) vector, a herpes simplex viral vector, a baculoviral vector, an alphaviral vector, a flaviviral vector, a rhabdoviral vector, a measles viral vector, a Newcastle disease viral vector, a poxviral vector, and a picornaviral vector (see e.g. Lundstrom, K., 2018. Diseases, 6(2), p.42).

Methods of preparing and modifying viral vectors and viral vector particles, such as those derived from AAV, are well known in the art. Suitable methods are described in Ayuso, E., et al., 2010. Current gene therapy, 10(6), pp.423-436, Merten, O.W., et al., 2016. Molecular Therapy-Methods & Clinical Development, 3, p.16017; and Nadeau, I. and Kamen, A., 2003. Biotechnology advances, 20(7-8), pp.475-489.

In some embodiments, the viral vector has a minimal viral genome. By "minimal viral genome" it is to be understood that the viral vector has been manipulated so as to remove the non-essential elements and to retain the essential elements in order to provide the required functionality to infect, transduce and deliver a nucleotide sequence of interest to a target host cell.

The viral vector genome may have a defined orientation. For example, a retroviral genome may be oriented from the 5' LTR to the 3' LTR. In the context of a viral vector, a "sense-oriented" expression cassette may have the same orientation as the viral vector genome. For example, following conversion of a lentiviral vector into double-stranded DNA, the expression cassette may be transcribed in the direction of from the 5' LTR to the 3' LTR. In the context of a viral vector, an "antisense-oriented" expression cassette may have the opposite orientation to the viral vector genome. For example, following conversion of a lentiviral vector into double-stranded DNA, the expression cassette may be transcribed in the direction of from the 3' LTR to the 5' LTR.

### Retroviral vectors

In some embodiments, the vector is a retroviral vector. In some embodiments, the vector is a retroviral vector particle.

Retroviruses are RNA viruses with a life cycle different to that of lytic viruses. In this regard, a retrovirus is an infectious entity that replicates through a DNA intermediate. When a retrovirus infects a cell, its genome is converted to a DNA form by a reverse transcriptase enzyme. The DNA copy serves as a template for the production of new RNA genomes and virally encoded proteins necessary for the assembly of infectious viral particles.

All retroviruses are characterized by the ability to synthesize, starting from the genomic RNA template, a double-stranded proviral DNA which stably integrates into the host genome. The genomic RNA molecules display at both ends two identical sequences called Repeats, ordinarily known as R regions, while unique U5 and U3 sequences are present specifically at the 5' and 3' end, correspondingly. During reverse-transcription of the RNA genome in double-stranded proviral DNA, the U5 and U3 sequences are duplicated at both ends of the vector sequence generating the Long Terminal Repeats (LTRs).

A retroviral vector may be derived from or may be derivable from any suitable retrovirus. A large number of different retroviruses have been identified. Examples include human immunodeficiency virus (HIV), murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV).

In some embodiments, the vector is a gammaretrovirus. In some embodiments, the vector is a gammaretrovirus vector particle. Gammaretrovirus is a genus in the Retroviridae family. Example species are Moloney murine leukemia virus (Mo-MLV), xenotropic MuLB-related virus, feline leukemia virus (FLV), and feline sarcoma virus (FSV). The double stranded DNA is highly stable and easily integrated into a host genome (see e.g. Maetzig, T., et al., 2011. Viruses, 3(6), pp.677-713).

In some embodiments, the vector is a lentiviral vector. In some embodiments, the vector is a lentiviral vector particle. Lentiviruses belong to the retrovirus family, but they can infect both dividing and non-dividing cells. The lentiviral vector may be any described below in the section entitled "Retroviral Vectors".

In some embodiments, the vector is an alpharetroviral vector. The most widely used alpharetroviral vector system is the RCAS (replication-competent avian leukosis virus LTR with a splice acceptor) system, which is based on Rous sarcoma virus (RSV) (see e.g. Suerth, J.D., et al., A., 2014. Viruses, 6(12), pp.4811-4838).

The basic structure of retrovirus genomes share many common features such as a 5' LTR and a 3' LTR. Between or within these are located a packaging signal to enable the genome to be packaged, a primer binding site, integration sites to enable integration into a host cell genome, and gag, pol and env genes encoding the packaging components. In a defective retroviral vector genome gag, pol and env may be absent or not functional.

In a typical retroviral vector, at least part of one or more protein coding regions essential for replication may be removed from the virus. This makes the viral vector replication-defective. Portions of the viral genome may also be replaced by a library encoding candidate modulating moieties operably linked to a regulatory control region and a reporter moiety in the vector genome in order to generate a vector comprising candidate modulating moieties which is capable of transducing a target host cell and/or integrating its genome into a host genome.

### Adeno-associated viral (AA V) vectors

In some embodiments, the vector is an AAV vector. In some embodiments, the vector is an AAV vector particle.

AAVs occurring in nature may be classified according to various biological systems. The AAV may any naturally derived serotype, isolate or clade of AAV.

AAV may be referred to in terms of their serotype. A serotype corresponds to a variant subspecies of AAV which, owing to its profile of expression of capsid surface antigens, has a distinctive reactivity which can be used to distinguish it from other variant subspecies. Typically, an AAV vector particle having a particular AAV serotype does not efficiently cross-react with neutralising antibodies specific for any other AAV serotype. AAV serotypes include AAV1, AAV2, AAV3, AAV4, AAV5, AAV6, AAV7, AAV8, AAV9, AAV10 and AAV11, and derivatives thereof.

Clades or clones refers to the phylogenetic relationship of naturally derived AAVs, and typically to a phylogenetic group of AAVs which can be traced back to a common ancestor, and includes all descendants thereof. Additionally, AAVs may be referred to in terms of a specific isolate, i.e. a genetic isolate of a specific AAV found in nature. The term genetic isolate describes a population of AAVs which has undergone limited genetic mixing with other naturally occurring AAVs, thereby defining a recognisably distinct population at a genetic level.

Typically, the AAV genome of a naturally derived serotype, isolate or clade of AAV comprises at least one inverted terminal repeat sequence (ITR). ITRs may be the only sequences required in cis next to the therapeutic gene.

The AAV genome may also comprise packaging genes, such as rep and/or cap genes which encode packaging functions for an AAV particle. A promoter may be operably linked to each of the packaging genes. Specific examples of such promoters include the p5, p19 and p40 promoters. For example, the p5 and p19 promoters are generally used to express the rep gene, while the p40 promoter is generally used to express the cap gene. The rep gene encodes one or more of the proteins Rep78, Rep68, Rep52 and Rep40 or variants thereof. The cap gene encodes one or more capsid proteins such as VP1, VP2 and VP3 or variants thereof. These proteins make up the capsid of an AAV particle, which determines the AAV serotype. VP1, VP2, and VP3 may be produced by alternate mRNA splicing (Trempe, J.P. and Carter, B.J., 1988. Journal of virology, 62(9), pp.3356-3363). Thus, VP1, VP2 and VP3 may have identical sequences, but wherein VP2 is truncated at the N-terminus relative to VP1, and VP3 is truncated at the N-terminus relative to VP2.

Preferably, the AAV genome is derivatised for the purpose of administration to patients. Such derivatisation is standard in the art and the invention encompasses the use of any known derivative of an AAV genome, and derivatives which could be generated by applying techniques known in the art. The AAV genome may be a derivative of any naturally occurring AAV. Suitably, the AAV genome is a derivative of AAV1, AAV2, AAV3, AAV4, AAV5, AAV6, AAV7, AAV8, AAV9, AAV10, or AAV11.

Derivatives of an AAV genome include any truncated or modified forms of an AAV genome which allow for expression of a transgene from an AAV vector of the invention *in vivo.* Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR. A preferred mutant ITR is one having a deletion of a trs (terminal resolution site). This deletion allows for continued replication of the genome to generate a single-stranded genome which contains both coding and complementary sequences, i.e. a self-complementary AAV (scAAV) genome. This allows for bypass of DNA replication in the target cell, and so enables accelerated transgene expression.

The AAV genome may comprise one or more ITR sequences from any naturally derived serotype, isolate or clade of AAV or a variant thereof. The AAV genome may comprise at least one, such as two, AAV1, AAV2, AAV3, AAV4, AAV5, AAV6, AAV7, AAV8, AAV9, AAV10, or AAV1 1 ITRs, or variants thereof.

The inclusion of one or more ITRs is preferred to aid concatamer formation of the AAV vector in the nucleus of a host cell, for example following the conversion of single-stranded vector DNA into double-stranded DNA by the action of host cell DNA polymerases. The formation of such episomal concatamers protects the AAV vector during the life of the host cell, thereby allowing for prolonged expression of the transgene in vivo.

Suitably, ITR elements will be the only sequences retained from the native AAV genome in the derivative. A derivative will preferably not include the rep and/or cap genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the protein-coding sequence.

The AAV vector particle may be encapsidated by capsid proteins. Suitably, the AAV vector particles may be transcapsidated forms wherein an AAV genome or derivative having an ITR of one serotype is packaged in the capsid of a different serotype. The AAV vector particle also includes mosaic forms wherein a mixture of unmodified capsid proteins from two or more different serotypes makes up the viral capsid. The AAV vector particle also includes chemically modified forms bearing ligands adsorbed to the capsid surface. For example, such ligands may include antibodies for targeting a particular cell surface receptor.

Where a derivative comprises capsid proteins i.e. VP1, VP2 and/or VP3, the derivative may be a chimeric, shuffled or capsid-modified derivative of one or more naturally occurring AAVs. In particular, capsid protein sequences from different serotypes, clades, clones, or isolates of AAV may be used within the same vector (i.e. a pseudotyped vector). The AAV vector may be in the form of a pseudotyped AAV vector particle.

### Adenoviral vectors

In some embodiments, the vector is an adenoviral vector. In some embodiments, the vector is an adenoviral vector particle.

The adenovirus is a double-stranded, linear DNA virus that does not go through an RNA intermediate. There are over 50 different human serotypes of adenovirus divided into 6 subgroups based on the genetic sequence homology. The natural targets of adenovirus are the respiratory and gastrointestinal epithelia, generally giving rise to only mild symptoms. Serotypes 2 and 5 (with 95% sequence homology) are most commonly used in adenoviral vector systems and are normally associated with upper respiratory tract infections in the young.

Adenoviruses have been used as vectors for gene therapy and for expression of heterologous genes. The large (36 kb) genome can accommodate up to 8 kb of foreign insert DNA and is able to replicate efficiently in complementing cell lines to produce very high titres of up to 10¹². Adenovirus is thus one of the best systems to study the expression of genes in primary non-replicative cells.

The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, they function episomally (independently from the host genome) as a linear genome in the host nucleus. Hence, the use of recombinant adenovirus alleviates the problems associated with random integration into the host genome.

### Other viral vectors

Other viral vectors include those described in Lundstrom, K., 2018. Diseases, 6(2), p.42.

In some embodiments, the vector is a herpes simplex vector. In some embodiments, the vector is a herpes simplex vector particle.

Herpes simplex virus (HSV) is a neurotropic DNA virus with favorable properties as a gene delivery vector. HSV is highly infectious, so HSV vectors are efficient vehicles for the delivery of exogenous genetic material to cells. Viral replication is readily disrupted by null mutations in immediate early genes that in vitro can be complemented in trans enabling straightforward production of high-titre pure preparations of non-pathogenic vector. The genome is large (152 Kb) and many of the viral genes are dispensable for replication in vitro, allowing their replacement with large or multiple transgenes. Latent infection with wild-type virus results in episomal viral persistence in sensory neuronal nuclei for the duration of the host lifetime. The vectors are non-pathogenic, unable to reactivate and persist long-term. The latency active promoter complex can be exploited in vector design to achieve long-term stable transgene expression in the nervous system. HSV vectors transduce a broad range of tissues because of the wide expression pattern of the cellular receptors recognized by the virus. Increasing understanding of the processes involved in cellular entry has allowed targeting the tropism of HSV vectors.

In some embodiments, the vector is baculoviral vector. In some embodiments, the vector is a baculoviral vector particle.

Baculoviruses have been extensively studied as potential vectors for both in vitro and in vivo gene therapy. Autographa californica multiple nucleopolyhedrovirus (AcMNPV) is one of the most well-studied baculoviruses. Given the highly efficient gene delivery into many cell types, baculoviruses have captured increasing interest as vectors for *in vivo* gene delivery. In comparison with other viral vectors, baculoviruses possess a number of advantages, including a large cloning capacity (see e.g. Hu, Y.C., 2006. Advances in virus research, 68, pp.287-320).

In some embodiments, the vector is an alphaviral vector. In some embodiments, the vector is an alphaviral vector particle. In some embodiments, the vector is a flaviviral vector. In some embodiments, the vector is a flaviviral vector particle.

Self-amplifying ssRNA viruses comprise of alphaviruses (e.g. Semliki Forest virus, Sindbis virus, Venezuelan equine encephalitis virus, and M1) and flaviviruses (e.g. Kunjin virus, West Nile virus, and Dengue virus) possessing a genome of positive polarity. Alphaviruses have been mainly applied in preclinical gene therapy studies for cancer treatment. Alphavirus vectors can be delivered in the form of naked RNA, layered plasmid DNA vectors and recombinant replication-deficient or -proficient particles.

In some embodiments, the vector is a rhabdoviral vector. In some embodiments, the vector is a rhabdoviral vector particle. In some embodiments, the vector is a measles viral vector. In some embodiments, the vector is a measles viral vector particle.

Rhabdoviruses (e.g. rabies and vesicular stomatitis virus) and measles viruses carry negative strand genomes. Among rhabdoviruses, recombinant vesicular stomatitis virus (VSV) has been applied for preclinical gene therapy studies. Measles viruses (e.g. MV-Edm) have found a number of gene therapy applications.

In some embodiments, the vector is a Newcastle disease viral vector. In some embodiments, the vector is a Newcastle disease viral vector particle.

The ssRNA paramyxovirus Newcastle disease virus (NDV) replicates specifically in tumour cells and has therefore been frequently applied for cancer gene therapy.

In some embodiments, the vector is a poxviral vector. In some embodiments, the vector is a poxviral vector particle.

The characteristic feature of poxviruses is their dsDNA genome, which can generously accommodate more than 30 kb of foreign DNA. Poxviruses have found several applications as gene therapy vectors. For instance, vaccinia virus vectors have demonstrated potential for treatment of cancer. Vaccinia virus is large enveloped poxvirus that has an approximately 190 kb linear, double-stranded DNA genome. Vaccinia virus can accommodate up to approximately 25 kb of foreign DNA, which also makes it useful for the delivery of large genes. A number of attenuated vaccinia virus strains are known in the art that are suitable for gene therapy applications, for example the MVA and NYVAC strains.

In some embodiments, the vector is a picornaviral vector. In some embodiments, the vector is a picornaviral vector particle.

Picornaviruses are non-enveloped ssRNA viruses. Coxsackieviruses belonging to Picornaviridae, have been applied as oncolytic vectors.

### Transposons

In some embodiments, the vector is a transposon vector. In some embodiments, a transposon system is provided comprising a transposon vector and a vector encoding a transposase.

Transposon vectors are integrating non-viral vectors based on mobile DNA elements that can stably integrate a therapeutic gene in the target cell genome (see e.g. Tipanee, J., et al., 2017. Human gene therapy, 28(11), pp.1087-1104). A vector comprising transposable elements and a vector comprising the corresponding transposase may be provided separately to provide a transposition system.

DNA transposons translocate via a "non-replicative mechanism," where two Terminal Inverted Repeats (TIRs) are recognized and cleaved by a transposase enzyme, releasing the cognate DNA transposons with free DNA ends. The excised DNA transposons can then integrate into a new genomic region where target sites are recognized and cut by the same transposase. This cut-and-paste mechanism usually duplicates DNA target sites upon insertion, leaving target site duplications (TSDs) (see e.g. Tipanee, J., et al., 2017. Human gene therapy, 28(11), pp.1087-1104).

DNA transposons can be classified into diverse families, which differ in terms of target site recognition, TSDs, TIRs, and transposon DNA sequences. Some of these DNA transposon subgroups are employed for human gene therapy to achieve sustained gene expression of the therapeutic transgene (see e.g. Di Matteo, M., et al., 2012. Expert opinion on biological therapy, 12(7), pp.841-858). The most commonly used transposon systems in gene therapy applications are the Sleeping Beauty (SB) and PiggyBac transposon systems.

In some embodiments, the vector is a Sleeping Beauty transposon vector. Sleeping Beauty (SB) is a member of the Tc1/mariner superfamily of transposable elements. A SB transposon vector typically comprises two inverted repeat/direct repeats (IR/DRs) flanking an expression cassette (see e.g. Izsvák, Z., et al., 2000. Journal of molecular biology, 302(1), pp.93-102). A SB transposon system may further comprise a vector encoding a SB transposase, for example a SB3, SB10, SB11, or SB100X transposase.

In some embodiments, the vector is a PiggyBac (PB) transposon vector. A PB transposon vector typically comprises an expression cassette flanked by a 311 bp 5' end and 235 bp 3' end, each containing TIR sequences. A PB transposon system may further comprise vector encoding PB transposase, for example a mPB or hyPB transposase. The PB transposase recognizes the two TIRs of the PB transposon and excises the DNA, leaving the free 3'-OH group, which forms the hairpin structure with 5'-TTAA overhangs of the transposon. The hairpin transposome complex is then released from the genome and binds to the site of integration in which the TTAA consensus sequence is present. PB elements have been used to transform the germline of more than a dozen species (see e.g. Ding, S., et al., 2005. Cell, 122(3), pp.473-483).

In some embodiments, the vector is a *Tol2* transposon vector. The *Tol2* transposable element belongs to the hAT (*hobo*/*Ac*/*Tam3*) superfamily and is composed of a gene encoding the transposase flanked by 12 bp TIRs (see e.g. Kwan, K.M., et al., 2007. Developmental dynamics, 236(11), pp.3088-3099). A *Tol2* transposon vector typically comprises two TIRs flanking an expression cassette. A *Tol2* transposon system may further comprise a vector encoding a *Tol2* transposase.

### Retroviral vectors

In one aspect, the present invention provides a retroviral vector comprising an expression cassette and a transcription termination site, wherein the polynucleotide further comprises:
(a) one or more miRNA target sequences having the same orientation as the expression cassette, optionally upstream from the expression cassette and/or downstream from the transcription termination site, preferably upstream from the expression cassette;
(b) one or more miRNA target sequences having the opposite orientation as the expression cassette, optionally upstream from the expression cassette and/or downstream from the expression cassette; and/or
(c) one or more insulator elements, optionally upstream from the expression cassette and/or downstream from the expression cassette.

A "retroviral vector" may comprise a retroviral genome, optionally wherein the retroviral genome is enveloped. As used herein, a "retroviral genome" may refer to a genome that comprises at least one element derived or derivable from a retrovirus genome. Suitably, a retroviral genome comprises at least one element that is involved in the mechanisms by which a retrovirus infects cells, expresses genes, and/or is replicated.

The retroviral vector may be a retroviral particle. A "retroviral particle" may refer to an enveloped retroviral genome. Retroviral particles may be generated by co-transfection of a plasmid containing a retroviral genome (e.g. a "transfer vector") with helper plasmids (e.g. "packaging vectors" and "envelope vectors") into host cells and harvesting of the retrovirus - containing supernatant afterwards.

In preferred embodiments, the retroviral vector is a lentiviral vector. A "lentiviral vector" may comprise a lentiviral genome, optionally wherein the lentiviral genome is enveloped. As used herein, a "lentiviral genome" may refer to a genome that comprises at least one element derived or derivable from a lentivirus genome. Suitably, a lentiviral genome comprises at least one element that is involved in the mechanisms by which a lentivirus infects cells, expresses genes, and/or is replicated.

Lentivirus is a genus of retroviruses, which contain an RNA genome that is converted to DNA in the transduced cell by a virally encoded reverse transcriptase. Lentiviral vectors can transduce a wide range of cell types and integrate into the host genome in both dividing and post-mitotic cells, resulting in long-term expression of the protein-coding sequence both *in vitro* and *in vivo* (see e.g. Tiscornia, G., et al., 2006. Nature protocols, 1(1), pp.241-245).

The basic genes required for lentivirus survival and function are the *gag, pol,* and *env* genes: gag encodes structural proteins; *pol* encodes enzymes required for reverse transcription and integration into the host cell genome; and *env* encodes the viral envelope glycoprotein (see e.g. Milone, M.C. and O'Doherty, U., 2018. Leukemia, 32(7), pp.1529-1541).

Lentiviruses may also have additional cis-acting elements, such as a rev response element (RRE), which enables the efficient export of RNA transcripts of the integrated provirus from the nucleus to the cytoplasm of an infected target cell; a retroviral psi packaging element, which is involved in regulating the essential process of packaging the retroviral RNA genome into the viral capsid during replication; a primer binding site (PBS), where reverse transcription is initiated; the TAT activation region (TAR); splice donor and acceptor sites; and central and terminal polypurine tracts, which allow initiation of plus-strand synthesis. In a lentivirus genome, these elements are typically flanked at both ends by long terminal repeats (LTRs). The LTRs are responsible for integration and transcription.

A lentiviral vector may comprise a minimal lentiviral genome. As used herein, a "minimal lentiviral genome" may mean that the lentiviral genome has been manipulated so as to remove the non-essential elements and to retain the essential elements in order to provide the required functionality to infect, transduce and deliver a nucleotide sequence of interest to a target host cell (see e.g. Kim, V.N., et al., 1998. Journal of virology, 72(1), pp.811-816 and Sertkaya, H., et al., 2021. Scientific reports, 11(1), pp.1-15).

A lentiviral genome may comprise from 5' to 3' direction: a 5' LTR, one or more lentiviral-derived cis-acting elements, and a 3' LTR. Suitably, a lentiviral genome may comprise from 5' to 3' direction: a 5' LTR, a RRE, and a 3' LTR. Suitably, a lentiviral genome may comprise from 5' to 3' direction: a 5' LTR, a retroviral psi packaging element, a RRE, and a 3' LTR.

Suitably, a lentiviral genome may comprise from 5' to 3' direction: a 5' LTR, a retroviral psi packaging element, a RRE, a cPPT, and a 3' LTR. Suitably, a lentiviral genome may comprise from 5' to 3' direction: a 5' LTR, a PBS, a retroviral psi packaging element, a RRE, a cPPT, and a 3' LTR. Suitably, a lentiviral genome may comprise from 5' to 3' direction: a 5' LTR, a RRE, an expression cassette, and a 3' LTR. Suitably, a lentiviral genome may comprise from 5' to 3' direction: a 5' LTR, a retroviral psi packaging element, a RRE, an expression cassette, and a 3' LTR. Suitably, a lentiviral genome may comprise from 5' to 3' direction: a 5' LTR, a retroviral psi packaging element, a RRE, a cPPT, an expression cassette, and a 3' LTR. Suitably, lentiviral genome may comprise from 5' to 3' direction: a 5' LTR, a PBS, a retroviral psi packaging element, a RRE, a cPPT, an expression cassette, and a 3' LTR.

In some embodiments, the lentiviral vector is replication-defective. Typically, at least part of one or more protein coding regions essential for replication may be removed from the lentiviral genome. This makes the lentiviral vector "replication-defective" or "replication-incompetent". Suitably, one or more of gag, *pol, rev,* and *env* genes are deleted (at least partially) in a replication-defective lentiviral vector. Suitably, each of the gag, *pol, rev,* and *env* genes are deleted (at least partially) in a replication-defective lentiviral vector. Optionally, the lentiviral vector lacks a functional *gag-pol* and/or *env* gene and/or other genes essential for replication.

A lentiviral vector may be derived from any lentivirus. As used herein "lentivirus-derived" or "lentivirus-based" may mean that the lentiviral genome comprises one or more elements from said lentivirus. For example, the coding regions of viral proteins may be deleted, but one or more *cis-acting* element may be retained from said lentivirus.

A lentiviral vector may be derived from a primate lentivirus. Examples of "primate" lentiviruses include, but are not limited to, human immunodeficiency virus (HIV) and simian immunodeficiency virus (SIV). A lentiviral vector may be derived from a non-primate lentivirus (i.e. derived from a lentivirus which does not primarily infect primates, especially humans). Examples of "non-primate" lentiviruses include, but are not limited to, the prototype "slow virus" visna/maedi virus (VMV), caprine arthritis-encephalitis virus (CAEV), equine infectious anaemia virus (EIAV), feline immunodeficiency virus (FIV), and bovine immunodeficiency virus (BIV).

Suitably, the lentiviral vector is a HIV-derived lentiviral vector. As used herein "HIV-derived" or "HIV-based" may mean that the lentiviral genome comprises one or more element from HIV. For example, the coding regions of HIV viral proteins may be deleted, and one or more HIV *cis-acting* element may retained in the lentiviral genome (see e.g. Johnson, N.M., et al., 2021. Molecular Therapy-Methods & Clinical Development, 21, pp.451-465). A HIV-derived lentiviral genome may comprise from 5' to 3' direction: a 5' LTR, one or more HIV-derived cis-acting elements (e.g. RRE and/or cPPT), and a 3' LTR.

Suitably, the lentiviral vector is a HIV-1-derived lentiviral vector. The prototype lentiviral vector system is based on HIV-1 (see e.g. Merten, O.W., et al., 2016. Molecular Therapy-Methods & Clinical Development, 3, p.16017). It has been shown that sequences that extend into the gag open reading frame may be important for packaging of HIV-1. Therefore, HIV-1 vectors often contain the relevant portion of *gag* in which the translational initiation codon has been mutated. In addition, HIV-1 vectors often also contain a portion of the *env* gene that includes the RRE. Rev binds to RRE, which permits the transport of full-length or singly spliced mRNAs from the nucleus to the cytoplasm. In the absence of *rev* and/or a RRE, full-length HIV-1 RNAs may accumulate in the nucleus. Alternatively, a constitutive transport element from certain simple retroviruses such as Mason-Pfizer monkey virus can be used to relieve the requirement for *rev* and a RRE. A HIV-1-derived lentiviral genome may comprise from 5' to 3' direction: a 5' LTR, one or more HIV-1-derived cis-acting elements (e.g. a PBS, a retroviral psi packaging element, a RRE and/or a cPPT), and a 3' LTR.

The lentiviral vector may be a self-inactivating lentiviral vector. As used herein, "self-inactivating" or "SIN" lentiviral vectors may comprise lentiviral genomes in which the lentiviral enhancer and promoter sequences have been deleted (see e.g. Zufferey, R., et al., 1998. Journal of virology, 72(12), pp.9873-9880 and Miyoshi, H., et al., 1998. Journal of virology, 72(10), pp.8150-8157). SIN lentiviral vectors can be generated and transduce non-dividing cells *in vivo* with an efficacy similar to that of wild-type vectors. The transcriptional inactivation of the long terminal repeat (LTR) in the SIN provirus can prevent mobilisation by replication-competent virus. This can also enable the regulated expression of genes from internal promoters by eliminating any cis-acting effects of the LTR. In preferred embodiments, the 3' LTR is a self-inactivating (SIN) 3' LTR.

In preferred embodiments, the lentiviral vector is integration proficient. As used herein, an "integration proficient" lentiviral vector is capable of integrating into the genome of a host cell. In some embodiments, the lentiviral vector is an integrase-proficient lentiviral vector. The one or more miRNA target sequences and/or the one or more insulator elements may reduce the genotoxicity of an integration proficient lentiviral vector.

In other embodiments, the lentiviral vector is integration defective. Integration defective lentiviral vectors can be produced, for example, either by packaging the lentiviral vector with catalytically inactive integrase (such as an HIV integrase bearing the D64V mutation in the catalytic site) or by modifying or deleting essential *att* sequences from the lentiviral genome LTR, or by a combination of the above (see e.g. Wanisch, K. and Yáñez-Muñoz, R.J., 2009. Molecular Therapy, 17(8), pp.1316-1332). In some embodiments, the lentiviral vector is integrase-defective (i.e. integrase-deficient).

The lentiviral vector may be a lentiviral particle. A "lentiviral particle" may refer to an enveloped lentiviral genome. Lentiviral particles may be generated by co-transfection of a plasmid containing a lentiviral genome (e.g. a "transfer vector") with helper plasmids (e.g. "packaging vectors" encoding *gag-pol* and/or *rev* and "envelope vectors" encoding *env*) into host cells and harvesting of the lentivirus-containing supernatant afterwards.

The lentiviral vector of may be pseudotyped. Pseudotyping lentiviral vectors with naturally occurring or engineered lentiviral envelopes can allow targeted transduction of specific cell types (see e.g. Joglekar, A.V. and Sandoval, S., 2017. Human Gene Therapy Methods, 28(6), pp.291-301). For example, the lentiviral vector may be VSV-G pseudotyped. VSV-G is a trimeric protein that binds phosphatidylserine and low-density lipoprotein receptors on a cell surface to endocytose into the cell.

### Retroviral vector (sense-oriented expression cassette)

In one aspect, the present invention provides a retroviral vector comprising from 5' to 3' direction: a 5' LTR; one or more sense-oriented miRNA target sequences; a sense-oriented expression cassette; and a 3' LTR. In preferred embodiments, the retroviral vector is a lentiviral vector.

It is preferred that the sense-oriented miRNA target sequence is upstream from a sense-oriented expression cassette, to prevent degradation of mRNA transcribed from the expression cassette. As described above, in the context of a retroviral vector, a "sense-oriented" expression cassette may have the same orientation as the retroviral viral vector genome. For example, following conversion of the retroviral vector into double-stranded DNA, the expression cassette may be transcribed in the direction of from the 5' LTR to the 3' LTR.

In some embodiments, the retroviral vector further comprises one or more antisense-oriented miRNA target sequences. In some embodiments, the retroviral vector further comprises one or more antisense-oriented miRNA target sequences upstream from the expression cassette and/or downstream from the expression cassette. In some embodiments, the retroviral vector comprises two or more antisense-oriented miRNA target sequences upstream from the expression cassette and/or downstream from the expression cassette. The antisense-oriented miRNA target sequences may be in any position relative to the expression cassette, since it will not cause degradation of mRNA transcribed from the expression cassette.

In some embodiments, the retroviral vector further comprises one or more insulator elements upstream from the expression cassette and/or downstream from the expression cassette.

In some embodiments, it is preferred for the one or more antisense-oriented miRNA target sequences and/or the one or more insulator elements to be comprised in the 3' LTR, because the 3' LTR may be copied to the 5' LTR during reverse transcription. In some embodiments, the 3' LTR comprises one or more antisense-oriented miRNA target sequences. In some embodiments, the 3' LTR comprises one or more insulator elements. In some embodiments, the 3' LTR comprises one or more antisense-oriented miRNA target sequences and one or more insulator elements.

The miRNA target sequences and the one or more insulator elements may be in any suitable configuration and may be any type described above in the section entitled "Polynucleotides".

Further, example configurations are shown in Figures 6A-C, Figures 7A-C, and Figures 7G-I. Figures 6 and 7 are schematics of the provirus (e.g. after the 3' LTR has been copied to the 5' LTR during reverse transcription). The 5'LTR of the retroviral vector may comprise none of the genetic elements present in the provirus (e.g. the 5'LTR of the retroviral vector may comprise only R and U5 regions).

### Example convergent-divergent retroviral vectors

In one aspect, the present invention provides a retroviral vector comprising from 5' to 3' direction: a 5' LTR; one or more sense-oriented miRNA target sequences; a sense-oriented expression cassette; and a 3' LTR, wherein the 3' LTR comprises from 5' to 3' direction: a first insulator element, one or more antisense-oriented miRNA target sequences, and a second insulator element. In some embodiments, the first insulator element and the second insulator element are in a divergent orientation. In preferred embodiments, the retroviral vector is a lentiviral vector.

In some embodiments, the one or more sense-oriented miRNA target sequences are sense-oriented miR-142 target sequences. In some embodiments, the one or more sense-oriented miRNA target sequences are sense-oriented miR-142-3p target sequences. In some embodiments, the one or more sense-oriented miRNA target sequences comprise or consist of the nucleotide sequence of SEQ ID NO: 1, or a variant thereof having one or two nucleotide variations. In some embodiments, the one or more sense-oriented miRNA target sequences comprise or consist of the nucleotide sequence of SEQ ID NO: 1.

In some embodiments, the one or more sense-oriented miRNA target sequences comprise two or more sense-oriented miRNA target sequences. In some embodiments, the one or more sense-oriented miRNA target sequences comprises two sense-oriented miRNA target sequences. In some embodiments, the one or more sense-oriented miRNA target sequences comprise two or more sense-oriented miR-142 target sequences. In some embodiments, the one or more sense-oriented miRNA target sequences two or more sense-oriented miR-142-3p target sequences. In some embodiments, the one or more sense-oriented miRNA target sequences comprise or consist of the nucleotide sequence of SEQ ID NO: 21, or a variant thereof having one to four nucleotide variations. In some embodiments, the one or more sense-oriented miRNA target sequences comprise or consist of the nucleotide sequence of SEQ ID NO: 21, or a variant thereof having one or two nucleotide variations. In some embodiments, the one or more sense-oriented miRNA target sequences comprise or consist of the nucleotide sequence of SEQ ID NO: 21.

In some embodiments, the one or more antisense-oriented miRNA target sequences are sense-oriented miR-223 target sequences. In some embodiments, the one or more antisense-oriented miRNA target sequences are sense-oriented miR-223-3p target sequences. In some embodiments, the one or more antisense-oriented miRNA target sequences comprise or consist of the nucleotide sequence of SEQ ID NO: 12, or a variant thereof having one or two nucleotide variations. In some embodiments, the one or more antisense-oriented miRNA target sequences comprise or consist of the nucleotide sequence of SEQ ID NO: 12.

In some embodiments, the one or more antisense-oriented miRNA target sequences comprise two or more antisense-oriented miRNA target sequences. In some embodiments, the one or more antisense-oriented miRNA target sequences comprises two antisense-oriented miRNA target sequences. In some embodiments, the one or more antisense-oriented miRNA target sequences comprise two or more antisense-oriented miR-223 target sequences. In some embodiments, the one or more antisense-oriented miRNA target sequences two or more antisense-oriented miR-223-3p target sequences. In some embodiments, the one or more antisense-oriented miRNA target sequences comprise or consist of the nucleotide sequence of SEQ ID NO: 22, or a variant thereof having one to four nucleotide variations. In some embodiments, the one or more antisense-oriented miRNA target sequences comprise or consist of the nucleotide sequence of SEQ ID NO: 22, or a variant thereof having one or two nucleotide variations. In some embodiments, the one or more antisense-oriented miRNA target sequences comprise or consist of the nucleotide sequence of SEQ ID NO: 22.

In some embodiments, the first insulator element is an A2 insulator element and/or the second insulator element is an E2 insulator element. In some embodiments, the first insulator element is an A2 insulator element and the second insulator element is an E2 insulator element.

In some embodiments, the first insulator element comprises CTCF-binding motif A (e.g. SEQ ID NO: 23) and the second insulator element comprises CTCF-binding motif E (e.g. SEQ ID NO: 27).

In some embodiments, the first insulator element and the second insulator element each comprise or consist of a full length insulator sequence. In some embodiments, the first insulator element is a full-length A2 insulator sequence. In some embodiments, the first insulator element comprises or consists of the nucleotide sequence of SEQ ID NO: 103, or a nucleotide sequence having at least 70% identity thereto. In some embodiments, the first insulator element comprises or consists of the nucleotide sequence of SEQ ID NO: 103. In some embodiments, the second insulator element is a full-length E2 insulator sequence. In some embodiments, the second insulator element comprises or consists of the nucleotide sequence of SEQ ID NO: 55, or a nucleotide sequence having at least 70% identity thereto. In some embodiments, the second insulator element comprises or consists of the nucleotide sequence of SEQ ID NO: 55.

An example retroviral vector sequence is provided as SEQ ID NO: 93, wherein (X)ₙ encodes an expression cassette. In some embodiments, the retroviral vector comprises or consists of a nucleotide sequence which is at least 70% identical to SEQ ID NO: 93. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 93. In some embodiments, the retroviral vector comprises or consists of the nucleotide sequence SEQ ID NO: 93. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
 
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
AAGACAAAGAGCGAGATTCCGTCTCAAAGAAAAAAAAAGTAATGAAATGAAGCAAATGAGTC
CTAGAGCCAGTAAATGTCGTAAATGTCTCAGCTAGTCAGGTAGTAAAAGGTCTCAACTAGGC
AGTGGCAGAGCAGGATTCAAATTCAGGGCTGTTGTGATGCCTCCGCAGACTCTGAGCGCCAC
CTGGTGGTAATTTGTCTGTGCCTCTTCTGACGTGGAAGAACAGCAACTAACACACTAACACG
GCATTTACTATGGGCCAGCCATTGTATGCATTGTCAGTTTGTCAAATACCCCATCGTGTCAG
TTTGTCAAATACCCCACCGCGGTTTGATTTCCTTCACTCTGGAATATACTACAGAATGTACT
AATATAAATGTGCTTGCCTATGTCTAATCTAGAATATAATTATCCTGAACTCTACATGGTAT
TTTAGTCTAGAAAGTACTGTAGCTTCTCTGAGTTCTAAATCAAACCAGGAAACAGCAGAGGG
CGCTGGTGCTGTGCTTTTGCAAGTGAGATGTGTGCTCATTTCAAGGGTCCACAAAATTTTCT
GGCATGCTGTTTCCATGGAGAGGAAAGTAGGTGTTTTCTTTTTCTTTTTTTTTTTCAGTCAA
GCCACCTGCAAACTGATCTGCTTTTTGCTTGTACTGGGTCTCTCTGGTTAGACCAGATCTGA
GCCTGGGAGCTCTCTGGCTAACTAGGGAACCCACTGCTTAAGCCTCAATAAAGCTTGCCTTG
AGTGCTTCAAGTAGTGTGTGCCCGTCTGTTGTGTGACTCTGGTAACTAGAGATCCCTCAGAC
CCTTTTAGTCAGTGTGGAAAATCTCTAGCAG
wherein n is from 1000 to 6000
Example Div-Conv LV (full CI) (SEQ ID NO: 93)

In some embodiments, the first insulator element and the second insulator element each consist of a core insulator sequence. In some embodiments, the first insulator element is a core A2 insulator sequence. In some embodiments, the first insulator element comprises or consists of the nucleotide sequence of SEQ ID NO: 34, or a nucleotide sequence having at least 80.0% identity thereto. In some embodiments, the first insulator element comprises or consists of the nucleotide sequence of SEQ ID NO: 34. In some embodiments, the second insulator element is a core E2 insulator sequence. In some embodiments, the second insulator element comprises or consists of the nucleotide sequence of SEQ ID NO: 32, or a nucleotide sequence having at least 80.0% identity thereto. In some embodiments, the second insulator element comprises or consists of the nucleotide sequence of SEQ ID NO: 42.

An example retroviral vector sequence is provided as SEQ ID NO: 94, wherein (X)ₙ encodes an expression cassette. In some embodiments, the retroviral vector comprises or consists of a nucleotide sequence which is at least 70% identical to SEQ ID NO: 94. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 94. In some embodiments, the retroviral vector comprises or consists of the nucleotide sequence SEQ ID NO: 94. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
AAGACAACGCAGACTCTGAGCGCCACCTGGTGGTAATTTGTCTGTGCATGCATTGTCAGTTT
GTCAAATACCCCATCGTGTCAGTTTGTCAAATACCCCACCGCGGATCAAACCAGGAAACAGC
AGAGGGCGCTGGTGCTGTGCTGATCTGCTTTTTGCTTGTACTGGGTCTCTCTGGTTAGACCA
GATCTGAGCCTGGGAGCTCTCTGGCTAACTAGGGAACCCACTGCTTAAGCCTCAATAAAGCT
 
TGCCTTGAGTGCTTCAAGTAGTGTGTGCCCGTCTGTTGTGTGACTCTGGTAACTAGAGATCC
CTCAGACCCTTTTAGTCAGTGTGGAAAATCTCTAGCAG
wherein n is from 1000 to 6000
Example Div-Conv LV (core CI) (SEQ ID NO: 94)

### Example convergent retroviral vectors

In one aspect, the present invention provides a retroviral vector comprising from 5' to 3' direction: a 5' LTR; one or more sense-oriented miRNA target sequences; a sense-oriented expression cassette; a first insulator element; and a 3' LTR, wherein the 3' LTR comprises from 5' to 3' direction: one or more antisense-oriented miRNA target sequences, and a second insulator element. In some embodiments, the first insulator element and the second insulator element are in a divergent orientation. In preferred embodiments, the retroviral vector is a lentiviral vector.

In some embodiments, the one or more sense-oriented miRNA target sequences are sense-oriented miR-142 target sequences. In some embodiments, the one or more sense-oriented miRNA target sequences are sense-oriented miR-142-3p target sequences. In some embodiments, the one or more sense-oriented miRNA target sequences comprise or consist of the nucleotide sequence of SEQ ID NO: 1, or a variant thereof having one or two nucleotide variations. In some embodiments, the one or more sense-oriented miRNA target sequences comprise or consist of the nucleotide sequence of SEQ ID NO: 1.

In some embodiments, the one or more sense-oriented miRNA target sequences comprise two or more sense-oriented miRNA target sequences. In some embodiments, the one or more sense-oriented miRNA target sequences comprises two sense-oriented miRNA target sequences. In some embodiments, the one or more sense-oriented miRNA target sequences comprise two or more sense-oriented miR-142 target sequences. In some embodiments, the one or more sense-oriented miRNA target sequences two or more sense-oriented miR-142-3p target sequences. In some embodiments, the one or more sense-oriented miRNA target sequences comprise or consist of the nucleotide sequence of SEQ ID NO: 21, or a variant thereof having one to four nucleotide variations. In some embodiments, the one or more sense-oriented miRNA target sequences comprise or consist of the nucleotide sequence of SEQ ID NO: 21, or a variant thereof having one or two nucleotide variations. In some embodiments, the one or more sense-oriented miRNA target sequences comprise or consist of the nucleotide sequence of SEQ ID NO: 21.

In some embodiments, the one or more antisense-oriented miRNA target sequences are sense-oriented miR-223 target sequences. In some embodiments, the one or more antisense-oriented miRNA target sequences are sense-oriented miR-223-3p target sequences. In some embodiments, the one or more antisense-oriented miRNA target sequences comprise or consist of the nucleotide sequence of SEQ ID NO: 12, or a variant thereof having one or two nucleotide variations. In some embodiments, the one or more antisense-oriented miRNA target sequences comprise or consist of the nucleotide sequence of SEQ ID NO: 12.

In some embodiments, the one or more antisense-oriented miRNA target sequences comprise two or more antisense-oriented miRNA target sequences. In some embodiments, the one or more antisense-oriented miRNA target sequences comprises two antisense-oriented miRNA target sequences. In some embodiments, the one or more antisense-oriented miRNA target sequences comprise two or more antisense-oriented miR-223 target sequences. In some embodiments, the one or more antisense-oriented miRNA target sequences two or more antisense-oriented miR-223-3p target sequences. In some embodiments, the one or more antisense-oriented miRNA target sequences comprise or consist of the nucleotide sequence of SEQ ID NO: 22, or a variant thereof having one to four nucleotide variations. In some embodiments, the one or more antisense-oriented miRNA target sequences comprise or consist of the nucleotide sequence of SEQ ID NO: 22, or a variant thereof having one or two nucleotide variations. In some embodiments, the one or more antisense-oriented miRNA target sequences comprise or consist of the nucleotide sequence of SEQ ID NO: 22.

In some embodiments, the first insulator element is an A2 insulator element and/or the second insulator element is an E2 insulator element. In some embodiments, the first insulator element is an A2 insulator element and the second insulator element is an E2 insulator element.

In some embodiments, the first insulator element comprises CTCF-binding motif A (e.g. SEQ ID NO: 23) and the second insulator element comprises CTCF-binding motif E (e.g. SEQ ID NO: 27).

In some embodiments, the first insulator element and the second insulator element each comprise or consist of a full length insulator sequence. In some embodiments, the first insulator element is a full-length A2 insulator sequence. In some embodiments, the first insulator element comprises or consists of the nucleotide sequence of SEQ ID NO: 103, or a nucleotide sequence having at least 70% identity thereto. In some embodiments, the first insulator element comprises or consists of the nucleotide sequence of SEQ ID NO: 103. In some embodiments, the second insulator element is a full-length E2 insulator sequence. In some embodiments, the second insulator element comprises or consists of the nucleotide sequence of SEQ ID NO: 55, or a nucleotide sequence having at least 70% identity thereto. In some embodiments, the second insulator element comprises or consists of the nucleotide sequence of SEQ ID NO: 55.

An example retroviral vector sequence is provided as SEQ ID NO: 95, wherein (X)ₙ encodes an expression cassette. In some embodiments, the retroviral vector comprises or consists of a nucleotide sequence which is at least 70% identical to SEQ ID NO: 95. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 95. In some embodiments, the retroviral vector comprises or consists of the nucleotide sequence SEQ ID NO: 95. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGTCCATAAAGTAGGAAACACTACA
CGATTCCATAAAGTAGGAAACACTACA(X)nAGAGCGAGATTCCGTCTCAAAGAAAAAAAAAG
TAATGAAATGAAGCAAATGAGTCCTAGAGCCAGTAAATGTCGTAAATGTCTCAGCTAGTCAG
GTAGTAAAAGGTCTCAACTAGGCAGTGGCAGAGCAGGATTCAAATTCAGGGCTGTTGTGATG
CCTCCGCAGACTCTGAGCGCCACCTGGTGGTAATTTGTCTGTGCCTCTTCTGACGTGGAAGA
 
ACAGCAACTAACACACTAACACGGCATTTACTATGGGCCAGCCATTGTGAATTCGAGCTCGG
TACCTTTAAGACCAATGACTTACAAGGCAGCTGTAGATCTTAGCCACTTTTTAAAAGAAAAG
GGGGGACTGGAAGGGCTAATTCACTCCCAACGAAGACAAGATCTGCTTTTTATGCATTGTCA
GTTTGTCAAATACCCCATCGTGTCAGTTTGTCAAATACCCCACCGCGGTTTGATTTCCTTCA
CTCTGGAATATACTACAGAATGTACTAATATAAATGTGCTTGCCTATGTCTAATCTAGAATA
TAATTATCCTGAACTCTACATGGTATTTTAGTCTAGAAAGTACTGTAGCTTCTCTGAGTTCT
AAATCAAACCAGGAAACAGCAGAGGGCGCTGGTGCTGTGCTTTTGCAAGTGAGATGTGTGCT
CATTTCAAGGGTCCACAAAATTTTCTGGCATGCTGTTTCCATGGAGAGGAAAGTAGGTGTTT
TCTTTTTCTTTTTTTTTTTCAGTCAAGCCACCTGCAAACTGAAGACAAGATCTGCTTTTTGC
TTGTACTGGGTCTCTCTGGTTAGACCAGATCTGAGCCTGGGAGCTCTCTGGCTAACTAGGGA
ACCCACTGCTTAAGCCTCAATAAAGCTTGCCTTGAGTGCTTCAAGTAGTGTGTGCCCGTCTG
TTGTGTGACTCTGGTAACTAGAGATCCCTCAGACCCTTTTAGTCAGTGTGGAAAATCTCTAG
CAG
wherein n is from 1000 to 6000
Example Split-Conv LV (full CI) (SEQ ID NO: 95)

### Example parallel retroviral vectors

In one aspect, the present invention provides a retroviral vector comprising from 5' to 3' direction: a 5' LTR; one or more sense-oriented miRNA target sequences; a sense-oriented expression cassette; and a 3' LTR, wherein the 3' LTR comprises from 5' to 3' direction: one or more insulator elements and one or more antisense-oriented miRNA target sequences. In preferred embodiments, the retroviral vector is a lentiviral vector.

In some embodiments, the one or more sense-oriented miRNA target sequences are sense-oriented miR-142 target sequences. In some embodiments, the one or more sense-oriented miRNA target sequences are sense-oriented miR-142-3p target sequences. In some embodiments, the one or more sense-oriented miRNA target sequences comprise or consist of the nucleotide sequence of SEQ ID NO: 1, or a variant thereof having one or two nucleotide variations. In some embodiments, the one or more sense-oriented miRNA target sequences comprise or consist of the nucleotide sequence of SEQ ID NO: 1.

In some embodiments, the one or more sense-oriented miRNA target sequences comprise two or more sense-oriented miRNA target sequences. In some embodiments, the one or more sense-oriented miRNA target sequences comprises two sense-oriented miRNA target sequences. In some embodiments, the one or more sense-oriented miRNA target sequences comprise two or more sense-oriented miR-142 target sequences. In some embodiments, the one or more sense-oriented miRNA target sequences two or more sense-oriented miR-142-3p target sequences. In some embodiments, the one or more sense-oriented miRNA target sequences comprise or consist of the nucleotide sequence of SEQ ID NO: 21, or a variant thereof having one or two nucleotide variations. In some embodiments, the one or more sense-oriented miRNA target sequences comprise or consist of the nucleotide sequence of SEQ ID NO: 21.

In some embodiments, the one or more antisense-oriented miRNA target sequences are sense-oriented miR-223 target sequences. In some embodiments, the one or more antisense-oriented miRNA target sequences are sense-oriented miR-223-3p target sequences. In some embodiments, the one or more antisense-oriented miRNA target sequences comprise or consist of the nucleotide sequence of SEQ ID NO: 12, or a variant thereof having one or two nucleotide variations. In some embodiments, the one or more antisense-oriented miRNA target sequences comprise or consist of the nucleotide sequence of SEQ ID NO: 12.

In some embodiments, the one or more antisense-oriented miRNA target sequences comprise two or more antisense-oriented miRNA target sequences. In some embodiments, the one or more antisense-oriented miRNA target sequences comprises two antisense-oriented miRNA target sequences. In some embodiments, the one or more antisense-oriented miRNA target sequences comprise two or more antisense-oriented miR-223 target sequences. In some embodiments, the one or more antisense-oriented miRNA target sequences two or more antisense-oriented miR-223-3p target sequences. In some embodiments, the one or more antisense-oriented miRNA target sequences comprise or consist of the nucleotide sequence of SEQ ID NO: 22, or a variant thereof having one to four nucleotide variations. In some embodiments, the one or more antisense-oriented miRNA target sequences comprise or consist of the nucleotide sequence of SEQ ID NO: 22, or a variant thereof having one or two nucleotide variations. In some embodiments, the one or more antisense-oriented miRNA target sequences comprise or consist of the nucleotide sequence of SEQ ID NO: 22.

In some embodiments, the one or more insulator elements consist of an A1 insulator element and an A2 insulator element in a parallel orientation.

In some embodiments, the first insulator element comprises CTCF-binding motif A (e.g. SEQ ID NO: 23) and the second insulator element comprises CTCF-binding motif A (e.g. SEQ ID NO: 23).

In some embodiments, the A1 insulator element and the A2 insulator element each comprise or consist of a full length insulator sequence. In some embodiments, the A1 insulator element is a full-length A1 insulator sequence. In some embodiments, the A1 insulator element comprises or consists of the nucleotide sequence of SEQ ID NO: 64, or a nucleotide sequence having at least 70% identity thereto. In some embodiments, the A1 insulator element comprises or consists of the nucleotide sequence of SEQ ID NO: 64. In some embodiments, the A2 insulator element is a full-length A2 insulator sequence. In some embodiments, the A2 insulator element comprises or consists of the nucleotide sequence of SEQ ID NO: 103, or a nucleotide sequence having at least 70% identity thereto. In some embodiments, the A2 insulator element comprises or consists of the nucleotide sequence of SEQ ID NO: 103.

An example retroviral vector sequence is provided as SEQ ID NO: 96, wherein (X)ₙ encodes an expression cassette. In some embodiments, the retroviral vector comprises or consists of a nucleotide sequence which is at least 70% identical to SEQ ID NO: 96. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 96. In some embodiments, the retroviral vector comprises or consists of the nucleotide sequence SEQ ID NO: 96. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
 
AAGACAAATGACGAGAGAGACAGAAGAATTCTTCAAGGTTAGTGTGTCCAGCATGCAACCTT
TCCTTCCTGGATGAGCATCCCTGGAATAGGAGAGCCAGCCTGCCTCCTGCGCTGGCACAGAG
CCCGGTTCCCTAGACAACTGCCTCTCCAAATCTGATGTCCAGCGCCACCTGGTGTCCACATC
AAGCAGACACAATTAATAGTCAACCTGTTCAGGAAAACTGTGAGGGGAAAAAAAAGAAAGA
GGATTTATGAAGGGAAAAGAAAGTTTAGAGGATATGCCACGATAGAGCGAGATTCCGTCTCA
AAGAAAAAAAAAAGTAATGAAATGAAGCAAATGAGTCCTAGAGCCAGTAAATGTCGTAAATGT
CTCAGCTAGTCAGGTAGTAAAAGGTCTCAACTAGGCAGTGGCAGAGCAGGATTCAAATTCAG
GGCTGTTGTGATGCCTCCGCAGACTCTGAGCGCCACCTGGTGGTAATTTGTCTGTGCCTCTT
CTGACGTGGAAGAACAGCAACTAACACACTAACACGGCATTTACTATGGGCCAGCCATTGTA
TGCATTGTCAGTTTGTCAAATACCCCATCGTGTCAGTTTGTCAAATACCCCAGATCTGCTTT
TTGCTTGTACTGGGTCTCTCTGGTTAGACCAGATCTGAGCCTGGGAGCTCTCTGGCTAACTA
GGGAACCCACTGCTTAAGCCTCAATAAAGCTTGCCTTGAGTGCTTCAAGTAGTGTGTGCCCG
TCTGTTGTGTGACTCTGGTAACTAGAGATCCCTCAGACCCTTTTAGTCAGTGTGGAAAATCT
CTAGCAG
wherein n is from 1000 to 6000
Example CI-COMBO (A1 and A2 full CI) (SEQ ID NO: 96)

In some embodiments, the A1 insulator element and the A2 insulator element each comprise or consist of a core insulator sequence. In some embodiments, the A1 insulator element is a core A1 insulator sequence. In some embodiments, the A1 insulator element comprises or consists of the nucleotide sequence of SEQ ID NO: 33, or a nucleotide sequence having at least 80.0% identity thereto. In some embodiments, the A1 insulator element comprises or consists of the nucleotide sequence of SEQ ID NO: 33. In some embodiments, the A2 insulator element is a core A2 insulator sequence. In some embodiments, the A2 insulator element comprises or consists of the nucleotide sequence of SEQ ID NO: 34, or a nucleotide sequence having at least 80.0% identity thereto. In some embodiments, the A2 insulator element comprises or consists of the nucleotide sequence of SEQ ID NO: 34.

An example retroviral vector sequence is provided as SEQ ID NO: 97, wherein (X)ₙ encodes an expression cassette. In some embodiments, the retroviral vector comprises or consists of a nucleotide sequence which is at least 70% identical to SEQ ID NO: 97. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 97. In some embodiments, the retroviral vector comprises or consists of the nucleotide sequence SEQ ID NO: 97. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
 
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
AAGACAAATCTGATGTCCAGCGCCACCTGGTGTCCACATCAAGCAGACGCAGACTCTGAGCG
CCACCTGGTGGTAATTTGTCTGTGCTTAATTAAATGCATTGTCAGTTTGTCAAATACCCCAT
CGTGTCAGTTTGTCAAATACCCCAGATCTGCTTTTTGCTTGTACTGGGTCTCTCTGGTTAGA
CCAGATCTGAGCCTGGGAGCTCTCTGGCTAACTAGGGAACCCACTGCTTAAGCCTCAATAAA
GCTTGCCTTGAGTGCTTCAAGTAGTGTGTGCCCGTCTGTTGTGTGACTCTGGTAACTAGAGA
TCCCTCAGACCCTTTTAGTCAGTGTGGAAAATCTCTAGCAG
wherein n is from 1000 to 6000
Example CI-COMBO (A1 and A2 core CI) (SEQ ID NO: 97)

In some embodiments, the one or more insulator elements consist of an A2 insulator element.

In some embodiments, the A2 insulator element comprises CTCF-binding motif A (e.g. SEQ ID NO: 23).

In some embodiments, the A2 insulator element is a full-length A2 insulator sequence. In some embodiments, the A2 insulator element comprises or consists of the nucleotide sequence of SEQ ID NO: 103, or a nucleotide sequence having at least 70% identity thereto. In some embodiments, the A2 insulator element comprises or consists of the nucleotide sequence of SEQ ID NO: 103.

An example retroviral vector sequence is provided as SEQ ID NO: 98, wherein (X)ₙ encodes an expression cassette. In some embodiments, the retroviral vector comprises or consists of a nucleotide sequence which is at least 70% identical to SEQ ID NO: 98. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 98. In some embodiments, the retroviral vector comprises or consists of the nucleotide sequence SEQ ID NO: 98. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
AAGACAAAGAGCGAGATTCCGTCTCAAAGAAAAAAAAAGTAATGAAATGAAGCAAATGAGTC
CTAGAGCCAGTAAATGTCGTAAATGTCTCAGCTAGTCAGGTAGTAAAAGGTCTCAACTAGGC
AGTGGCAGAGCAGGATTCAAATTCAGGGCTGTTGTGATGCCTCCGCAGACTCTGAGCGCCAC
CTGGTGGTAATTTGTCTGTGCCTCTTCTGACGTGGAAGAACAGCAACTAACACACTAACACG
GCATTTACTATGGCCAGCCATTGTATGCATTGTCAGTTTGTCAAATACCCCATCGTGTCAG
TTTGTCAAATACCCCAGATCTGCTTTTTGCTTGTACTGGGTCTCTCTGGTTAGACCAGATCT
GAGCCTGGGAGCTCTCTGGCTAACTAGGGAACCCACTGCTTAAGCCTCAATAAAGCTTGCCT
TGAGTGCTTCAAGTAGTGTGTGCCCGTCTGTTGTGTGACTCTGGTAACTAGAGATCCCTCAG
ACCCTTTTAGTCAGTGTGGAAAATCTCTAGCAG
wherein n is from 1000 to 6000
Example CI-COMBO (A2 full CI) (SEQ ID NO: 98)

In some embodiments, the A2 insulator element is a core A2 insulator sequence. In some embodiments, the A2 insulator element comprises or consists of the nucleotide sequence of SEQ ID NO: 34, or a nucleotide sequence having at least 80.0% identity thereto. In some embodiments, the A2 insulator element comprises or consists of the nucleotide sequence of SEQ ID NO: 34.

An example retroviral vector sequence is provided as SEQ ID NO: 99, wherein (X)ₙ encodes an expression cassette. In some embodiments, the retroviral vector comprises or consists of a nucleotide sequence which is at least 70% identical to SEQ ID NO: 99. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 99. In some embodiments, the retroviral vector comprises or consists of the nucleotide sequence SEQ ID NO: 99. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
AAGACAACGCAGACTCTGAGCGCCACCTGGTGGTAATTTGTCTGTGCATGCATTGTCAGTTT
 
GTCAAATACCCCATCGTGTCAGTTTGTCAAATACCCCAGATCTGCTTTTTGCTTGTACTGGG
TCTCTCTGGTTAGACCAGATCTGAGCCTGGGAGCTCTCTGGCTAACTAGGGAACCCACTGCT
TAAGCCTCAATAAAGCTTGCCTTGAGTGCTTCAAGTAGTGTGTGCCCGTCTGTTGTGTGACT
CTGGTAACTAGAGATCCCTCAGACCCTTTTAGTCAGTGTGGAAAATCTCTAGCAG
wherein n is from 1000 to 6000
Example CI-COMBO (A2 core CI) (SEQ ID NO: 99)

In some embodiments, the one or more insulator elements consist of an A1 insulator element.

In some embodiments, the A1 insulator element comprises CTCF-binding motif A (e.g. SEQ ID NO: 23).

In some embodiments, the A1 insulator element is a core A1 insulator sequence. In some embodiments, the A1 insulator element comprises or consists of the nucleotide sequence of SEQ ID NO: 33, or a nucleotide sequence having at least 80.0% identity thereto. In some embodiments, the A1 insulator element comprises or consists of the nucleotide sequence of SEQ ID NO: 33.

An example retroviral vector sequence is provided as SEQ ID NO: 100, wherein (C)ₙ encodes an expression cassette. In some embodiments, the retroviral vector comprises or consists of a nucleotide sequence which is at least 70% identical to SEQ ID NO: 100. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 100. In some embodiments, the retroviral vector comprises or consists of the nucleotide sequence SEQ ID NO: 100. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
 
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
AAGACAAATCTGATGTCCAGCGCCACCTGGTGTCCACATCAAGCAGAATGCATTGTCAGTTT
GTCAAATACCCCATCGTGTCAGTTTGTCAAATACCCCAGATCTGCTTTTTGCTTGTACTGGG
TCTCTCTGGTTAGACCAGATCTGAGCCTGGGAGCTCTCTGGCTAACTAGGGAACCCACTGCT
TAAGCCTCAATAAAGCTTGCCTTGAGTGCTTCAAGTAGTGTGTGCCCGTCTGTTGTGTGACT
CTGGTAACTAGAGATCCCTCAGACCCTTTTAGTCAGTGTGGAAAATCTCTAGCAG
wherein n is from 1000 to 6000
Example CI-COMBO (A1 core CI) (SEQ ID NO: 100)

In some embodiments, the one or more insulator elements consist of a B2 insulator element.

In some embodiments, the B2 insulator element comprises CTCF-binding motif B (e.g. SEQ ID NO: 24).

In some embodiments, the B2 insulator element is a core B2 insulator sequence. In some embodiments, B2 insulator element comprises or consists of the nucleotide sequence of SEQ ID NO: 35, or a nucleotide sequence having at least 80.0% identity thereto. In some embodiments, the B2 insulator element comprises or consists of the nucleotide sequence of SEQ ID NO: 35.

An example retroviral vector sequence is provided as SEQ ID NO: 101, wherein (X)ₙ encodes an expression cassette. In some embodiments, the retroviral vector comprises or consists of a nucleotide sequence which is at least 70% identical to SEQ ID NO: 101. In some embodiments, the retroviral vector comprises or consists of a nucleotide sequence which is at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% identical to SEQ ID NO: 101. In some embodiments, the retroviral vector comprises or consists of the nucleotide sequence SEQ ID NO: 101. In some embodiments, n is from 1000 to 5000, from 1000 to 4000 or from 1000 to 3000. 
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACCTGAAAGCGAAAGGGAAACCAGAGCTCTCTCGACGCAGGACTCGGCTT
GCTGAAGCGCGCACGGCAAGAGGCGAGGGGCGGCGACTGGTGAGTACGCCAAAAATTTTGAC
TAGCGGAGGCTAGAAGGAGAGAGATGGGTGCGAGAGCGTCAGTATTAAGCGGGGGAGAATTA
GATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATC
AGAAGGCTGTAGACAAATACTGGGACAGCTACAACCATCCCTTCAGACAGGATCAGAAGAAC
TTAGATCATTATATAATACAGTAGCAACCCTCTATTGTGTGCATCAAAGGATAGAGATAAAA
GACACCAAGGAAGCTTTAGACAAGATAGAGGAAGAGCAAAACAAAAGTAAGACCACCGCACA
GCAAGCGGCCGCTGATCTTCAGACCTGGAGGAGGAGATATGAGGGACAATTGGAGAAGTGAA
TTATATAAATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTGGGTTCTTGG
GAGCAGCAGGAAGCACTATGGGCGCAGCCTCAATGACGCTGACGGTACAGGCCAGACAATTA
TTGTCTGGTATAGTGCAGCAGCAGAACAATTTGCTGAGGGCTATTGAGGCGCAACAGCATCT
GTTGCAACTCACAGTCTGGGGCATCAAGCAGCTCCAGGCAAGAATCCTGGCTGTGGAAAGAT
ACCTAAAGGATCAACAGCTCCTGGGGATTTGGGGTTGCTCTGGAAAACTCATTTGCACCACT
GCTGTGCCTTGGAATGCTAGTTGGAGTAATAAATCTCTGGAACAGATTTGGAATCACACGAC
CTGGATGGAGTGGGACAGAGAAATTAACAATTACACAAGCTTAATACACTCCTTAATTGAAG
AATCGCAAAACCAGCAAGAAAAGAATGAACAAGAATTATTGGAATTAGATAAATGGGCAAGT
TTGTGGAATTGGTTTAACATAACAAATTGGCTGTGGTATATAAAATTATTCATAATGATAGT
AGGAGGCTTGGTAGGTTTAAGAATAGTTTTTGCTGTACTTTCTATAGTGAATAGAGTTAGGC
AGGGATATTCACCATTATCGTTTCAGACCCACCTCCCAACCCCGAGGGGACCCGACAGGCCC
GAAGGAATAGAAGAAGAAGGTGGAGAGAGAGACAGAGACAGATCCATTCGATTAGTGAACGG
ATCTCGACGGTATCGGTTAACTTTTAAAAGAAAAGGGGGGATTGGGGGGTACAGTGCAGGGG
AAAGAATAGTAGACATAATAGCAACAGACATACAAACTAAAGAATTACAAAAACAAATTACA
AAAATTCAAAATTTTATCGATCACGAGACTAGCCTCGAGAAGCTTGTCCATAAAGTAGGAAA
CACTACACGATTCCATAAAGTAGGAAACACTACA(X)nTGGAAGGGCTAATTCACTCCCAACG
AAGACAACTGCGTCCAGCAGCTCCCCCTGGTGGCCAGAAGCAAACGCTATGCATTGTCAGT
TTGTCAAATACCCCATCGTGTCAGTTTGTCAAATACCCCAGATCTGCTTTTTGCTTGTACTG
GGTCTCTCTGGTTAGACCAGATCTGAGCCTGGGAGCTCTCTGGCTAACTAGGGAACCCACTG
CTTAAGCCTCAATAAAGCTTGCCTTGAGTGCTTCAAGTAGTGTGTGCCCGTCTGTTGTGTGA
CTCTGGTAACTAGAGATCCCTCAGACCCTTTTAGTCAGTGTGGAAAATCTCTAGCAG
wherein n is from 1000 to 6000
Example CI-COMBO (B2 core CI) (SEQ ID NO: 101)

### Retroviral vector (antisense-oriented expression cassette)

In one aspect, the present invention provides a retroviral vector comprising from 5' to 3' direction: a 5' LTR; an antisense-oriented expression cassette; and a 3' LTR, wherein the retroviral vector further comprises: (a) one or more antisense-oriented miRNA target sequences; (b) one or more sense-oriented miRNA target sequences; and (c) one or more insulator elements. In preferred embodiments, the retroviral vector is a lentiviral vector.

As described above, in the context of a retroviral vector, an "antisense-oriented" expression cassette may have the opposite orientation to the retroviral vector genome. For example, following conversion of a retroviral vector into double-stranded DNA, the expression cassette may be transcribed in the direction of from the 3' LTR to the 5' LTR.

In preferred embodiments, the one or more antisense-oriented miRNA target sequences are upstream from the expression cassette and/or downstream from the expression cassette. In some embodiments, the one or more sense-oriented miRNA target sequences are upstream from the expression cassette and/or downstream from the expression cassette. In some embodiments, the one or more insulator elements are upstream from the expression cassette and/or downstream from the expression cassette. In some embodiments, the one or more antisense-oriented miRNA target sequences are upstream from the expression cassette and/or downstream from the expression cassette; the one or more sense-oriented miRNA target sequences are upstream from the expression cassette and/or downstream from the expression cassette; and the one or more insulator elements are upstream from the expression cassette and/or downstream from the expression cassette.

In some embodiments, the 3' LTR does not comprise one or more antisense-oriented miRNA target sequences. In some embodiments, the 3' LTR does not comprise one or more sense-oriented miRNA target sequences. In some embodiments, the 3' LTR does not comprise one or more miRNA target sequences.

In some embodiments, the one or more sense-oriented miRNA target sequences are sense-oriented miR-142 target sequences. In some embodiments, the one or more sense-oriented miRNA target sequences are sense-oriented miR-142-3p target sequences. In some embodiments, the one or more sense-oriented miRNA target sequences comprise or consist of the nucleotide sequence of SEQ ID NO: 1, or a variant thereof having one or two nucleotide variations. In some embodiments, the one or more sense-oriented miRNA target sequences comprise or consist of the nucleotide sequence of SEQ ID NO: 1.

In some embodiments, the one or more sense-oriented miRNA target sequences comprise two or more sense-oriented miRNA target sequences. In some embodiments, the one or more sense-oriented miRNA target sequences comprises two sense-oriented miRNA target sequences. In some embodiments, the one or more sense-oriented miRNA target sequences comprise two or more sense-oriented miR-142 target sequences. In some embodiments, the one or more sense-oriented miRNA target sequences two or more sense-oriented miR-142-3p target sequences. In some embodiments, the one or more sense-oriented miRNA target sequences comprise or consist of the nucleotide sequence of SEQ ID NO: 21, or a variant thereof having one or two nucleotide variations. In some embodiments, the one or more sense-oriented miRNA target sequences comprise or consist of the nucleotide sequence of SEQ ID NO: 21.

In some embodiments, the one or more antisense-oriented miRNA target sequences are sense-oriented miR-223 target sequences. In some embodiments, the one or more antisense-oriented miRNA target sequences are sense-oriented miR-223-3p target sequences. In some embodiments, the one or more antisense-oriented miRNA target sequences comprise or consist of the nucleotide sequence of SEQ ID NO: 12, or a variant thereof having one or two nucleotide variations. In some embodiments, the one or more antisense-oriented miRNA target sequences comprise or consist of the nucleotide sequence of SEQ ID NO: 12.

In some embodiments, the one or more antisense-oriented miRNA target sequences comprise two or more antisense-oriented miRNA target sequences. In some embodiments, the one or more antisense-oriented miRNA target sequences comprises two antisense-oriented miRNA target sequences. In some embodiments, the one or more antisense-oriented miRNA target sequences comprise two or more antisense-oriented miR-223 target sequences. In some embodiments, the one or more antisense-oriented miRNA target sequences two or more antisense-oriented miR-223-3p target sequences. In some embodiments, the one or more antisense-oriented miRNA target sequences comprise or consist of the nucleotide sequence of SEQ ID NO: 22, or a variant thereof having one to four nucleotide variations. In some embodiments, the one or more antisense-oriented miRNA target sequences comprise or consist of the nucleotide sequence of SEQ ID NO: 22, or a variant thereof having one or two nucleotide variations. In some embodiments, the one or more antisense-oriented miRNA target sequences comprise or consist of the nucleotide sequence of SEQ ID NO: 22.

The miRNA target sequences and the one or more insulator elements may be in any configuration and may be any type described above in the section entitled "Polynucleotides".

Further, example configurations are shown in Figures 6D-G, Figures 7D-F, and Figures 7J-L. As described above, Figures 6 and 7 are schematics of the provirus. The 5'LTR of the retroviral vector may comprise none of the genetic elements present in the provirus (e.g. the 5'LTR of the retroviral vector may comprise only R and U5 regions).

### Methods of improving the safety of a polynucleotide, vector, or retroviral vector

In one aspect, the present invention provides a method of improving the safety of a polynucleotide or vector (e.g. by reducing genotoxicity), wherein the method comprises introducing into the polynucleotide or vector:
(a) one or more miRNA target sequences having the same orientation as the expression cassette;
(b) one or more miRNA target sequences having the opposite orientation as the expression cassette; and/or
(c) one or more insulator elements.

The methods be carried out using standard recombinant DNA techniques such as site-directed mutagenesis or gene-editing. Where insertions are to be made, synthetic DNA encoding the insertion together with 5' and 3' flanking regions corresponding to the naturally-occurring sequence either side of the insertion site may be made. The flanking regions may contain convenient restriction sites corresponding to sites in the naturally-occurring sequence so that the sequence may be cut with the appropriate enzyme(s) and the synthetic DNA ligated into the cut. Numerous other standard techniques are known in the art for manipulation of DNA sequences and other known techniques may also be used, such as methods using RNA-guided nucleases.

The configurations and types of miRNA target sequences and insulator elements introduced into the polynucleotide or vector and their configurations may be any described above in the sections entitled "Polynucleotides" or "Vectors".

In another aspect, the present invention provides a method of improving the safety of a retroviral vector (e.g. by reducing genotoxicity) comprising a sense-oriented expression cassette, wherein the method comprises introducing into the retroviral vector (a) one or more sense-oriented miRNA target sequences upstream from the sense-oriented expression cassette. In preferred embodiments, the retroviral vector is a lentiviral vector.

In some embodiments, the method further comprises introducing into the retroviral vector (b) one or more antisense-oriented miRNA target sequences upstream from the sense-oriented expression cassette and/or downstream from the sense-oriented expression cassette. In some embodiments, the method further comprises introducing into the retroviral vector (c) one or more insulator elements upstream from the sense-oriented expression cassette and/or downstream from the sense-oriented expression cassette. In some embodiments, the method further comprises introducing into the retroviral vector (b) one or more antisense-oriented miRNA target sequences upstream from the sense-oriented expression cassette and/or downstream from the sense-oriented expression cassette and (c) one or more insulator elements upstream from the sense-oriented expression cassette and/or downstream from the sense-oriented expression cassette.

The configurations and types of miRNA target sequences and insulator elements introduced into the retroviral vector may be any described above in the section entitled "Retroviral vector (sense-oriented expression cassette)".

In another aspect, the present invention provides a method of improving the safety of a retroviral vector (e.g. by reducing genotoxicity) comprising an antisense-oriented expression cassette, wherein the method comprises introducing into the retroviral vector: (a) one or more antisense-oriented miRNA target sequences upstream from the antisense-oriented expression cassette and/or downstream from the antisense-oriented expression cassette; (b) one or more sense-oriented miRNA target sequences upstream from the antisense-oriented expression cassette and/or downstream from the antisense-oriented expression cassette; and (c) one or more insulator elements upstream from the antisense-oriented expression cassette and/or downstream from the antisense-oriented expression cassette. In preferred embodiments, the retroviral vector is a lentiviral vector.

The configurations and types of miRNA target sequences and insulator elements introduced into the retroviral vector may be any described above in the section entitled "Retroviral vector (antisense-oriented expression cassette)".

### Safety and reduced genotoxicity of polynucleotides, vectors, or retroviral vectors

The polynucleotides, vectors, or retroviral vectors of the present invention may have improved safety.

miRNA target sequences upstream from the expression cassette and/or downstream from the expression cassette may improve safety of the polynucleotides, vectors, or retroviral vectors by avoiding and/or reducing aberrant transcription, aberrant splicing, and/or read-through events. The miRNA target sequences may specifically degrade aberrant transcripts. Insulator elements may improve safety of the polynucleotides, vectors, or retroviral vectors by avoiding and/or reducing enhancer-mediated gene activation.

The polynucleotides, vectors, or retroviral vectors of the present invention may be substantially non-genotoxic and/or have reduced genotoxicity. As used herein, "genotoxicity" may refer to damage to the genetic information within a cell causing mutations, which may lead to cancer. The reduction in genotoxicity may be determined by any method known in the art (see e.g. Cesana, D., et al. 2014. Mol Ther 22, 774-785).

In some embodiments, the polynucleotide, vector, or retroviral vector is substantially non-genotoxic compared to a mock control. A "mock control" may refer to a control setting where the specimen is treated the same way as the specimen administered the polynucleotide, vector, or retroviral vector, except no polynucleotide, vector, retroviral vector is administered.

In some embodiments, the polynucleotide, vector, or retroviral vector has reduced genotoxicity compared to a control polynucleotide, vector, or retroviral vector. A "control polynucleotide, vector, or retroviral vector" may refer to a polynucleotide, vector, or retroviral vector in which one or more of the safety genetic elements are absent or in which all of the safety genetic elements are absent, but in which all other genetic elements are present. In some embodiments, a control polynucleotide, vector, or retroviral vector is identical to the polynucleotide, vector, or retroviral vector of the present invention but does not comprise any of: (a) a miRNA target sequence having the same orientation as the expression cassette, upstream from the expression cassette and/or downstream from the transcription termination site; (b) a miRNA target sequence having the opposite orientation as the expression cassette, upstream from the expression cassette and/or downstream from the expression cassette; or (c) an insulator element, upstream from the expression cassette and/or downstream from the expression cassette.

The genotoxicity of the polynucleotides, vectors, or retroviral vectors may be determined by an *in vivo* genotoxicity assay based on a tumor prone mouse model. A tumor-prone mouse model based on a *Cdkn2a*^{+/-} mice has previously been described (see e.g. Cesana, D., et al. 2014. Mol Ther 22, 774-785). *Cdkn2a*^{+/-} mice are significantly more sensitive than wild type mice since they develop early tumors when treated with genotoxic retroviral vectors.

In the *Cdkn2a*^{+/-} mouse model, the survival kinetics may be used to determine the genotoxicity of the polynucleotides, vectors, or retroviral vectors. In some embodiments, *Cdkn2a*^{+/-} mice which are administered the polynucleotides, vectors, or retroviral vectors of the present invention do not have a significantly reduced survival compared to *Cdkn2a*^{+/-} mice which are administered a mock control. In some embodiments, *Cdkn2a*^{+/-} mice which are administered the polynucleotides, vectors, or retroviral vectors of the present invention have a significantly increased survival compared to *Cdkn2a*^{+/-} mice which are administered a control polynucleotide, vector, or retroviral vector.

In the *Cdkn2a*^{+/-} mouse model, the time to tumor onset may be used to determine the genotoxicity of the polynucleotides, vectors, or retroviral vectors. In some embodiments, *Cdkn2a*^{+/-} mice which are administered the polynucleotides, vectors, or retroviral vectors of the present invention do not have a significantly increased time to tumor onset compared to *Cdkn2a*^{+/-} mice which are administered a mock control. In some embodiments, *Cdkn2a*^{+/-} mice which are administered the polynucleotides, vectors, or retroviral vectors of the present invention have a significantly reduced time to tumor onset compared to *Cdkn2a*^{+/-} mice which are administered a control polynucleotide, vector, or retroviral vector.

Alternatively or additionally, in the *Cdkn2a*^{+/-} mouse model, histopathological analysis may be used to determine the genotoxicity of the polynucleotides, vectors, or retroviral vectors. The histopathological analysis may determine the incidence of tumors (e.g. liver tumors) and/or tissue abnormalities (e.g. focal cell alterations). In some embodiments, *Cdkn2a*^{+/-} mice which are administered the polynucleotides, vectors, or retroviral vectors of the present invention do not develop tumours and/or tissue abnormalities. In some embodiments, *Cdkn2a*^{+/-} mice which are administered the polynucleotides, vectors, or retroviral vectors of the present invention do not develop significantly more tumours and/or tissue abnormalities compared to *Cdkn2a*^{+/-} mice which are administered a mock control. In some embodiments, *Cdkn2a*^{+/-} mice which are administered the polynucleotides, vectors, or retroviral vectors of the present invention develop significantly fewer tumours and/or tissue abnormalities compared to *Cdkn2a*^{+/-} mice which are administered a control polynucleotide, vector, or retroviral vector.

Alternatively or additionally, in the *Cdkn2a*^{+/-} mouse model, the frequency of insertions targeting common integration sites (CIS) genes may be used to determine the genotoxicity of the polynucleotides, vectors, or retroviral vectors. The CIS genes may be cancer-causing CIS genes. In some embodiments, two or less, one or less, or no CIS genes are identified in *Cdkn2a*^{+/-} mice which are administered the polynucleotides, vectors, or retroviral vectors of the present invention. In some embodiments, significantly fewer CIS genes are identified in *Cdkn2a*^{+/-} mice which are administered the polynucleotides, vectors, or retroviral vectors of the present invention compared to *Cdkn2a*^{+/-} mice which are administered a control polynucleotide, vector, or retroviral vector.

### Variants, derivatives, analogues, and fragments

In addition to the specific polypeptides and polynucleotides mentioned herein, the invention also encompasses variants, derivatives, analogues, and fragments thereof.

In the context of the present invention, a "variant" of any given sequence is a sequence in which the specific sequence of residues (whether amino acid or nucleic acid residues) has been modified in such a manner that the polypeptide or polynucleotide in question substantially retains at least one of its functions. Suitably, for example, any amino acid changes in the TCR sequences should maintain the capacity of the TCR to bind the peptide presented by MHC molecules.

A variant amino acid or nucleic acid sequence can be obtained by addition, deletion, substitution, modification, replacement and/or variation of at least one residue present in the naturally-occurring polypeptide or polynucleotide. A variant amino acid sequence of the invention referred to as having up to three amino acid substitutions, additions or deletions may have, for example, one, two or three amino acid substitutions, additions or deletions. Suitably, for example, the variation may be concentrated in one or more regions, such as the constant regions, the linker, or the framework regions of the α or β chains, or they may be spread throughout the TCR.

The term "derivative" as used herein, in relation to proteins or polypeptides of the invention includes any substitution of, variation of, modification of, replacement of, deletion of and/or addition of one (or more) amino acid residues from or to the sequence providing that the resultant protein or polypeptide substantially retains at least one of its functions.

The term "analogue" as used herein, in relation to polypeptides or polynucleotides includes any mimetic, that is, a chemical compound that possesses at least one of the functions of the polypeptides or polynucleotides which it mimics.

Polypeptides of the present invention may have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Typically, amino acid substitutions may be made, for example from 1, 2 or 3 to 10 or 20 substitutions provided that the modified sequence substantially retains the required activity or ability. Amino acid substitutions may include the use of non-naturally occurring analogues. A substitution may involve replacement of an amino acid for a similar amino acid (a conservative substitution). A similar amino acid is one which has a side chain moiety with related properties as grouped together, for example as shown below:
(i) basic side chains: lysine (K), arginine (R), histidine (H);
(ii) acidic side chains: aspartic acid (D) and glutamic acid (E);
(iii) uncharged polar side chains: asparagine (N), glutamine (Q), serine (S), threonine (T) and tyrosine (Y); or
(iv) non-polar side chains: glycine (G), alanine (A), valine (V), leucine (L), isoleucine (I), proline (P), phenylalanine (F), methionine (M), tryptophan (W) and cysteine (C).

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The effect of additions, deletions, substitutions, modifications, replacements and/or variations may be predicted using any suitable prediction tool e.g. SIFT (Vaser, R., et al., 2016. Nature protocols, 11(1), pp.1-9), PolyPhen-2 (Adzhubei, I., et al., 2013. Current protocols in human genetics, 76(1), pp.7-20), CADD (Rentzsch, P., et al., 2021. Genome medicine, 13(1), pp.1-12), REVEL (loannidis, N.M., et al., 2016. The American Journal of Human Genetics, 99(4), pp.877-885), MetaLR (Dong, C., et al., 2015. Human molecular genetics, 24(8), pp.2125-2137), and/or MutationAssessor (Reva, B., et al., 2011. Nucleic acids research, 39(17), pp.e118-e118).

Typically, a variant may have a certain identity with the subject amino acid sequence or the subject nucleotide sequence. Suitably, reference to a sequence which has a percent identity to any one of the SEQ ID NOs detailed herein refers to a sequence which has the stated percent identity over the entire length of the SEQ ID NO referred to.

In the present context, a variant amino acid sequence is taken to include an amino acid sequence which may be at least 50%, at least 55%, at least 60%, at least 65%, at least 70%, at least 75%, at least 80%, at least 85% or at least 90% identical, suitably at least 95%, at least 96%, at least 97%, at least 98% or at least 99% identical to the subject sequence. Although a variant can also be considered in terms of similarity (i.e. amino acid residues having similar chemical properties/functions), in the context of the present invention it is preferred to express in terms of sequence identity.

In the present context, a variant nucleotide sequence is taken to include a nucleotide sequence which may be at least 50%, at least 55%, at least 60%, at least 65%, at least 70%, at least 75%, at least 80%, at least 85% or at least 90% identical, suitably at least 95%, at least 96%, at least 97%, at least 98% or at least 99% identical to the subject sequence. Although a variant can also be considered in terms of similarity, in the context of the present invention it is preferred to express it in terms of sequence identity.

Sequence identity comparisons can be conducted by eye, or more usually, with the aid of readily available sequence comparison programs. These commercially available computer programs can calculate percent identity between two or more sequences.

Percent identity may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent identity when a global alignment is performed. Consequently, most sequence comparison methods are designed to produce optimal alignments that take into consideration possible insertions and deletions without penalising unduly the overall identity score. This is achieved by inserting "gaps" in the sequence alignment to try to maximise local identity.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percent identity therefore firstly requires the production of an optimal alignment, taking into consideration gap penalties. A suitable computer program for carrying out such an alignment is the GCG Wisconsin Bestfit package (see e.g. Devereux, J., et al., 1984. Nucleic acids research, 12(1), pp.387-395). Examples of other software that can perform sequence comparisons include, but are not limited to, the BLAST package (see e.g. Altschul, S.F., et al., 1990. Journal of molecular biology, 215(3), pp.403-410), BLAST 2 (see e.g. Tatusova, T.A. and Madden, T.L., 1999. FEMS microbiology letters, 174(2), pp.247-250), FASTA (see e.g. Pearson, W.R. and Lipman, D.J., 1988. PNAS, 85(8), pp.2444-2448.), EMBOSS Needle (Madeira, F., et al., 2019. Nucleic acids research, 47(W1), pp.W636-W641) and the GENEWORKS suite of comparison tools. For some applications, it is preferred to use EMBOSS Needle.

Although the final percent identity can be measured, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix.

Once the software has produced an optimal alignment, it is possible to calculate percent sequence identity. The software typically does this as part of the sequence comparison and generates a numerical result. The percent sequence identity may be calculated as the number of identical residues as a percentage of the total residues in the SEQ ID NO referred to.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants, derivatives, analogues and fragments may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded polypeptide. These methods are only illustrative of the numerous standard techniques known in the art for manipulation of DNA sequences and other known techniques may also be used.

### Expression vectors, kits and systems

In one aspect, the present invention provides a vector encoding the polynucleotide, vector, or retroviral vector of the present invention. In another aspect, the present invention provides a vector encoding the virus genome (e.g. retrovirus genome) of the present invention.

The vector may be a transfer vector. For example, the vector may be a plasmid and/or the retroviral genome may be operably linked to a promoter (e.g. a viral promoter, such as a CMV promoter).

In another aspect, the present invention provides a kit or system for producing the viral vector (e.g. retroviral vector) of the present invention.

The kit or system may be a virus packaging kit or system or a virus production kit or system. As used herein, a "virus packaging kit or system" may comprise one or more components, and optionally instructions, for packaging the viral vector of the present invention. As used herein, a "virus production kit or system" may comprise one or more components, and optionally instructions, for producing the viral vector of the present invention.

The kit or system may comprise a transfer vector encoding the virus (e.g. retrovirus genome) genome of the present invention and optionally one or more helper vectors. The kit or system may further comprise host cells (e.g. packaging cells or producer cells) and/or other reagents (e.g. transfection reagent, culture medium, etc.). The kit or system may further comprise any other suitable components, and optionally instructions for packaging and/or producing the viral vector (e.g. retroviral vector) of the present invention.

In another aspect, the present invention provides a gene-editing kit, composition, or system comprising the polynucleotide, vector, or retroviral vector of the present invention.

As used herein, a "gene-editing kit, composition, or system" is a kit, composition, or system which comprises all components necessary to edit a genome using the polynucleotide, vector, or retroviral vector of the invention. In some embodiments, the kit, composition, or gene-editing system comprises the polynucleotide, vector, or retroviral vector of the invention and a guide RNA. The kit, composition, or gene-editing system may further comprise an RNA-guided nuclease. The RNA-guided nuclease may be in a complex with the guide RNA, i.e. the guide RNA and the RNA-guided nuclease together form a ribonucleoprotein (RNP).

### Cells

In one aspect, the present invention provides a cell comprising the polynucleotide, vector, viral particle, or retroviral vector of the present invention. The cell may be an isolated cell.

Suitably, the cell is a mammalian cell, for example a human cell. The cell may be an isolated human cell.

In some embodiments, the cell genome comprises the polynucleotide of the present invention. In some embodiments, the vector or retroviral vector of the present invention is integrated into the cell genome. For example, a retroviral vector of the present invention may be reverse-transcribed into proviral DNA and subsequently the proviral DNA integrated into the cell genome.

In another aspect, the present invention provides a population or cells comprising the cell of the present invention.

Suitably, the cell may be a producer cell. The term "producer cell" includes a cell that produces viral particles, after transient transfection, stable transfection or vector transduction of all the elements necessary to produce the viral particles or any cell engineered to stably comprise the elements necessary to produce the viral particles. Suitable producer cells will be well known to those of skill in the art and may include HEK293, COS-1, COS-7, CV-1, HeLa, CHO, and A549 cell lines. In some embodiments, the producer cell is a HEK293 cell, or a derivative thereof (e.g. a HEK293T cell, a HEK293T Lenti-X, a HEK293T-Rex cell, a HEK293FT cell, a HEK293SF-3F6 cell, a HEK293SF-3F9 cell, a HEK293-EBNA1 cell, or a SJ293TS cell).

Suitably, the cell may be a packaging cell. The term "packaging cell" includes a cell which contains some or all of the elements necessary for packaging a recombinant virus genome. Typically, such packaging cells contain one or more vectors which are capable of expressing viral structural proteins (e.g. *gag-pol, rev, env*) and/or one or more genes encoding the viral structural proteins have been integrated into the genome of the packaging cell. Cells comprising only some of the elements required for the production of enveloped viral particles are useful as intermediate reagents in the generation of viral particle producer cell lines, through subsequent steps of transient transfection, transduction or stable integration of each additional required element. These intermediate reagents are encompassed by the term "packaging cell". Suitable packaging cells will be well known to those of skill in the art (see e.g. Merten, O.W., et al., 2016. Molecular Therapy-Methods & Clinical Development, 3, p.16017).

Suitably, the cell may be a hematopoietic stem cell (HSC), a hematopoietic progenitor cell (HPC), or a lymphoid progenitor cell (LPC). In some embodiments, the cell is a HSC or a HPC, optionally the cell is a HSC. As used herein "hematopoietic stem cells" are stem cells that have no differentiation potential to cells other than hematopoietic cells, "hematopoietic progenitor cells" are progenitor cells that have no differentiation potential to cells other than hematopoietic cells, and "lymphoid progenitor cells" are progenitor cells that have no differentiation potential to cells other than lymphocytes.

Suitably, the cell may be a non-stem cell, for example any non-stem cell suitable for cell therapy. Suitable non-stem cells include fibroblasts, chondrocytes, keratinocytes, hepatocytes, pancreatic islet cells, and immune cells, such as T cells, dendritic cells (DCs), natural killer (NK) cells, and macrophages (see e.g. El-Kadiry, et al., 2021. Frontiers in Medicine, 8, p.756029). In some embodiments, the cell is a liver cell. In some embodiments, the cell is a T cell.

The cell can be obtained from any source. The cell may be autologous or allogeneic. The cell may be obtained or obtainable from any biological sample, such as peripheral blood or cord blood. Peripheral blood may be treated with mobilising agent, i.e. may be mobilised peripheral blood. The cell may be a universal cell.

The cell may be isolated or isolatable using commercially available antibodies that bind to cell surface antigens, e.g. CD34, using methods known to those of skill in the art. For example, the antibodies may be conjugated to magnetic beads and immunological procedures utilized to recover the desired cell type. Suitably, the cell is identified by the presence or absence of one or more antigenic markers. Suitable antigenic markers include CD34, CD133, CD90, CD45, CD4, CD19, CD13, CD3, CD56, CD14, CD61/41, CD135, CD45RA, CD33, CD66b, CD38, CD45, CD10, CD11c, CD19, CD7, and CD71.

Suitably, the cell is identified by the presence of the antigenic marker CD34 (CD34+), i.e. the cell is a CD34+ cell. For example, the cell may be a cord blood CD34+ cell or a (mobilised) peripheral blood CD34+ cell. The cell may be a CD34+ HSC, a CD34+ HPC, or a CD34+ LPC, optionally the cell is a CD34+ HSC.

In some embodiments, the cell is identified by the presence of CD34 and the presence or absence or one or more further antigenic markers. The further antigenic markers may be selected from one or more of CD133, CD90, CD3, CD56, CD14, CD61/41, CD135, CD45RA, CD33, CD66b, CD38, CD45, CD10, CD11c, CD19, CD7, and CD71. For example, the cell may be a CD34+CD133+CD90+ cell, a CD34+CD133+CD90- cell, or a CD34+CD133-CD90- cell.

### Pharmaceutical compositions

In one aspect, the present invention provides pharmaceutical composition comprising the polynucleotide, vector, viral particle, retroviral vector or cell of the present invention. In preferred embodiments, the pharmaceutical composition comprises the retroviral vector of the present invention in the form of a retroviral particle.

A pharmaceutical composition is a composition that comprises or consists of a therapeutically effective amount of a pharmaceutically active agent e.g. the retroviral vector. A pharmaceutical composition preferably includes a pharmaceutically acceptable carrier, diluent or excipient (including combinations thereof).

By "pharmaceutically acceptable" is included that the formulation is sterile and pyrogen free. The carrier, diluent, and/or excipient must be "acceptable" in the sense of being compatible with the retroviral vector and not deleterious to the recipients thereof. Typically, the carriers, diluents, and excipients will be saline or infusion media which will be sterile and pyrogen free, however, other acceptable carriers, diluents, and excipients may be used.

Acceptable carriers, diluents, and excipients for therapeutic use are well known in the pharmaceutical art. The choice of pharmaceutical carrier, excipient or diluent can be selected with regard to the intended route of administration and standard pharmaceutical practice. The pharmaceutical compositions may comprise as (or in addition to) the carrier, excipient or diluent any suitable binder(s), lubricant(s), suspending agent(s), coating agent(s) or solubilising agent(s).

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

The pharmaceutical compositions may comprise polynucleotides, vectors, viral particles, retroviral vectors or cells of the invention in infusion media, for example sterile isotonic solution. The pharmaceutical composition may be enclosed in ampoules, disposable syringes or multiple dose vials made of glass or plastic.

The invention further includes kits comprising the polynucleotide, vector, viral particle, retroviral vector, cell and/or pharmaceutical composition of the present invention. Preferably said kits comprise instructions for use of the kit components as a medicament.

### Medicaments

In one aspect, the present invention provides the polynucleotide, vector, viral particle, retroviral vector, cell, or pharmaceutical composition according to the present invention, for use as a medicament.

In another aspect, the present invention provides use of the polynucleotide, vector, viral particle, retroviral vector, cell, or pharmaceutical composition according to the present invention in the manufacture of a medicament.

In one aspect, the present invention provides a method of administering a therapeutically effective amount of the polynucleotide, vector, viral particle, retroviral vector, cell, or pharmaceutical composition according to the present invention to a subject in need thereof.

The polynucleotide, vector, viral particle, retroviral vector, cell or pharmaceutical composition may be administered to any subject in need thereof. The subject may be a mammal (e.g. a human). In some embodiments, the retroviral vector is administered in the form of a retroviral particle.

The polynucleotide, vector, viral particle, retroviral vector, cell, or pharmaceutical composition according to the present invention may be administered in a manner appropriate for treating and/or preventing disease in the subject. The quantity and frequency of administration may be determined by the skilled person, for example depending by such factors as the condition of the subject, and the type and severity of the subject's disease.

The polynucleotide, vector, viral particle, retroviral vector, cell or pharmaceutical composition according to the present invention may be administered parenterally, (e.g. intravenous, intra-arterial, intramuscular, intrathecal, subcutaneous), or by infusion techniques. The polynucleotide, vector, viral particle, retroviral vector, cell or pharmaceutical composition may be administered in the form of a sterile aqueous solution which may contain other substances, for example, enough salts or glucose to make the solution isotonic with blood. The aqueous solution may be suitably buffered (preferably to a pH of from 3 to 9). The preparation of suitable parenteral formulations under sterile conditions is readily accomplished by standard pharmaceutical techniques well-known to those skilled in the art.

The polynucleotide, vector, viral particle, retroviral vector, cell or pharmaceutical composition according to the present invention may be administered systemically, for example by intravenous injection or intraperitoneal injection. In some embodiments, the polynucleotide, vector, viral particle, retroviral vector, cell or pharmaceutical composition according to the present invention is administered by intravenous injection. The polynucleotide, vector, viral particle, retroviral vector, cell or pharmaceutical composition according to the present invention may be administered locally, for example by direct injection, intra-arterial injection, or intraportal injection.

The polynucleotide, vector, viral particle, retroviral vector, cell or pharmaceutical composition may be administered in a single or in multiple doses. Suitably, the polynucleotide, vector, viral particle, retroviral vector, cell or pharmaceutical composition may be administered in a single, one off dose.

The polynucleotide, vector, viral particle, retroviral vector, cell or pharmaceutical composition may be administered at varying doses (e.g. measured in Transducing Units (TU) per kg). The physician in any event may determine the actual dosage which will be most suitable for any individual subject and the dosage may, for example, vary with the age, weight and response of the particular subject.

Suitably, the retroviral vector of the present invention is administered at a dose of at least about 10⁸ TU/kg, at least about 10⁹ TU/kg, or at least about 10¹⁰ TU/kg. Suitably, the retroviral vector of the present invention is administered at a dose of about 10¹³ TU/kg or less, about 10¹² TU/kg or less, or about 10¹¹ TU/kg or less. Suitably, the retroviral vector of the present invention is administered in a dose of from about 10⁸ to about 10¹³ TU/kg, from about 10⁹ to about 10¹³ TU/kg, or from about 10¹⁰ to about 10¹³ TU/kg. Suitably, the retroviral vector of the present invention is administered in a dose of from about 10⁸ to about 10¹² TU/kg, from about 10⁹ to about 10¹² TU/kg, or from about 10¹⁰ to about 10¹² TU/kg. Suitably, the retroviral vector of the present invention is administered in a dose of from about 10⁸ to about 10¹¹ TU/kg, from about 10⁹ to about 10¹¹ TU/kg, or from about 10¹⁰ to about 10¹¹ TU/kg. In some embodiments, the retroviral vector of the present invention is administered in a dose of from about 10⁸ to about 10¹¹ TU/kg, from about 10⁸ to about 10¹⁰ TU/kg, or from about 10⁹ to about 10¹⁰ TU/kg. In some embodiments, the retroviral vector of the present invention is administered in a dose of from about 10⁹ to about 10¹⁰ TU/kg.

### Variant A2 insulator elements

In one aspect, the present invention provides a variant A2 insulator element.

### Example forward variant A2 insulator elements

An example forward variant A2 insulator element is provided below as SEQ ID NO: 102. 
ACAATGGCTGGCCCATAGTAAATGCCGTGTTAGTGTGTTAGTTGCTGTTCTTCCACGTCAGA
AGAGGCACAGACAAATTACCACCAGGTGGCGCTCAGAGTCTGCGGAGGCATCACAACAGCCC
TGAATTTGAATCCTGCTCTGCCACTGCCTAGTTGAGACCTTTTACTACCTGACTAGCTGAGA
 
CATTTACGACATTTACTGGCTCTAGGACTCATTTGCTTCATTTCATTACTTTTTTTTTCTTT
GAGACGGAATCTCGCTCT
 Example variant A2 insulator sequence (forward) (SEQ ID NO: 102)

In some embodiments, the insulator element comprises or consists of a sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 102, wherein the insulator element comprises the nucleotides G and C at positions corresponding to nucleotides 221 and 222, respectively, wherein the nucleotides are numbered with reference to SEQ ID NO: 102.

In some embodiments, the insulator element consists of a sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 102, wherein the insulator element comprises the nucleotides G and C at positions corresponding to nucleotides 221 and 222, respectively, wherein the nucleotides are numbered with reference to SEQ ID NO: 102.

In preferred embodiments, the insulator element comprises a core A2 insulator sequence. In some embodiments, the insulator element comprises SEQ ID NO: 30.

In some embodiments, the insulator element comprises or consists of SEQ ID NO: 102.

In some embodiments, the insulator element consists of SEQ ID NO: 102.

### Example reverse variant A2 insulator elements

An example reverse variant A2 insulator element is provided below as SEQ ID NO: 103. 
AGAGCGAGATTCCGTCTCAAAGAAAAAAAAAGTAATGAAATGAAGCAAATGAGTCCTAGAGC
CAGTAAATGTCGTAAATGTCTCAGCTAGTCAGGTAGTAAAAGGTCTCAACTAGGCAGTGGCA
GAGCAGGATTCAAATTCAGGGCTGTTGTGATGCCTCCGCAGACTCTGAGCGCCACCTGGTGG
TAATTTGTCTGTGCCTCTTCTGACGTGGAAGAACAGCAACTAACACACTAACACGGCATTTA
CTATGGGCCAGCCATTGT
 Example variant A2 insulator sequence (reverse) (SEQ ID NO: 103)

In some embodiments, the insulator element comprises or consists of a sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 103, wherein the insulator element comprises the nucleotides G and C at positions corresponding to nucleotides 45 and 46, respectively, wherein the nucleotides are numbered with reference to SEQ ID NO: 103.

In some embodiments, the insulator element consists of a sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 103, wherein the insulator element comprises the nucleotides G and C at positions corresponding to nucleotides 45 and 46, respectively, wherein the nucleotides are numbered with reference to SEQ ID NO: 103.

In preferred embodiments, the insulator element comprises a core A2 insulator sequence. In some embodiments, the insulator element comprises SEQ ID NO: 34.

In some embodiments, the insulator element comprises or consists of SEQ ID NO: 103.

In some embodiments, the insulator element consists of SEQ ID NO: 103.

### Polynucleotides, vectors, cells, and pharmaceutical compositions

In one aspect, the present invention provides a polynucleotide comprising the variant A2 insulator element. The polynucleotide may be an isolated polynucleotide. The polynucleotide may be double or single stranded. The polynucleotide may comprise or consist of DNA and/or RNA. In some embodiments, the polynucleotide is DNA. In some embodiments, the polynucleotide is RNA. The polynucleotide may be any described herein in the section entitled "Polynucleotides".

In some embodiments, the polynucleotide further comprises an expression cassette and the insulator element flanks the expression cassette. The expression cassette may be any described herein in the section entitled "Expression cassettes".

In one aspect, the present invention provides a vector comprising the variant A2 insulator element or a polynucleotide comprising the variant A2 insulator element. The vector may be any described herein in the section entitled "Vectors".

In one aspect, the present invention provides a cell comprising a polynucleotide comprising the variant A2 insulator element, or a vector comprising the variant A2 insulator element or polynucleotide. The cell may be any described herein in the section entitled "Cells".

In one aspect, the present invention provides a pharmaceutical composition comprising a polynucleotide comprising the variant A2 insulator element, a vector comprising the variant A2 insulator element or polynucleotide, or a cell comprising the polynucleotide or vector. The pharmaceutical composition may be any described herein in the section entitled "Pharmaceutical compositions".

### 3' LTRs

In one aspect, the present invention provides a 3' LTR comprising one or more antisense-oriented miRNA target sequences and one or more insulator elements. The 3' LTR may be a self-inactivating (SIN) 3' LTR.

In some embodiments, the 3' LTR comprises from 5' to 3' direction: a first insulator element; one or more antisense-oriented miRNA target sequences; and a second insulator element. In some embodiments, the first insulator element is in the reverse orientation and/or the second insulator element is in the forward orientation. In some embodiments, the first insulator element is in the reverse orientation and the second insulator element is in the forward orientation.

In some embodiments, the first insulator element is not present, i.e., the 3' LTR comprises from 5' to 3' direction: one or more antisense-oriented miRNA target sequences; and the second insulator element.

In other embodiments, the second insulator element is not present, i.e. the 3' LTR comprises from 5' to 3' direction: the first insulator element; and one or more antisense-oriented miRNA target sequences.

The first insulator element and second insulator may each comprise multiple insulator sequences. In some embodiments, the first insulator element and second insulator element each comprise one or more full-length insulator sequence. In some embodiments, the first insulator element and second insulator element each comprise one or more core insulator sequence.

### Antisense-oriented miRNA target sequences

The one or more antisense-oriented miRNA target sequences may be any described above in the section entitled "microRNA (miRNA) target sequences"
In some embodiments, the 3' LTR comprises one or more antisense-oriented miR223 target sequences. In some embodiments, the 3' LTR comprises one or more antisense-oriented miR223-3p target sequences.

In some embodiments, the 3' LTR comprises one or more antisense-oriented miRNA target sequences comprising or consisting of the nucleotide sequence of SEQ ID NO: 12, or a variant thereof having one or two nucleotide variations. In some embodiments, the 3' LTR comprises one or more antisense-oriented miRNA target sequences comprising or consisting of the nucleotide sequence of SEQ ID NO: 12.

In some embodiments, the 3' LTR comprises two antisense-oriented miR223 target sequences. In some embodiments, the 3' LTR comprises two antisense-oriented miR223-3p target sequences.

In some embodiments, the 3' LTR comprises one or more antisense-oriented miRNA target sequences comprising or consisting of the nucleotide sequence of SEQ ID NO: 22, or a variant thereof having one to four nucleotide variations. In some embodiments, the 3' LTR comprises one or more antisense-oriented miRNA target sequences comprising or consisting of the nucleotide sequence of SEQ ID NO: 22, or a variant thereof having one or two nucleotide variations. In some embodiments, the 3' LTR comprises one or more antisense-oriented miRNA target sequences comprising or consisting of the nucleotide sequence of SEQ ID NO: 22.

### Insulator elements

The one or more insulator elements may be any described above in the section entitled "Insulator elements" or "Variant A2 insulator elements".

In some embodiments, the 3' LTR comprises an A1 insulator element, an A2 insulator element, a B2 insulator element, and/or an E2 insulator element. In some embodiments, the insulator elements are full-length insulator sequences. In some embodiments, the insulator elements are core insulator sequences.

In some embodiments, the 3' LTR comprises a CTCF-binding motif comprising or consisting of a nucleotide sequence selected from the group consisting of SEQ ID NOs: 23 to 28. In some embodiments, the 3' LTR comprises a CTCF-binding motif comprising or consisting of a nucleotide sequence selected from the group consisting of SEQ ID NOs: 23, 24, and 27. In some embodiments, the 3' LTR comprises a CTCF-binding motif comprising or consisting of the nucleotide sequence of SEQ ID NO: 23. In some embodiments, the 3' LTR comprises a CTCF-binding motif comprising or consisting of the nucleotide sequence of SEQ ID NO: 24. In some embodiments, the 3' LTR comprises a CTCF-binding motif comprising or consisting of the nucleotide sequence of SEQ ID NO: 27.

In some embodiments, the 3' LTR comprises an insulator element comprising or consisting of a nucleotide sequence selected from the group consisting of SEQ ID NOs: 29 to 36, or variants thereof having at least 80.0%, at least 82.5%, at least 85.0%, at least 87.5%, at least 90.0%, at least 92.5%, at least 95.0%, or at least 97.5% sequence identity thereto. Suitably, the variants comprise a CTCF-binding motif and one or more variation is made in the flanking sequences. In some embodiments, the 3' LTR comprises an insulator element comprising or consisting of a nucleotide sequence selected from the group consisting of SEQ ID NOs: 29 to 36.

In some embodiments, the 3' LTR comprises an insulator element comprising or consisting of a nucleotide sequence selected from the group consisting of SEQ ID NOs: 37, 38, 43, 55, 64, 65, 70, 82, or variants thereof having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity thereto. Suitably, the variants comprise a CTCF-binding motif and one or more variation is made in the flanking sequences.

In some embodiments, the 3' LTR comprises an insulator element comprising or consisting of a nucleotide sequence selected from the group consisting of SEQ ID NOs: 37, 38, 43, 55, 64, 65, 70, or 82.

In some embodiments, the 3' LTR comprises an A2 insulator element and an E2 insulator element. In some embodiments, the A2 insulator element and the E2 insulator element are full-length insulator sequences. In some embodiments, the A2 insulator element and the E2 insulator element are core insulator sequences. In some embodiments, the A2 insulator element and the E2 insulator element are in divergent orientation. In some embodiments, the A2 insulator element is in a reverse orientation and the E2 insulator element is in a forward orientation.

In some embodiments, the 3' LTR comprises an E2 insulator element. In some embodiments, the E2 insulator element is a full-length insulator sequence. In some embodiments, the E2 insulator element is in a forward orientation.

In some embodiments, the 3' LTR comprises an A1 insulator element and an A2 insulator element. In some embodiments, the A1 insulator element and the A2 insulator element are full-length insulator sequences. In some embodiments, the A1 insulator element and the A2 insulator element are core insulator sequences. In some embodiments, the A1 insulator element and the A2 insulator element are in parallel orientation. In some embodiments, the A1 insulator element is in a reverse orientation and the A2 insulator element is in a reverse orientation.

In some embodiments, the 3' LTR comprises an A2 insulator element. In some embodiments, the A2 insulator element is a full-length insulator sequence. In some embodiments, the A2 insulator element is a core insulator sequence. In some embodiments, the A2 insulator element is in a reverse orientation.

In some embodiments, the 3' LTR comprises an A1 insulator element. In some embodiments, the A1 insulator element is a core insulator sequence. In some embodiments, the A1 insulator element is in a reverse orientation.

In some embodiments, the 3' LTR comprises a B2 insulator element. In some embodiments, the B2 insulator element is a core insulator sequence. In some embodiments, the B2 insulator element is in a reverse orientation.

### Example 3' LTRs

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to a nucleotide sequence selected from the group consisting of SEQ ID NOs: 104 to 112. In some embodiments, the 3' LTR comprises or consists of a nucleotide sequence selected from the group consisting of SEQ ID NOs: 104 to 112.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 104. In some embodiments, the 3' LTR comprises or consists of the nucleotide sequence of SEQ ID NO: 104.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 105. In some embodiments, the 3' LTR comprises or consists of the nucleotide sequence of SEQ ID NO: 105.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 106. In some embodiments, the 3' LTR comprises or consists of the nucleotide sequence of SEQ ID NO: 106.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 107. In some embodiments, the 3' LTR comprises or consists of the nucleotide sequence of SEQ ID NO: 107.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 108. In some embodiments, the 3' LTR comprises or consists of the nucleotide sequence of SEQ ID NO: 108.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 109. In some embodiments, the 3' LTR comprises or consists of the nucleotide sequence of SEQ ID NO: 109.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 110. In some embodiments, the 3' LTR comprises or consists of the nucleotide sequence of SEQ ID NO: 110.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 111. In some embodiments, the 3' LTR comprises or consists of the nucleotide sequence of SEQ ID NO: 111.

In one aspect, the present invention provides a 3' LTR comprising or consisting of a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide of SEQ ID NOs: 112. In some embodiments, the 3' LTR comprises or consists of the nucleotide sequence of SEQ ID NO: 112. 
TGGAAGGGCTAATTCACTCCCAACGAAGACAAGAGCGAGATTCCGTCTCAAAGAAAAAAA
AGTAATGAAATGAAGCAAATGAGTCCTAGAGCCAGTAAATGTCGTAAATGTCTCAGCTAGTC
AGGTAGTAAAAGGTCTCAACTAGGCAGTGGCAGAGCAGGATTCAAATTCAGGGCTGTTGTGA
TGCCTCCGCAGACTCTGAGCGCCACCTGGTGGTAATTTGTCTGTGCCTCTTCTGACGTGGAA
GAACAGCAACTAACACACTAACACGGCATTTACTATGGGCCAGCCATTGTATGCATTGTCAG
TTTGTCAAATACCCCATCGTGTCAGTTTGTCAAATACCCCACCGCGGTTTGATTTCCTTCAC
TCTGGAATATACTACAGAATGTACTAATATAAATGTGCTTGCCTATGTCTAATCTAGAATAT
AATTATCCTGAACTCTACATGGTATTTTAGTCTAGAAAGTACTGTAGCTTCTCTGAGTTCTA
AATCAAACCAGGAAACAGCAGAGGGCGCTGGTGCTGTGCTTTTGCAAGTGAGATGTGTGCTC
ATTTCAAGGGTCCACAAAATTTTCTGGCATGCTGTTTCCATGGAGAGGAAAGTAGGTGTTTT
CTTTTTCTTTTTTTTTTTCAGTCAAGCCACCTGCAAACTGATCTGCTTTTTGCTTGTACTGG
GTCTCTCTGGTTAGACCAGATCTGAGCCTGGGAGCTCTCTGGCTAACTAGGGAACCCACTGC
TTAAGCCTCAATAAAGCTTGCCTTGAGTGCTTCAAGTAGTGTGTGCCCGTCTGTTGTGTGAC
TCTGGTAACTAGAGATCCCTCAGACCCTTTTAGTCAGTGTGGAAAATCTCTAGCA
 Example A2(-)-miRT(-)-E2(+) 3'LTR (SEQ ID NO: 104) 
TGGAAGGGCTAATTCACTCCCAACGAAGACAACGCAGACTCTGAGCGCCACCTGGTGGTAAT
TTGTCTGTGCATGCATTGTCAGTTTGTCAAATACCCCATCGTGTCAGTTTGTCAAATACCCC
ACCGCGGATCAAACCAGGAAACAGCAGAGGGCGCTGGTGCTGTGCTGATCTGCTTTTTGCTT
GTACTGGGTCTCTCTGGTTAGACCAGATCTGAGCCTGGGAGCTCTCTGGCTAACTAGGGAAC
CCACTGCTTAAGCCTCAATAAAGCTTGCCTTGAGTGCTTCAAGTAGTGTGTGCCCGTCTGTT
GTGTGACTCTGGTAACTAGAGATCCCTCAGACCCTTTTAGTCAGTGTGGAAAATCTCTAGCA
 Example A2_core(-)-miRT(-)-E2_core(+) 3'LTR (SEQ ID NO: 105) 
TGGAAGGGCTAATTCACTCCCAACGAAGACAAGATCTGCTTTTTATGCATTGTCAGTTTGTC
AAATACCCCATCGTGTCAGTTTGTCAAATACCCCACCGCGGTTTGATTTCCTTCACTCTGGA
ATATACTACAGAATGTACTAATATAAATGTGCTTGCCTATGTCTAATCTAGAATATAATTAT
CCTGAACTCTACATGGTATTTTAGTCTAGAAAGTACTGTAGCTTCTCTGAGTTCTAAATCAA
ACCAGGAAACAGCAGAGGGCGCTGGTGCTGTGCTTTTGCAAGTGAGATGTGTGCTCATTTCA
AGGGTCCACAAAATTTTCTGGCATGCTGTTTCCATGGAGAGGAAAGTAGGTGTTTTCTTTTT
CTTTTTTTTTTTCAGTCAAGCCACCTGCAAACTGAAGACAAGATCTGCTTTTTGCTTGTACT
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCA
 Example miRT(-)-E2(+) 3'LTR (SEQ ID NO: 106) 
TGGAAGGGCTAATTCACTCCCAACGAAGACAAATGACGAGAGACAGAAGAATTCTTCAAG
GTTAGTGTGTCCAGCATGCAACCTTTCCTTCCTGGATGAGCATCCCTGGAATAGGAGAGCCA
GCCTGCCTCCTGCGCTGGCACAGAGCCCGGTTCCCTAGACAACTGCCTCTCCAAATCTGATG
TCCAGCGCCACCTGGTGTCCACATCAAGCAGACACAATTAATAGTCAACCTGTTCAGGAAAA
CTGTGAGGGGGAAAAAAAAGAAAGAGGATTTATGAAGGGAAAAGAAAGTTTAGAGGATATGC
CACGATAGAGCGAGATTCCGTCTCAAAGAAAAAAAAAGTAATGAAATGAAGCAAATGAGTCC
TAGAGCCAGTAAATGTCGTAAATGTCTCAGCTAGTCAGGTAGTAAAAGGTCTCAACTAGGCA
GTGGCAGAGCAGGATTCAAATTCAGGGCTGTTGTGATGCCTCCGCAGACTCTGAGCGCCACC
TGGTGGTAATTTGTCTGTGCCTCTTCTGACGTGGAAGAACAGCAACTAACACACTAACACGG
 
CATTTACTATGGCCAGCCATTGTATGCATTGTCAGTTTGTCAAATACCCCATCGTGTCAGT
TTGTCAAATACCCCAGATCTGCTTTTTGCTTGTACTGGGTCTCTCTGGTTAGACCAGATCTG
AGCCTGGGAGCTCTCTGGCTAACTAGGGAACCCACTGCTTAAGCCTCAATAAAGCTTGCCTT
GAGTGCTTCAAGTAGTGTGTGCCCGTCTGTTGTGTGACTCTGGTAACTAGAGATCCCTCAGA
CCCTTTTAGTCAGTGTGGAAAATCTCTAGCA
 Example A1(-)-A2(-)-miRT(-) 3'LTR (SEQ ID NO: 107) 
TGGAAGGGCTAATTCACTCCCAACGAAGACAAATCTGATGTCCAGCGCCACCTGGTGTCCAC
ATCAAGCAGACGCAGACTCTGAGCGCCACCTGGTGGTAATTTGTCTGTGCTTAATTAAATGC
ATTGTCAGTTTGTCAAATACCCCATCGTGTCAGTTTGTCAAATACCCCAGATCTGCTTTTTG
CTTGTACTGGGTCTCTCTGGTTAGACCAGATCTGAGCCTGGGAGCTCTCTGGCTAACTAGGG
AACCCACTGCTTAAGCCTCAATAAAGCTTGCCTTGAGTGCTTCAAGTAGTGTGTGCCCGTCT
GTTGTGTGACTCTGGTAACTAGAGATCCCTCAGACCCTTTTAGTCAGTGTGGAAAATCTCTA
GCA
 Example A1_core(-)-A2_core(-)-miRT(-) 3'LTR (SEQ ID NO: 108) 
TGGAAGGGCTAATTCACTCCCAACGAAGACAAGAGCGAGATTCCGTCTCAAAGAAAAAAA
AGTAATGAAATGAAGCAAATGAGTCCTAGAGCCAGTAAATGTCGTAAATGTCTCAGCTAGTC
AGGTAGTAAAAGGTCTCAACTAGGCAGTGGCAGAGCAGGATTCAAATTCAGGGCTGTTGTGA
TGCCTCCGCAGACTCTGAGCGCCACCTGGTGGTAATTTGTCTGTGCCTCTTCTGACGTGGAA
GAACAGCAACTAACACACTAACACGGCATTTACTATGGGCCAGCCATTGTATGCATTGTCAG
TTTGTCAAATACCCCATCGTGTCAGTTTGTCAAATACCCCAGATCTGCTTTTTGCTTGTACT
GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCA
 Example A2(-)-miRT(-) 3'LTR (SEQ ID NO: 109) 
TGGAAGGGCTAATTCACTCCCAACGAAGACAACGCAGACTCTGAGCGCCACCTGGTGGTAAT
TTGTCTGTGCATGCATTGTCAGTTTGTCAAATACCCCATCGTGTCAGTTTGTCAAATACCCC
AGATCTGCTTTTTGCTTGTACTGGGTCTCTCTGGTTAGACCAGATCTGAGCCTGGGAGCTCT
CTGGCTAACTAGGGAACCCACTGCTTAAGCCTCAATAAAGCTTGCCTTGAGTGCTTCAAGTA
GTGTGTGCCCGTCTGTTGTGTGACTCTGGTAACTAGAGATCCCTCAGACCCTTTTAGTCAGT
GTGGAAAATCTCTAGCA
 Example A2_core(-)-miRT(-) 3'LTR (SEQ ID NO: 110) 
TGGAAGGGCTAATTCACTCCCAACGAAGACAAATCTGATGTCCAGCGCCACCTGGTGTCCAC
ATCAAGCAGAATGCATTGTCAGTTTGTCAAATACCCCATCGTGTCAGTTTGTCAAATACCCC
AGATCTGCTTTTTGCTTGTACTGGGTCTCTCTGGTTAGACCAGATCTGAGCCTGGGAGCTCT
CTGGCTAACTAGGGAACCCACTGCTTAAGCCTCAATAAAGCTTGCCTTGAGTGCTTCAAGTA
GTGTGTGCCCGTCTGTTGTGTGACTCTGGTAACTAGAGATCCCTCAGACCCTTTTAGTCAGT
GTGGAAAATCTCTAGCA
 Example Al_core(-)-miRT(-) 3'LTR (SEQ ID NO: 111) 
TGGAAGGGCTAATTCACTCCCAACGAAGACAACTGCGTCCCAGCAGCTCCCCCTGGTGGCCA
GAAGCAAACGCTATGCATTGTCAGTTTGTCAAATACCCCATCGTGTCAGTTTGTCAAATACC
CCAGATCTGCTTTTTGCTTGTACTGGGTCTCTCTGGTTAGACCAGATCTGAGCCTGGGAGCT
CTCTGGCTAACTAGGGAACCCACTGCTTAAGCCTCAATAAAGCTTGCCTTGAGTGCTTCAAG
TAGTGTGTGCCCGTCTGTTGTGTGACTCTGGTAACTAGAGATCCCTCAGACCCTTTTAGTCA
GTGTGGAAAATCTCTAGCA
 Example B2_core(-)-miRT(-) 3'LTR (SEQ ID NO: 112)

### Polynucleotides, vectors, cells, and pharmaceutical compositions

In one aspect, the present invention provides a polynucleotide comprising the 3' LTR of the present invention. The polynucleotide may be an isolated polynucleotide. The polynucleotide may be double or single stranded. The polynucleotide may comprise or consist of DNA and/or RNA. In some embodiments, the polynucleotide is DNA. In some embodiments, the polynucleotide is RNA. The polynucleotide may be any described herein in the section entitled "Polynucleotides".

In some embodiments, the polynucleotide further comprises a sense-oriented expression cassette and the 3' LTR of the present invention flanks the expression cassette (e.g. the 3' LTR is downstream from the expression cassette). The expression cassette may be any described herein in the section entitled "Expression cassettes". In some embodiments, the expression cassette consists of from 5' to 3' direction: a promoter or enhancer-promoter, an open reading frame, and a 3' regulatory region.

In some embodiments, the polynucleotide further comprises one or more sense-oriented miRNA target sequences, upstream from the sense-oriented expression cassette. The one or more sense-oriented miRNA target sequences may be any described herein in the section entitled "microRNA (miRNA) target sequences". In some embodiments, the sense-oriented miRNA target sequences are miR142 target sequences. In some embodiments, the sense-oriented miRNA target sequences are miR142-3p target sequences. In some embodiments, the sense-oriented miRNA target sequences comprise or consist of the nucleotide sequence of SEQ ID NO: 1, or a variant thereof having one or two nucleotide variations. In some embodiments, the sense-oriented miRNA target sequences comprise or consist of the nucleotide sequence of SEQ ID NO: 1. In some embodiments, the sense-oriented miRNA target sequences comprise or consist of the nucleotide sequence of SEQ ID NO: 21, or a variant thereof having one to four nucleotide variations. In some embodiments, the sense-oriented miRNA target sequences comprise or consist of the nucleotide sequence of SEQ ID NO: 21, or a variant thereof having one or two nucleotide variations. In some embodiments, the sense-oriented miRNA target sequences comprise or consist of the nucleotide sequence of SEQ ID NO: 21.

In some embodiments, the polynucleotide further comprises a 5'LTR. The 5'LTR may be any described herein in the section entitled "5' LTR". In some embodiments, the 5'LTR comprises or consists of a nucleotide sequence which is at least 70%, at least 75%, at least 80%, at least 85%, at least 90%, at least 95%, at least 96%, at least 97%, at least 98%, or at least 99% identical to SEQ ID NO: 92. In some embodiments, the 5'LTR comprises or consists of the nucleotide sequence SEQ ID NO: 92.

In some embodiments, the polynucleotide further comprises one or more lentiviral-derived cis-acting elements. The lentiviral-derived cis-acting elements may be any described herein in the section entitled "Retroviral vectors". Suitably, the lentiviral-derived cis-acting elements may comprise or consist of from 5' to 3' direction: a PBS, a retroviral psi packaging element, a RRE, and a cPPT.

In some embodiments, the polynucleotide comprises from 5' to 3' direction: a 5'LTR; one or more lentiviral-derived cis-acting elements; one or more sense-oriented miRNA target sequences; a sense-oriented expression cassette; and the 3' LTR of the present invention.

In one aspect, the present invention provides a vector comprising the 3' LTR of the present invention or a polynucleotide comprising the 3' LTR of the present invention. The vector may be any described herein in the section entitled "Vectors". In some embodiments, the vector is a retroviral vector. In some embodiments, the vector is a lentiviral vector. In some embodiments, the vector is a plasmid.

In one aspect, the present invention provides a cell comprising a polynucleotide comprising the 3' LTR of the present invention, or a vector comprising the 3' LTR of the present invention or polynucleotide. The cell may be any described herein in the section entitled "Cells".

In one aspect, the present invention provides a pharmaceutical composition comprising a polynucleotide comprising the 3' LTR of the present invention, a vector comprising the 3' LTR of the present invention or polynucleotide, or a cell comprising the polynucleotide or vector. The pharmaceutical composition may be any described herein in the section entitled "Pharmaceutical compositions".

The practice of the invention will employ, unless otherwise indicated, conventional techniques of cell biology, molecular biology, histology, immunology, oncology, which are within the capabilities of a person of ordinary skill in the art. Such techniques are explained in the literature. See, for example: Skoog, D.A., et al. (2013) Fundamentals of Analytical Chemistry, 9th edition, Cengage learning; Walker J.M. (2009) The Protein Protocols Handbook, 3rd edition, Springer Nature; Green, M.R. and Sambrook, J. (2012) Molecular Cloning: A Laboratory Manual, 4th Edition, Cold Spring Harbor Laboratory Press; Ausubel, F.M., et al. (2003) Current Protocols in Molecular Biology, John Wiley & Sons; Hill, A. J. (2013) DNA Sequencing Protocols, Humana Press; Nielsen, B.S. and Jones, J. (2021) In Situ Hybridization Protocols, Springer US; Herdewijn, P. (2010) Oligonucleotide Synthesis: Methods and Applications, Humana Press; and Luo, Y. (2019) CRISPR Gene Editing: Methods and Protocols, Springer New York. Each of these general texts is herein incorporated by reference.

### EXAMPLES

The invention will now be further described by way of Examples, which are meant to serve to assist one of ordinary skill in the art in carrying out the invention and are not intended in any way to limit the scope of the invention.

### Example 1 - Evaluating the safety profile of Cl-harbouring SIN.LVs in vitro and in vivo

SIN.LVs were generated with a strong enhancer/promoter derived from the spleen focus forming virus (SFFV), driving the expression Green Fluorescent Protein (GFP), a neutral marker transgene, in presence or absence of chromatin insulators (**Figure 1A**).

The human CTCF-based chromatin insulators A2 and E2 (Liu, M., et al. 2015. Nat Biotechnol 33, 198-203) were cloned within the vector U3 region of the 3' LTR, so that after reverse transcription such insulators are present in both LTRs and in parallel orientation flanking the expression cassette. Such insulators harbour a human-derived CTCF-binding site included in the LV in antisense orientation compared to the transcription of the lentiviral vector backbone and of the expression cassette.

### Generation of insulator-harbouring SIN.LVs and in vitro characterization

The different insulated LVs were generated that were tested for vector titre and infectivity, showing no significant differences comparted to the parental un-insulated vector performances.

The impact of the insulated vectors on the surrounding genome was investigated. We transduced K562 cells at high multiplicity of infection (MOI=50) with each of the different vectors. Upon bulk transduction of the K562 cells, single cells clones were FACS-sorted to purity by GFP expression and two clones for each vector treatment with high copies were selected. This strategy allowed to study a total of 214 different genomic loci to address the impact of the different features in different genomic loci within the same genomic contexts by means of i) chromatin folding; ii) CTCF-binding and iii) gene expression **(**Figure 1B).

We addressed the impact of the inserted constructs on genome folding by performing in-situ HiC that allows capturing long range chromatin interactions throughout the genome (Rao, S.S., et al. 2014. Cell 159, 1665-1680). On the global scale, the comparison of the HiC data derived from the different vector marked clones across the entire genome showed a good similarity, indicating that the LV insertions did not impact the overall genome structure.

To address the impact of vector integration on chromatin topology we inspected the chromatin folding at the insertion sites found in the different clones. From the HiC-derived sequencing pairs, we first extracted *in silico* the reads containing both host-genomic and vector-derived sequences, which are the result of vector and genome interaction, and mapped the individual reads to the corresponding reference genome (human and lentiviral genomes, respectively). We next associated the reads to the genomic vector insertion site mapped on the human genome to identify bona fide vector-derived interactions with the surrounding human genome within 1.5Mb from the associated IS. Finally, by binning the individual interactions at 1Kb windows, we generated peaks of clustered sequencing reads to stringently identify vector interaction sites (ITR) of the integrated vector in the different genomic locations. We then positioned all the vector insertion sites in the same orientation and looked if interactions displayed a specific preference upstream or downstream the 5' or 3' LV LTR respectively. Insertions of the strong enhancer promoter alone (SIN.LV) engaged in long-range interactions with a minimal bias towards insertion-upstream loci, while A2 and E2 insulator-bearing LVs, showed a significant enrichment of long range ITRs upstream the 5' or 3' LV LTR (**Figure 1C**).

These results were further validated by an orthogonal method, LV-specific chromatin conformation capture (LV-4C). LV-4C technique is an adaptation of the well-establish technique 4C (Zhao, Z., et al. 2006. Nat Genet 38, 1341-1347) designed to retrieve LTR sequence together with the flanking host cellular genomic sequence flanking the vector insertion locus fused by proximity ligation to the long-range interacting site. Therefore, LV-4C allows to precisely map both the vector integration site and to the cognate long-range interactor. In two independent clones transduced with the A2 insulator bearing LV, we observed a significant skewing of interactions with cellular genomic CTCF insulator binding sites in convergent orientation with the A2 insulator present in the LV. Such enrichment of ITRs with convergent CTCF sites in the genome was not observed in the control LV without insulators (**Figures 1D and 1E**).

Finally, we analysed the global chromatin topology surrounding the IS exclusively from the genome-wide HiC data. By performing an insulation boundary analysis, we addressed the conformation of the genome in random loci compared to the ones harbouring vector integrations. This analysis further confirmed the skewing of orientation of interactions mediated by A2 and E2 insulators compared to the SIN LV harbouring IS (p<0.0001) (**Figure 1F**).

Overall, these data indicate that the inclusion of chromatin insulators within the vector LTR can impact chromatin topology, redirecting interactions mediated by the vector towards genomic sites containing convergent CTCF-sites.

### In vivo safety study of Cl-harbouring SIN.LVs in tumour prone mice

To study the impact of the insulators on vector safety *in vivo,* we took advantage of a previously established genotoxicity assay based on tumour prone *Cdkn2a* KO mouse models, which are extremely sensitive to vector-mediated insertional mutagenesis and resulting in accelerated tumour onset when treated with genotoxic vectors compared to untreated controls (Cantore, A., et al. 2015. Sci Transl Med 7, 277ra228).

We previously showed that *Cdkn2a*^{+/-} mice are less sensitive to genotoxic stress compared to homozygous KO mice since they do not develop spontaneous tumours until at late age. On the other hand, *Cdkn2a*^{+/-} mice are significantly more sensitive than wild type mice since they develop early tumours when treated with genotoxic lentiviral vectors (Cesana, D., et al. 2014. Mol Ther 22, 774-785). The virtual lack of background oncogenesis in *Cdkn2a*^{+/-} mice allows to broaden the time interval for the observation of potential late manifestations of vector-induced oncogenesis. Therefore, we injected newborn *Cdkn2a*^{+/-} mice with concentrated vector preparations at a dose of 1×10⁸ TU/mouse, which is sufficient for sensing vector-mediated insertional mutagenesis in this assay (**Figure 2A**).

Injection of the parental uninsulated SIN.LV in *Cdkn2a*^{+/-} mice induced a significant acceleration of tumour onset in these mice compared to Mock controls. On the other hand, the treatment with A2.LV and E2.LV did not cause any significant acceleration in the time of tumour onset compared to Mock controls (**Figure 2B**).

Furthermore, the insulated LVs resulted to be significantly less genotoxic compared to the parental un-insulated SIN.LV. Since the liver resulted to be the most affected tissue, we extracted the genomic DNA from the tumour infiltrated liver tissue of vector-treated *Cdkn2a*^{+/-} mice (n=21 for A2, and n=15 for E2.LV) to perform Linear Amplification Method (LAM)-PCR for the retrieval of vector integration sites. The PCR-products were sequenced by Illumina platform and the resulting sequences mapped on the murine genome (mm9) and analysed by bioinformatics pipelines for integration site analysis (Calabria, A., et al. (2020). Bioinformatics 36, 1622-1624).

We retrieved a total of >1000 integration sites from vector marked tissues and analysed common integration sites (CIS) to identify the recurrently targeted genes in tumour tissues as possible culprits of oncogenesis. In the insulator-harbouring test groups, only two CIS were found and the comparison with the CIS genes identified in mice treated with the parental vector, show a marked reduction in the total number of CIS genes identified with the insulated vectors (**Figure 2C**). Myotrophin *(Mtpn)* was the only CIS in the E2 IS dataset. This gene is involved in the growth but not proliferation of myocytes, with no evidence of role in cancer. Phosphatase and Tensine Homologue (*Pten*), a known tumour suppressor gene, was the only CIS of the A2-treated group.

Overall, this *in vivo* study shows that the inclusion of A2 and E2 insulator in the SIN.LV backbone can improve the survival kinetics of the treated mice compared to the parental virus and is associated with a reduced burden of oncogenic insertions at common integration sites genes. These results show for the first time that the inclusion of chromatin insulators in the LTR can improve the safety of the vectors *in vivo.*

### Example 2 - In vivo safety evaluation of SIN.LVs with reduced aberrant-splicing/read-through potential

As chromatin insulators act on enhancer-mediated genotoxicity, we further aimed our efforts at tacking read-through/promoter insertion and aberrant-splicing events which can be potentially mediated by constitutive of cryptic sites present in the vector backbone.

To this aim, we exploited a strategy using the micro-RNA target sites (miRT) to specifically degrade aberrant transcripts containing vector sequences. We identified the LV sequences most frequently involved in chimeric transcript formation. These LV regions could be tagged by sequences complementary to miRT elements active in target cells, thus allowing selective degradation, through the miRNA pathway, of vector-mediated aberrant spliced transcripts.

We equipped a SIN.LV with the moderate PGK enhancer-promoter with two tandem repeats of miRT from the target tissues (hematopoietic) in sense and antisense orientation compared to the LV transcriptional orientation to allow targeted degradation of chimeric transcripts generated by sense and antisense read-through/spicing events. We specifically designed SIN LVs harbouring miRT sequences recognized by mir223-3p and mir142-3p, that are expressed in hematopoietic lineages, within the SIN LTR (miRT-LTR LV) or in the vector backbone and outside the gene expression cassette (mirT LV) (**Figure 3A**).

We then assessed the genotoxicity of the SIN LVs harbouring miRT sequences by taking advantage of an *in vivo* model based on *Cdkn2a*^{-/-} mice (**Figure 3B**). We observed that while the parental vector without any miR sequence led to a significant acceleration in the time of tumor onset compared to control un-injected mice (n=37), the injection of miRT-LTR LV (n=38) and miRT LV (n=33) in *Cdkn2a*^{-/-} mice did not cause any significant acceleration in hematopoietic tumor onset **(****Figure 3C**). Vector integration sites analysis showed no oncogenic CIS associated with the miRT-LTR-LV. miRT LV targeted *Pten* and *Neil3,* a DNA glycosylase involved in DNA repair **(****Figure 3D****).**

Overall, these studies show that this new advanced design lentiviral vectors completely abrogated the residual vector genotoxicity in the highly sensitive mouse model.

### Example 3 - Combination of safety-features to enhance vector safety

Having demonstrated the efficacy of the two distinct safety features on vector-mediated insertional mutagenesis mechanisms, we combined within the SIN.LV backbone the miRT sequences and Cl. As miRT and CI act on two different insertional mutagenesis mechanisms, their activity may synergize in order to further improve LV safety by avoiding or reducing enhancer-mediated gene activation and aberrant transcription and chimeric transcripts generation.

We took a step further in vector design, by applying the knowledge that CI explicit their action in the genome mainly when present in convergent orientation between each other, and cloned these elements within the vector backbone in two main configurations **(****Figure 4A****):**
i) convergent orientation (COMBO.CONV), to allow the generation of a vector-internal chromatin loop that isolates the vector expression cassette from the host genome; and
ii) divergent configuration (COMBO.DIV), to allow interaction to be mediated between vector and host genomic insulators. This configuration allows excluding the vector cassette and its enhancer/promoter from the host genes containing loops.

We generated novel vectors in the context of a liver-specific expression cassette, by using the hepatocyte-specific Enhanced Transthyretin (ET) enhancer-promoter (Cantore, A., et al. 2015. Sci Transl Med 7, 277ra228) and the neutral transgene GFP. As positive a for genotoxicity, a LV with active LTRs harbouring the same ET enhancer/promoter was used as we showed in previous studies being highly genotoxic.

As the inclusion of the miRT requires the inversion of the expression cassette to avoid the degradation of the transgene, we generated a control vector harbouring SIN.LTRs and the ET-GFP-pA cassette in antisense orientation. Such cassette includes also miRT regulatory sites for micro-RNA 142-3p to avoid clearance of transduced hepatocytes. We included in the antisense cassettes the E2-A2 convergent (COMBO.CONV) and divergent (COMBO.DIV) configurations and with the miRT sites in the LTRs. We included a total of 4 tandem copies of miRT sequences in the LTR, where the liver-active miRT-22 and the hematopoietic restricted miRT-223, were placed in antisense orientation to the LV transcription, while liver-specific miRT-122 and hematopoietic restricted miRT-223, were cloned in sense orientation **(****Figure 4B****).**

Additionally, we included a strong polyA signal for the optimal transgene mRNA termination and expression. A schematic of the vectors is shown in **Figure 4C****.**

We conducted the biosafety study in WT mice injected with the described liver-specific vectors and treated with cancer promoting agent CCI₄ to promote liver tumour formation. This *in vivo* model was previously reported being sensitive to vector-mediated insertional mutagenesis in liver. In detail, newborn WT mice were treated by temporal-vein injection with 5e7-1e8 TU (2.5-5e10 TU/kg) with the different LVs. To promote the development of hepatocellular carcinoma (HCC), all mice (both, injected with LV and untreated controls) were subjected to CCI₄-treatment twice a week, for 6 weeks, starting since 8 weeks of age as previously described. Mice were sacrificed after one year of follow-up to collect tissue samples for histopathology analysis, integration sites analysis, VCN determination, GFP expression analysis **(****Figure 4D****).**

Histopathological analysis at experiment endpoint identified 34 tumour masses from the genotoxic control group (ET.LTR) and 14 from the SIN vector with the antisense expression cassette only. Differently the LVs armed with CIs and miRTs showed a significantly lower incidence of liver tumours compared to controls. Three microscopic tumours were found in mice treated with the LV with the insulators in convergent orientation and one HCC in the LV with the CIs in divergent orientation **(****Figure 4E****, left hand side).** Tissue abnormalities defined as focal cell alterations were significantly more frequent in the genotoxic control group and in the SIN parental vector compared to the LVs with insulators and miRTs **(****Figure 4E****, right hand side),** indicating a superior safety profile of the latter vectors compared to the parental one and to the genotoxic control.

Integration site analysis on DNA from healthy tissues and liver-derived masses indicated the culprits of oncogenesis for each experimental group. From the genotoxic control group (ET.LTR), we confirmed CIS gene *Rian* as predominant target of genotoxicity accompanied by clones with insertions in *Sos1* and *Fign* as possible targets of insertional mutagenesis as previously described **(****Figure 4F****, top left).** On the other hand, in the DNA from liver masses marked with the SIN.ET LV with the antisense cassette alone, *Rian* integrations were predominant and highly abundant. Other dominant clones showed integration targeting *Lingo2, Cchcr1* and *Diap3,* whose upregulation might be involved in proliferation and transformation **(****Figure 4F****, top right).** For the COMBO.CONV treated mice, the three tumours detected by histopathology were microscopical and not visible by macroscopic tissue inspection, so that integration sites information on these tumours was not possible to be analysed **(****Figure 4F****, bottom left).** The only tumour arisen in the COMBO.DIV treatment group was marked by an integration upstream the *Erh* gene, which is involved in proliferation **(****Figure 4F****, bottom right).**

Taken together the reduced frequency of tumours induced by the COMBO.LVs and the absence of targeting of well-known genotoxic genes commonly found in the liver genotoxicity, such as *Rian, Fign* and Sos1 indicated the ability of these features in to reduce vector-mediated insertional mutagenesis *in vivo.*

### Example 4 - Optimisation of vector designs comprising a combination of safety-features

We further improved the COMBO-vector design to optimize vector transgene expression, vector titres and infectivity.

We found that the cloning of insulator sequences in internal position, reduced transgene expression and vector infectivity. Moreover, also the orientation of the expression cassette, when cloned in opposite orientation to the vector backbone could impact these vector features. Thus, to optimize the vector constructs harbouring CI and miRTs, we designed new vector versions with the expression cassette placed in sense configuration. Here, we included for all vectors the antisense-oriented miRTs in the LTR, as the orientation-dependent nature of these feature would not affect transgene expression. Conversely, the miRT in sense orientation were cloned upstream the expression cassette.

Insulator sequences were added to this design. Besides the previously validated A2 and E2 insulators, we decided to test in this configuration additional insulator sequences from Liu et al. as original element and/or as a minimal element harbouring the CTCF-core and a minimal flanking sequence.

We generated and tested the following constructs **(****Figure 5A****):**
- Div-Conv.LV: This design incudes A2mut insulator, where we eliminated a cryptic polyadenylation site of the original A2 insulator sequence, and E2 insulator. These two elements are placed in divergent orientation in the LTR and spaced by antisense miRT sequences. Sense oriented mirT sites are present upstream the expression cassette.
- Div-Conv.Core.LV: In this vector construct we included A2 and E2 insulator core sequences placed in divergent orientation and spaced by antisense oriented miRT. Sense oriented mirT sites are present upstream the expression cassette.
- Split-Conv.LV: In this design we placed A2mut insulator in a single copy in internal position and E2 within the LTR. MirT target sites are cloned as described above.
- CI-COMBO 1-6: We generated a family of 6 vector constructs harboring different CIs within the LTR. In all these constructs, mirT target sites are cloned as described above:
   - CI-COMBO 1: contains the original A1 insulator from Liu et al. and the A2mut insulator (in which we eliminated a cryptic polyadenylation site from the original sequence). These CI are placed in parallel orientation in the LTR and in antisense orientation to the transcriptional orientation of the LV, as our previous *in vivo* data showed such configuration being safe and able to promote insulating loops.
   - CI-COMBO 2: contains the A1 core and the A2 core CTCF binding sites and minimal flanking sequences, also called core insulation sequences. These CI are placed in parallel orientation in the LTR and in antisense orientation to the LV transcription and expression cassette.
   - CI-COMBO 3: contains the A2mut insulator sequence in antisense orientation.
   - CI-COMBO 4: contains the A2 insulator CTCF binding sites and minimal flanking sequences, also called core insulation sequences. This CI is cloned in the LTR and in antisense orientation compared to LV backbone.
   - CI-COMBO 5: contains the A1 insulator CTCF binding sites and minimal flanking sequences, also called core insulation sequences. This CI is cloned in the LTR and in antisense orientation compared to LV backbone.
   - CI-COMBO 6: contains the B2 insulator CTCF binding sites and minimal flanking sequences, also called core insulation sequences. This CI is cloned in the LTR and in antisense orientation compared to LV backbone.

LV stocks were prepared from all the new constructs using state-of-the-art protocol and vector titerstitres, infectivity and GFP expression were tested on 293T cells in technical triplicates for each construct. For each vector preparation, vectors were used for transduction of 293T cells to perform vector titertitre in technical triplicates. Supernatant from LV dilutions was collected to perform infectivity analysis based on P24 detection by ELISA. Cells from single copy dilutions, were harvested for GFP MFI evaluation at FACS and titertitre was analyzedanalysed by integration. A standard PGK.GFP vector was added to the test vector panel as standard readout of lab-grade gene therapy vector.

Compared to the original COMBO LVs' configurations, the optimized combo-vectors display a very high MFI value, similar to their relative parental construct devoid of the safety features (**Figure 5B**).

In general, vector titre and infectivity performances were good and above the threshold of 2×10⁷ TU/mL and 1×10⁴ TU/ng p24 respectively (**Figure 5C** **and** **5D**), which define a good lab-grade LV, as shown by a standard PGK LV control. The CI-COMBO#2 to #6 and Div-Conv.Core LVs had the overall best performances in terms of vector titre and infectivity. The presence of core sequences, either individual or in combination, or a single full-length insulator did not impact vector titre and infectivity that resulted comparable to control LVs. In line with this, A1 and A2mut or A2mut and E2 full length combinations performed better when only the core insulator element was used.

Overall, our in vitro testing showed that the novel combo-configurations display an infectivity and transgene expression similar to the parental LVs.

By engineering LVs with combinations of chromatin insulators and micro-RNA-target sites, acting on different insertional mutagenesis mechanisms, we demonstrate the ability of these features in the vector backbone to improve its safety profile.

We exploited tailored vector designs, with specific enhancer-promoters and expression cassettes and assayed these vectors armed with safety features in different stringent *in vivo* genotoxicity assays, to study the impact on safety of these features independently, in combination and different configurations, demonstrating superior safety *in vivo* of LVs harbouring these features.

We first evaluated the impact of LV insertions harbouring chromatin insulators on the host genome *in vitro* in K562 cell lines. To challenge the insulation abilities of the Cl, we decided to include these elements in LV-vectors harbouring a strong-viral enhancer promoter driving a neutral expression cassette. In this context we addressed multiple independent genomic loci to understand the mechanism of action of chromatin insulators when inserted in the genome. By an extensive study taking advantage of different chromatin conformation capture-technologies and ChlP-Seq analysis we demonstrated that CI in the vector redirect genomic interactions towards CTCF-harbouring genomic sites in convergent orientation. These data show for the first time that time the impact of the insulators when administered by lentiviral-vector insertions throughout the genome. Our finding concerning the mechanism of action of the CI in the vectors, are in line with the evidence that CI based on CTCF-exploit their insulation ability when placed in convergent configuration.

We next addressed the implications of these effects in terms of vector biosafety *in vivo* in a sensitive assay based on tumour prone *Cdkn2a+*/*-* mice. We exploited this mouse model in this setting as the parental vector, harbouring the SF enhancer-promoter driving the expression cassette, accelerates the survival kinetics versus Mock-controls proportionally faster than in *Cdkn2a-*/*-* mice. Thus, the time window to screen and detect improvements in vector safety is more favourable in this setting. Indeed, we observed that *Cdkn2a+*/*-* tumour prone mice injected with A2 and E2 insulated SIN.LVs display a significantly better survival compared to the parental LV, which is in line with the reduction or even absence of cancer-causing CIS identified in tumour infiltrated tissues of these mice.

We further studied the use of miRT-sequences in the SIN.LV design to abrogate aberrant transcription, a mechanism on which insulator sequences cannot act. miRT sequences have been used in the past in the vector-context to reduce transgenes' off-target expression and/or to reduce clearance of transduced cells from the immune system. Differently, here we exploited for the first time the use of miRT as safety features. After identifying candidate positions in the LV to include the miRT-sequences to be retained in aberrant transcripts, we tested two main configurations harbouring miRT in the LTRs and in the vector backbone upstream the 3'LTR.

To allow the evaluation of any improvement on read-through/aberrant transcription, we needed to use a vector construct able to promote these specific events and in absence of other predominant insertional mutagenesis mechanisms that could potentially hinder the detection of the safety improvements mediated by the inclusion of miRT. Therefore, we chose a SIN.LV design with the PGK moderate cellular promoter that we previously showed being able to induce aberrant splicing and minimal/virtually absent enhancer-mediated activation events. In this case, to allow stringent evaluation of the vector safety *in vivo,* we used the *Cdkn2a*^{-/-} tumour prone mouse model, sensitive to genotoxicity induced by the insertions of the parental virus. The inclusion of miRT in the LV instead, had a positive impact on vector safety, both in terms of survival kinetics *in vivo* and on reduction or even absence of insertions targeting cancer-causing CIS genes.

As both safety features act on distinct insertional mutagenesis mechanisms, we combined both elements to virtually abrogate any ability of the vector to induce enhancer-mediate activation and read-through/aberrant transcription. We took advantage of the *in vivo* assay of liver-oncogenesis, a sensitive model we previously used to study insertional mutagenesis in the liver in WT mice, exploiting expression cassettes with the liver-specific ET enhancer-promoter driving neutral expression cassettes. We observed that by reverting the expression cassette and removing the PRE elements from the SIN vector, the ET enhancer-promoter now placed immediately upstream the 3'LTR made the SIN.ET vector more genotoxic. We thus used this backbone to engineer within the LV the combination of the miRT and insulator safety features, testing two different combinations with miRT elements in the LTR and insulators in internal position either in convergent or divergent orientation surround the expression cassette. Since CTCF is expressed in all cell types we expected the insulator sequences used to be active in the original hematopoietic cells as well as in the liver. Differently, miRT elements are susceptible to tissue specificity. Therefore, in our design, the inclusion of miRT active in liver and hematopoietic cells, would mean that neither hepatocytes nor tissue resident hematopoietic cells would be targeted by read-through/aberrant transcription.

Our results indicate that the combination of miRT and insulators significantly improve the safety of the parental vector, both in terms of phenotype, represented by a reduced tumour-burden in the mice accompanied with the strong reduction of cancer-causing CIS.

In addition to safety improvements, feasibility of vector manufacturing and clinical applications need to be considered, of which transgene expression, vector titre and infectivity are fundamental measures. We observed that the reversal of the expression cassette may have an impact on the expression of the transgene. We therefore redesigned the vectors with insulators and miRT combinations within the more standard forward-oriented expression cassette. Since sense-oriented expression cassettes are typically used in gene therapy applications, this could help in the easy inclusion of the novel backbone design with the combined safety features in current gene therapy vectors. We therefore used a vector backbone with sense-oriented expression cassette, in which we engineered different combinations of insulators and miRTs. These novel combinations harbour antisense-oriented miRT in the LTR, while sense-oriented miRT upstream the expression cassette, to avoid the degradation of the transgene. Aimed at maintaining the convergent nature of insulators within the LV, we designed i) Div.Conv, ii) Div.Conv.Core and iii) Split.Conv vectors. We also decided to test if the reduction of the full-length insulator sequence to bare CTCF-core element and a minimal flanking sequence could be beneficial to vector performances. We also tested insulators in the LTR in parallel orientation, in single or tandem configuration, as strongly insulated chromatin borders in the genome benefit from multiple CTCF sites (Huang, H., et al. 2021. Nat Genet 53, 1064-1074).

The comparison of the novel combination vectors to a standard PGK control as reference and to the original COMBO CON and DIV show significant improvements in terms of transgene expression, vector titres and infectivity. We hypothesize that the recovery in GFP expression and titre might be mostly dependent by the presence of the expression cassette in sense orientation. In addition, an effect on vector titre and infectivity could be dependent on the convergent nature of the insulators, as their engagement in the plasmid during vector production might be an obstacle to efficient transcription of the RNA genome of the vector.

Overall, we showed that the novel combinations of insulators and miRT harbouring vectors are produced efficiently, and that together with their improved safety profile could benefit future gene therapy clinical applications.

### EMBODIMENTS

Various preferred features and embodiments of the present invention will now be described with reference to the following numbered paragraphs (paras).
1. A polynucleotide comprising a nucleotide sequence comprising an expression cassette and a transcription termination site, wherein the nucleotide sequence further comprises:
   (a) one or more miRNA target sequences having the same orientation as the expression cassette, upstream from the expression cassette and/or downstream from the transcription termination site;
   (b) one or more miRNA target sequences having the opposite orientation as the expression cassette; and
   (c) one or more insulator elements.
2. The polynucleotide of para 1, wherein the nucleotide sequence comprises two or more, three or more, or four or more miRNA target sequences having the same orientation as the expression cassette, upstream from the expression cassette and/or downstream from the transcription termination site.
3. The polynucleotide of para 1 or 2, wherein the nucleotide sequence comprises two or more miRNA target sequences having the same orientation as the expression cassette, upstream from the expression cassette, optionally wherein the two or more miRNA target sequences are two or more copies of the same miRNA target sequence arranged in tandem; and/or two or more miRNA target sequences having the same orientation as the expression cassette, downstream from the transcription termination site, optionally wherein the two or more miRNA target sequences are two or more copies of the same miRNA target sequence arranged in tandem.
4. The polynucleotide of any preceding para, wherein the nucleotide sequence comprises one or more miRNA target sequences having the same orientation as the expression cassette, upstream from the expression cassette, optionally wherein the nucleotide sequence comprises one or more miRNA target sequences having the same orientation as the expression cassette, directly upstream from the expression cassette and/or wherein the nucleotide sequence comprises one or more miRNA target sequences having the same orientation as the expression cassette 100 bp or less upstream from the expression cassette.
5. The polynucleotide of any preceding para, wherein the nucleotide sequence comprises one or more miRNA target sequences having the same orientation as the expression cassette, downstream from the transcription termination site, optionally wherein the nucleotide sequence comprises one or more miRNA target sequences having the same orientation as the expression cassette, directly downstream from the transcription termination site and/or wherein the nucleotide sequence comprises one or more miRNA target sequences having the same orientation as the expression cassette 100 bp or less downstream from the transcription termination site.
6. The polynucleotide of any preceding para, wherein the one or more miRNA target sequences having the same orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, or a combination thereof, preferably wherein the one or more miRNA target sequences having the same orientation as the expression cassette are miR142-3p target sequences.
7. The polynucleotide of any preceding para, wherein the one or more miRNA target sequences having the same orientation as the expression cassette each comprise or consist of a nucleotide sequence selected from the group consisting of SEQ ID NOs: 1 to 10, preferably wherein the one or more miRNA target sequences having the same orientation as the expression cassette each comprise or consist of the nucleotide sequence of SEQ ID NO: 1.
8. The polynucleotide of any preceding para, wherein the nucleotide sequence comprises one or more miRNA target sequences having the opposite orientation as the expression cassette, upstream from the expression cassette and/or downstream from the expression cassette.
9. The polynucleotide of any preceding para, wherein the nucleotide sequence comprises two or more, three or more, or four or more miRNA target sequences having the opposite orientation as the expression cassette, optionally upstream from the expression cassette and/or downstream from the transcription termination site.
10. The polynucleotide of any preceding para, wherein the nucleotide sequence comprises two or more miRNA target sequences having the opposite orientation as the expression cassette, upstream from the expression cassette, optionally wherein the two or more miRNA target sequences are two or more copies of the same miRNA target sequence arranged in tandem; and/or two or more miRNA target sequences having the opposite orientation as the expression cassette, downstream from the expression cassette, optionally wherein the two or more miRNA target sequences are two or more copies of the same miRNA target sequence arranged in tandem.
11. The polynucleotide of any preceding para, wherein the nucleotide sequence comprises one or more miRNA target sequences having the opposite orientation as the expression cassette, upstream from the expression cassette, optionally wherein the nucleotide sequence comprises one or more miRNA target sequences having the opposite orientation as the expression cassette, directly upstream from the expression cassette and/or wherein the nucleotide sequence comprises one or more miRNA target sequences having the opposite orientation as the expression cassette 100 bp or less upstream from the expression cassette.
12. The polynucleotide of any preceding para, wherein the nucleotide sequence comprises one or more miRNA target sequences having the opposite orientation as the expression cassette, downstream from the expression cassette, optionally wherein the nucleotide sequence comprises one or more miRNA target sequences having the opposite orientation as the expression cassette, directly downstream from the expression cassette and/or wherein the nucleotide sequence comprises one or more miRNA target sequences having the opposite orientation as the expression cassette 100 bp or less downstream from the expression cassette.
13. The polynucleotide of any preceding para, wherein the one or more miRNA target sequences having the opposite orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, or a combination thereof, preferably wherein the one or more miRNA target sequences having the opposite orientation as the expression cassette are miR223-3p target sequences.
14. The polynucleotide of any preceding para, wherein the one or more miRNA target sequences having the opposite orientation as the expression cassette each comprise or consist of a nucleotide sequence selected from the group consisting of SEQ ID NOs: 11 to 20, preferably wherein the one or more miRNA target sequences having the opposite orientation as the expression cassette each comprise or consist of the nucleotide sequence of SEQ ID NO: 12.
15. The polynucleotide of any preceding para, wherein the nucleotide sequence comprises one or more insulator elements, upstream from the expression cassette and/or downstream from the expression cassette.
16. The polynucleotide of any preceding para, wherein the nucleotide sequence comprises a pair of insulator elements flanking the expression cassette.
17. The polynucleotide of para 16, wherein the pair of insulator elements is in a divergent orientation.
18. The polynucleotide of para 16, wherein the pair of insulator elements is in a convergent orientation.
19. The polynucleotide of para 16, wherein the pair of insulator elements is in a parallel orientation.
20. The polynucleotide of any preceding para, wherein the one or more insulator elements are CTCF-based insulator elements.
21. The polynucleotide of any preceding para, wherein the one or more insulator elements each comprise a nucleotide sequence selected from the group consisting of SEQ ID NOs: 23 to 28.
22. The polynucleotide of any preceding para, wherein the one or more insulator elements each comprise or consist of a core insulator sequence.
23. The polynucleotide of any preceding para, wherein the one or more insulator elements each comprise or consist of a nucleotide sequence selected from the group consisting of SEQ ID NOs: 29 to 36.
24. The polynucleotide of any preceding para, wherein the one or more insulator elements each comprise or consist of a full length insulator sequence.
25. The polynucleotide of any preceding para, wherein the one or more insulator elements each comprise or consist of a nucleotide sequence selected from the group consisting of SEQ ID NOs: 37 to 90, 102 or 103, or a nucleotide sequence having at least 90% identity thereto.
26. The polynucleotide of any preceding para, wherein the nucleotide sequence further comprises a 3' LTR, optionally wherein the 3' LTR is a self-inactivating (SIN) 3' LTR.
27. The polynucleotide of para 26, wherein the 3' LTR comprises one or more miRNA target sequences having the same orientation as the expression cassette.
28. The polynucleotide of para 26, wherein the 3' LTR does not comprise a miRNA target sequence having the same orientation as the expression cassette.
29. The polynucleotide of any of paras 26 to 28, wherein the 3' LTR comprises one or more miRNA target sequences having the opposite orientation as the expression cassette.
30. The polynucleotide of any of paras 26 to 28, wherein the 3' LTR does not comprise a miRNA target sequence having the opposite orientation as the expression cassette.
31. The polynucleotide of any of paras 26 to 30, wherein the 3' LTR comprises one or more insulator elements.
32. The polynucleotide of any of paras 26 to 30, wherein the 3' LTR does not comprise an insulator element.
33. The polynucleotide of any preceding para, wherein the nucleotide sequence further comprises a 5' LTR, optionally wherein the 5' LTR is a self-inactivating (SIN) 5' LTR.
34. The polynucleotide of para 33, wherein the 5' LTR comprises one or more miRNA target sequences having the same orientation as the expression cassette.
35. The polynucleotide of para 33, wherein the 5' LTR does not comprise one or more miRNA target sequences having the same orientation as the expression cassette.
36. The polynucleotide of any of paras 33 to 35, wherein the 5' LTR comprises one or more miRNA target sequences having the opposite orientation as the expression cassette.
37. The polynucleotide of any of paras 33 to 35, wherein the 5' LTR does not comprise a miRNA target sequence having the opposite orientation as the expression cassette.
38. The polynucleotide of any of paras 33 to 37, wherein the 5' LTR comprises one or more insulator elements.
39. The polynucleotide of any of paras 33 to 37, wherein the 5' LTR does not comprise an insulator element.
40. A vector comprising the polynucleotide of any preceding para, optionally wherein the vector is a viral vector, a plasmid, or a transposon
41. The vector of para 40, wherein the vector is a viral vector selected from the group consisting of a lentiviral vector, a retroviral vector, and an adeno-associated viral (AAV) vector, preferably wherein the vector is a retroviral vector, more preferably wherein the vector is a lentiviral vector.
42. A viral particle comprising the polynucleotide of any of paras 1 to 39.
43. The viral particle of para 42, wherein the viral particle is selected from the group consisting of a lentiviral particle, a retroviral particle, and an adeno-associated viral (AAV) particle, preferably wherein the viral particle is a retroviral particle, more preferably wherein the viral particle is a lentiviral particle.
44. A retroviral vector comprising from 5' to 3' direction: a 5' LTR; one or more sense-oriented miRNA target sequences; a sense-oriented expression cassette; and a 3' LTR.
45. The retroviral vector of para 44, wherein the retroviral vector further comprises one or more antisense-oriented miRNA target sequences upstream from the expression cassette and/or downstream from the expression cassette.
46. The retroviral vector of para 44 or 45, wherein the 3' LTR comprises one or more antisense-oriented miRNA target sequences.
47. The retroviral vector of any of paras 44 to 46, wherein the retroviral vector further comprises one or more insulator elements upstream from the expression cassette and/or downstream from the expression cassette.
48. The retroviral vector of any of paras 44 to 47, wherein the 3' LTR comprises one or more insulator elements.
49. The retroviral vector of any of paras 44 to 48, wherein the 3' LTR comprises one or more antisense-oriented miRNA target sequences and one or more insulator elements.
50. The retroviral vector of any of paras 44 to 49, wherein the retroviral vector comprises from 5' to 3' direction: a 5' LTR; one or more sense-oriented miRNA target sequences; a sense-oriented expression cassette; and a 3' LTR, wherein the 3' LTR comprises from 5' to 3' direction: a first insulator element, one or more antisense-oriented miRNA target sequences, and a second insulator element, wherein the first insulator element and the second insulator element are in a divergent orientation.
51. The retroviral vector of para 50, wherein the first insulator element is an A2 insulator element and the second insulator element is an E2 insulator element.
52. The retroviral vector of para 50 or 51, wherein the first insulator element and the second insulator element each comprise or consist of a full length insulator sequence, optionally wherein the first insulator element comprises or consists of the nucleotide sequence of SEQ ID NO: 103, or a nucleotide sequence having at least 90% identity thereto, and the second insulator element comprises or consists of the nucleotide sequence of SEQ ID NO: 55, or a nucleotide sequence having at least 90% identity thereto.
53. The retroviral vector of para 50 or 51, wherein the first insulator element and the second insulator element each consist of a core insulator sequence, optionally wherein the first insulator element comprises or consists of the nucleotide sequence of SEQ ID NO: 34 and the second insulator element comprises or consists of the nucleotide sequence of SEQ ID NO: 32.
54. The retroviral vector of any of paras 44 to 49, wherein the retroviral vector comprises from 5' to 3' direction: a 5' LTR; one or more sense-oriented miRNA target sequences; a sense-oriented expression cassette; a first insulator element; and a 3' LTR, wherein the 3' LTR comprises from 5' to 3' direction: one or more antisense-oriented miRNA target sequences, and a second insulator element, wherein the first insulator element and the second insulator element are in a divergent orientation.
55. The retroviral vector of para 54, wherein the first insulator element is an A2 insulator element and the second insulator element is an E2 insulator element, optionally wherein the first insulator element comprises or consists of the nucleotide sequence of SEQ ID NO: 103, or a nucleotide sequence having at least 90% identity thereto, and the second insulator element comprises or consists of the nucleotide sequence of SEQ ID NO: 55, or a nucleotide sequence having at least 90% identity thereto.
56. The retroviral vector of any of paras 44 to 49, wherein the retroviral vector comprises from 5' to 3' direction: a 5' LTR; one or more sense-oriented miRNA target sequences; a sense-oriented expression cassette; and a 3' LTR, wherein the 3' LTR comprises from 5' to 3' direction: one or more insulator elements and one or more antisense-oriented miRNA target sequences.
57. The retroviral vector of para 56, wherein the one or more insulator elements consist of an A1 insulator element and an A2 insulator element in a parallel orientation, wherein the A1 insulator element and the A2 insulator element each comprise or consist of a full length insulator sequence, optionally wherein the A1 insulator element comprises or consists of the nucleotide sequence of SEQ ID NO: 64, or a nucleotide sequence having at least 90% identity thereto, and the A2 insulator element comprises or consists of the nucleotide sequence of SEQ ID NO: 103, or a nucleotide sequence having at least 90% identity thereto.
58. The retroviral vector of para 56, wherein the one or more insulator elements consist of an A1 insulator element and an A2 insulator element in a parallel orientation, wherein the A1 insulator element and the A2 insulator element each consist of a core insulator sequence, optionally wherein the A1 insulator element comprises or consists of the nucleotide sequence of SEQ ID NO: 33 and the A2 insulator element comprises or consists of the nucleotide sequence of SEQ ID NO: 34.
59. The retroviral vector of para 56, wherein the one or more insulator elements consist of an A2 insulator element, wherein the A2 insulator element comprises or consists of a full length insulator sequence, optionally wherein the A2 insulator element comprises or consists of the nucleotide sequence of SEQ ID NO: 103, or a nucleotide sequence having at least 90% identity thereto.
60. The retroviral vector of para 56, wherein the one or more insulator elements consist of an A2 insulator element, wherein the A2 insulator element consists of a core insulator sequence, optionally wherein the A2 insulator element comprises or consists of the nucleotide sequence of SEQ ID NO: 34.
61. The retroviral vector of para 56, wherein the one or more insulator elements consist of an A1 insulator element, wherein the A1 insulator element consists of a core insulator sequence, optionally wherein the A1 insulator element comprises or consists of the nucleotide sequence of SEQ ID NO: 33.
62. The retroviral vector of para 56, wherein the one or more insulator elements consist of a B2 insulator element, wherein the B2 insulator element consists of a core insulator sequence, optionally wherein the A2 insulator element comprises or consists of the nucleotide sequence of SEQ ID NO: 35.
63. A retroviral vector comprising from 5' to 3' direction: a 5' LTR; an antisense-oriented expression cassette; and a 3' LTR, wherein the retroviral vector further comprises:
   (a) one or more antisense-oriented miRNA target sequences, upstream from the expression cassette and/or downstream from the expression cassette;
   (b) one or more sense-oriented miRNA target sequences, optionally upstream from the expression cassette and/or downstream from the expression cassette; and
   (c) one or more insulator elements, optionally upstream from the expression cassette and/or downstream from the expression cassette.
64. The retroviral vector of para 63, wherein the 3' LTR does not comprise one or more antisense-oriented miRNA target sequences.
65. The retroviral vector of para 63 or 64, wherein the 3' LTR does not comprise one or more sense-oriented miRNA target sequences.
66. The retroviral vector of any of paras 44 to 65, wherein the one or more sense-oriented miRNA target sequences comprise or consist of two or more miR142-3p target sequences, optionally wherein the one or more sense-oriented miRNA target sequences comprise or consist of the nucleotide sequence of SEQ ID NO: 21.
67. The retroviral vector of any of paras 44 to 66, wherein the one or more antisense-oriented miRNA target sequences comprise or consist of two or more miR223-3p target sequences, optionally wherein the one or more antisense-oriented miRNA target sequences comprise or consist of the nucleotide sequence of SEQ ID NO: 22.
68. The retroviral vector of any of paras 44 to 67, wherein the 3' LTR is a self-inactivating (SIN) 3' LTR.
69. The retroviral vector of any of paras 44 to 68, wherein the retroviral vector is a lentiviral vector, preferably wherein the retroviral vector is an integration-proficient lentiviral vector (IPLV).
70. The retroviral vector of any of paras 44 to 69, wherein the retroviral vector is substantially non-genotoxic.
71. The retroviral vector of any of paras 44 to 70, wherein the one or more sense-oriented miRNA target sequences and the one or more antisense-oriented miRNA target sequences avoid and/or reduce aberrant splicing and/or read-through events.
72. The retroviral vector of any of paras 44 to 71, wherein the one or more insulator elements avoid and/or reduce enhancer-mediated gene activation.
73. An isolated cell comprising the polynucleotide of any of paras 1 to 39, the vector of para 40 or 41, the viral particle of para 42 or 43, or the retroviral vector of any of paras 44 to 72.
74. The isolated cell of para 73, wherein the cell is a hematopoietic stem and/or progenitor cell (HSPC) or a non-stem cell, such as a liver cell or a T cell.
75. A pharmaceutical composition comprising the polynucleotide of any of paras 1 to 39, the vector of any of para 40 or 41, the viral particle of para 42 or 43, the retroviral vector of any of paras 44 to 72, or the isolated cell of para 73 or 74, in combination with a pharmaceutically acceptable carrier, diluent or excipient.
76. The polynucleotide of any of paras 1 to 39, the vector of any of para 40 or 41, the viral particle of para 42 or 43, the retroviral vector of any of paras 44 to 72, the isolated cell of para 73 or 74, or the pharmaceutical composition of para 75, for use as a medicament.
77. A method of reducing the genotoxicity of a polynucleotide or vector comprising a nucleotide sequence comprising an expression cassette and a transcription termination site, wherein the method comprises introducing into the nucleotide sequence:
   (a) one or more miRNA target sequences having the same orientation as the expression cassette, upstream from the expression cassette and/or downstream from the transcription termination site;
   (b) one or more miRNA target sequences having the opposite orientation as the expression cassette; and
   (c) one or more insulator elements.
78. A method of reducing the genotoxicity of a retroviral vector comprising a sense-oriented expression cassette, wherein the method comprises introducing into the retroviral vector:
   (a) one or more sense-oriented miRNA target sequences upstream from the sense-oriented expression cassette.
79. The method according to para 78, wherein the method further comprises introducing into the retroviral vector:
   (b) one or more antisense-oriented miRNA target sequences upstream from the sense-oriented expression cassette and/or downstream from the sense-oriented expression cassette; and/or
   (c) one or more insulator elements upstream from the sense-oriented expression cassette and/or downstream from the sense-oriented expression cassette.
80. A method of reducing the genotoxicity of a retroviral vector comprising an antisense-oriented expression cassette, wherein the method comprises introducing into the retroviral vector:
   (a) one or more antisense-oriented miRNA target sequences upstream from the antisense-oriented expression cassette and/or downstream from the antisense-oriented expression cassette;
   (b) one or more sense-oriented miRNA target sequences, optionally upstream from the antisense-oriented expression cassette and/or downstream from the antisense-oriented expression cassette; and
   (c) one or more insulator elements, optionally upstream from the antisense-oriented expression cassette and/or downstream from the antisense-oriented expression cassette.
81. An insulator element comprising or consisting of a sequence having at least 95% sequence identity to SEQ ID NO: 103, wherein the insulator element comprises the nucleotides G and C at positions corresponding to nucleotides 45 and 46, respectively, wherein the nucleotides are numbered with reference to SEQ ID NO: 103.
82. The insulator element of para 81, wherein the insulator element comprises SEQ ID NO: 34.
83. The insulator element of para 81 or 82, wherein the insulator element comprises or consists of SEQ ID NO: 103.
84. An insulator element comprising or consisting of a sequence having at least 95% sequence identity to SEQ ID NO: 102, wherein the insulator element comprises the nucleotides G and C at positions corresponding to nucleotides 221 and 222, respectively, wherein the nucleotides are numbered with reference to SEQ ID NO: 102.
85. The insulator element of para 84, wherein the insulator element comprises SEQ ID NO: 30.
86. The insulator element of para 84 or 85, wherein the insulator element comprises or consists of SEQ ID NO: 102.
87. A polynucleotide comprising a nucleotide sequence comprising the insulator element of any of paras 81 to 86.
88. The polynucleotide of para 87, wherein the nucleotide sequence further comprises an expression cassette and the insulator element flanks the expression cassette.
89. A vector comprising the polynucleotide of para 87 or 88.
90. An isolated cell comprising the polynucleotide of para 87 or 88, or the vector of para 89.
91. A self-inactivating (SIN) 3' LTR comprising one or more antisense-oriented miRNA target sequences and one or more insulator elements.
92. A self-inactivating (SIN) 3' LTR comprising or consisting of a nucleotide sequence having at least 90% sequence identity to a nucleotide sequence selected from the group consisting of SEQ ID NOs: 104 to 112.
93. The SIN 3' LTR of para 91 or 92, wherein the SIN 3' LTR comprises an antisense-oriented miRNA target sequence comprising or consisting of the nucleotide sequence of SEQ ID NO: 22.
94. The SIN 3' LTR any of paras 91 to 93, wherein the SIN 3' LTR comprises an insulator element comprising or consisting of a nucleotide sequence selected from the group consisting of SEQ ID NOs: 29 to 36.
95. A polynucleotide comprising a nucleotide sequence comprising the SIN 3' LTR of any of paras 91 to 94.
96. A vector comprising the polynucleotide of para 95.
97. An isolated cell comprising the polynucleotide of para 95, or the vector of para 91.

## Claims

1. A polynucleotide comprising a nucleotide sequence comprising an expression cassette and a transcription termination site, wherein the nucleotide sequence further comprises:
(a) one or more miRNA target sequences having the same orientation as the expression cassette, upstream from the expression cassette and/or downstream from the transcription termination site;
(b) one or more miRNA target sequences having the opposite orientation as the expression cassette; and
(c) one or more insulator elements.

2. The polynucleotide of claim 1, wherein the one or more miRNA target sequences having the same orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, or a combination thereof, preferably wherein the one or more miRNA target sequences having the same orientation as the expression cassette are miR142-3p target sequences; and/or wherein the one or more miRNA target sequences having the opposite orientation as the expression cassette are selected from a miR223 target sequence, a miR142 target sequence, a miR22 target sequence, a miR122 target sequence, a miR126 target sequence, a miR124 target sequence, a miR133a target sequence, a miR133b target sequence, a let7f target sequence, a miR7 target sequence, or a combination thereof, preferably wherein the one or more miRNA target sequences having the opposite orientation as the expression cassette are miR223-3p target sequences.

3. The polynucleotide of claim 1 or 2, wherein the nucleotide sequence comprises a pair of insulator elements flanking the expression cassette, optionally wherein the pair of insulator elements is in a divergent orientation, a convergent orientation or a parallel orientation and/or wherein the one or more insulator elements are CTCF-based insulator elements.

4. A vector comprising the polynucleotide of any of claims 1 to 3, optionally wherein the vector is a viral vector selected from the group consisting of a lentiviral vector, a retroviral vector, and an adeno-associated viral (AAV) vector, preferably wherein the vector is a retroviral vector, more preferably wherein the vector is a lentiviral vector.

5. A viral particle comprising the polynucleotide of any of claims 1 to 3, optionally wherein the viral particle is selected from the group consisting of a lentiviral particle, a retroviral particle, and an adeno-associated viral (AAV) particle, preferably wherein the viral particle is a retroviral particle, more preferably wherein the viral particle is a lentiviral particle.

6. A retroviral vector comprising from 5' to 3' direction: a 5' LTR; one or more sense-oriented miRNA target sequences; a sense-oriented expression cassette; and a 3' LTR, optionally wherein the retroviral vector further comprises one or more antisense-oriented miRNA target sequences upstream from the expression cassette and/or downstream from the expression cassette; and/or one or more insulator elements upstream from the expression cassette and/or downstream from the expression cassette.

7. A retroviral vector comprising from 5' to 3' direction: a 5' LTR; an antisense-oriented expression cassette; and a 3' LTR, wherein the retroviral vector further comprises:
(a) one or more antisense-oriented miRNA target sequences, upstream from the expression cassette and/or downstream from the expression cassette;
(b) one or more sense-oriented miRNA target sequences, optionally upstream from the expression cassette and/or downstream from the expression cassette; and
(c) one or more insulator elements, optionally upstream from the expression cassette and/or downstream from the expression cassette.

8. The retroviral vector of claim 6 or 7, wherein the retroviral vector is substantially non-genotoxic, wherein the one or more sense-oriented miRNA target sequences and the one or more antisense-oriented miRNA target sequences avoid and/or reduce aberrant splicing and/or read-through events, and/or wherein the one or more insulator elements avoid and/or reduce enhancer-mediated gene activation.

9. An isolated cell comprising the polynucleotide of any of claims 1 to 3, the vector of claim 4, the viral particle of claim 5, or the retroviral vector of any of claims 6 to 8, optionally wherein the cell is a hematopoietic stem and/or progenitor cell (HSPC) or a non-stem cell, such as a liver cell or a T cell.

10. A pharmaceutical composition comprising the polynucleotide of any of claims 1 to 3, the vector of claim 4, the viral particle of claim 5, the retroviral vector of any of claims 6 to 8, or the isolated cell of claim 9, in combination with a pharmaceutically acceptable carrier, diluent or excipient.

11. The polynucleotide of any of claims 1 to 3, the vector of any of claim 4, the viral particle of claim 5, the retroviral vector of any of claims 6 to 8, the isolated cell of claim 9, or the pharmaceutical composition of claim 10, for use as a medicament.

12. A method of reducing the genotoxicity of a polynucleotide or vector comprising a nucleotide sequence comprising an expression cassette and a transcription termination site, wherein the method comprises introducing into the nucleotide sequence:
(a) one or more miRNA target sequences having the same orientation as the expression cassette, upstream from the expression cassette and/or downstream from the transcription termination site;
(b) one or more miRNA target sequences having the opposite orientation as the expression cassette; and
(c) one or more insulator elements.

13. A method of reducing the genotoxicity of a retroviral vector comprising a sense-oriented expression cassette, wherein the method comprises introducing into the retroviral vector:
(a) one or more sense-oriented miRNA target sequences upstream from the sense-oriented expression cassette,
optionally wherein the method further comprises introducing into the retroviral vector:
(b) one or more antisense-oriented miRNA target sequences upstream from the sense-oriented expression cassette and/or downstream from the sense-oriented expression cassette; and/or
(c) one or more insulator elements upstream from the sense-oriented expression cassette and/or downstream from the sense-oriented expression cassette.

14. A method of reducing the genotoxicity of a retroviral vector comprising an antisense-oriented expression cassette, wherein the method comprises introducing into the retroviral vector:
(a) one or more antisense-oriented miRNA target sequences upstream from the antisense-oriented expression cassette and/or downstream from the antisense-oriented expression cassette;
(b) one or more sense-oriented miRNA target sequences, optionally upstream from the antisense-oriented expression cassette and/or downstream from the antisense-oriented expression cassette; and
(c) one or more insulator elements, optionally upstream from the antisense-oriented expression cassette and/or downstream from the antisense-oriented expression cassette.

15. An insulator element comprising or consisting of:
(a) a sequence having at least 95% sequence identity to SEQ ID NO: 103, wherein the insulator element comprises the nucleotides G and C at positions corresponding to nucleotides 45 and 46, respectively, wherein the nucleotides are numbered with reference to SEQ ID NO: 103; or
(b) a sequence having at least 95% sequence identity to SEQ ID NO: 102, wherein the insulator element comprises the nucleotides G and C at positions corresponding to nucleotides 221 and 222, respectively, wherein the nucleotides are numbered with reference to SEQ ID NO: 102.

16. A self-inactivating (SIN) 3' LTR comprising one or more antisense-oriented miRNA target sequences and one or more insulator elements; optionally wherein the SIN 3' LTR comprises or consists of a nucleotide sequence having at least 90% sequence identity to a nucleotide sequence selected from the group consisting of SEQ ID NOs: 104 to 112.

17. A polynucleotide comprising a nucleotide sequence comprising the insulator element or claim 15 or the SIN 3' LTR of claim 16.

18. A vector comprising the polynucleotide of claim 17.

19. An isolated cell comprising the polynucleotide of claim 17 or the vector of claim 18.
